(19) **Europäisches Patentamt / European Patent Office / Office européen des brevets**

(11) **EP 3 474 859 B1**

(12) # EUROPEAN PATENT SPECIFICATION

(45) Date of publication and mention
of the grant of the patent:
**24.12.2025 Bulletin 2025/52**

(21) Application number: **17830134.7**

(22) Date of filing: **22.06.2017**

(51) International Patent Classification (IPC):
$A61K\ 31/616^{(2006.01)}$     $A61K\ 31/155^{(2006.01)}$
$A61K\ 31/215^{(2006.01)}$     $A61K\ 31/7068^{(2006.01)}$
$A61K\ 39/395^{(2006.01)}$     $A61P\ 35/00^{(2006.01)}$
$A61P\ 35/04^{(2006.01)}$     $A61K\ 45/06^{(2006.01)}$

(52) Cooperative Patent Classification (CPC):
**A61K 31/155; A61K 31/215; A61K 31/415;
A61K 31/616; A61K 31/7068; A61K 45/06;
A61P 35/00; A61P 35/04; C07D 231/12;
C12Q 1/6886;** C12Q 2600/106; C12Q 2600/158

(86) International application number:
**PCT/CA2017/050768**

(87) International publication number:
**WO 2018/014111 (25.01.2018 Gazette 2018/04)**

(54) **CANCER TREATMENT USING AN ANTI-CANCER STEM CELL AGENT IN COMBINATION WITH A NEUL SIALIDASE INHIBITOR AFTER PRIMARY CANCER TREATMENT**

BEHANDLUNG VON KREBS MIT EINEM ANTI-KREBS-STAMMZELLENMITTEL IN KOMBINATION MIT EINEM NEULSIALIDASE-HEMMER NACH PRIMÄRER KREBSTHERAPIE

TRAITEMENT DU CANCER UTILISANT UN AGENT ANTI-CELLULES SOUCHES CANCÉREUSES EN COMBINAISON AVEC UN INHIBITEUR DE LA SIALIDASE (NEUL) APRÈS UN TRAITEMENT DU CANCER PRIMAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.06.2016 US 201662353340 P**

(43) Date of publication of application:
**01.05.2019 Bulletin 2019/18**

(73) Proprietors:
• **Harless, William Warren**
**Middle River, Nova Scotia B0E 1B0 (CA)**
• **Szewczuk, Myron**
**Kingston, Ontario K7L 3N6 (CA)**

(72) Inventors:
• **Harless, William Warren**
**Middle River, Nova Scotia B0E 1B0 (CA)**
• **Szewczuk, Myron**
**Kingston, Ontario K7L 3N6 (CA)**

(74) Representative: **Danubia Patent & Law Office LLC
Bajcsy-Zsilinszky út 16
1051 Budapest (HU)**

(56) References cited:
**WO-A1-2013/063679     WO-A1-2016/101060**

• **ZHANG HUI-HUI ET AL: "Combinational strategies of metformin and chemotherapy in cancers", CANCER CHEMOTHERAPY AND PHARMACOLOGY, SPRINGER VERLAG, BERLIN, DE, vol. 78, no. 1, 27 April 2016 (2016-04-27), pages 13 - 26, XP035990416, ISSN: 0344-5704, [retrieved on 20160427], DOI: 10.1007/ S00280-016-3037-3**
• **BARON BYRON ET AL: "Resistance to Gemcitabine in the Pancreatic Cancer Cell Line KLM1-R Reversed by Metformin Action", ANTICANCER RESEARCH, vol. 35, no. 4, April 2015 (2015-04-01), pages 1941 - 1949, XP002797523**

**EP 3 474 859 B1**

**(Cont. next page)**

- WEN YUE ET AL: "Metformin combined with aspirin significantly inhibit pancreatic cancer cell growth *in vitro* and *in vivo* by suppressing anti-apoptotic proteins Mcl-1 and Bcl-2", ONCOTARGET, vol. 6, no. 25, 28 August 2015 (2015-08-28), XP055666744, DOI: 10.18632/oncotarget.4126
- YOUNG KWANG CHAE ET AL: "Repurposing metformin for cancer treatment: current clinical studies", ONCOTARGET, vol. 7, no. 26, 19 March 2016 (2016-03-19), pages 40767 - 40780, XP055432349, DOI: 10.18632/oncotarget.8194
- ALLISON STEPHANIE ET AL: "Encapsulation of oseltamivir phosphate and gemcitabine in poly (D, L-lactic-co-glycolic acid) polymer as a delivery vehicle in the treatment of pancreatic cancer in heterotopic xenografts of tumors growing in RAGxC gamma double mutant mice", GLYCOBIOLOGY, vol. 23, no. 11, November 2013 (2013-11-01), & ANNUAL CONFERENCE OF THE SOCIETY-FOR-GLYCOBIOLOGY; ST PETERSBURG, FL, USA; NOVEMBER 17 -20, 2013, pages 1403, XP002797524
- O'SHEA ET AL.: "Therapeutic targeting ofNeul sialidase with oseltamivir phosphate (Tamiflu®) disables cancer cell survival in human pancreatic cancer with acquired chemoresistance", ONCOTARGETS AND THERAPY, vol. 7, 16 January 2014 (2014-01-16), pages 117 - 134, XP055452856
- HAXHO ET AL.: "Oseltamivir phosphate monotherapy ablates tumor neovascularization, growth, and metastasis in mouse model of human triple-negative breast adenocarcinoma", BREAST CANCER, vol. 6, 2014, pages 191 - 203, XP055452859
- JIN ET AL.: "Observation of Curative Efficacy and Prognosis Following Combination Chemotherapy with Celecoxib in the Treatment of Advanced Colorectal Cancer", THE JOURNAL OF INTERNATIONAL MEDICAL RESEARCH, vol. 39, no. 6, 2011, pages 2129 - 2140, XP055452864
- BHOLA ET AL.: "TGF-beta inhibition enhances chemotherapy action against triple-negative breast cancer", THE JOURNAL OF CLINICAL INVESTIGATION, vol. 123, no. 3, 1 March 2013 (2013-03-01), pages 1348 - 1358, XP055452867
- WANG ET AL.: "IL-6 Inhibition Reduces STAT3 Activation and Enhances the Antitumor Effect of Carboplatin", MEDIATORS OF INFLAMMATION, vol. 2016, 1 January 2016 (2016-01-01), pages 1 - 8, XP055452869, ISSN: 14661861
- KURTOVA ET AL.: "Blocking PGE2-induced tumour repopulation abrogates bladder cancer chemoresistance", NATURE, vol. 517, no. 7533, 8 January 2015 (2015-01-08), pages 209 - 213, XP055452873
- ZHANG ET AL.: "Aspirin counteracts cancer stem cell features, desmoplasia and gemcitabine resistance in pancreatic cancer", ONCOTARGET, vol. 6, no. 12, 5 February 2015 (2015-02-05), pages 9999 - 10015, XP055452876
- HARLESS: "Cancer treatments transform residual cancer cell phenotype", CANCER CELL INTERNATIONAL, vol. 11, no. 1, 7 January 2011 (2011-01-07), pages 1 - 4, XP021088183, ISSN: 14752867
- HARLESS: "Revisiting perioperative chemotherapy: the critical importance of targeting residual cancer prior to wound healing", BMC CANCER, vol. 9, no. 1, 22 April 2009 (2009-04-22), pages 118, XP021048996

**Description**

FIELD OF THE INVENTION

The present invention relates to compositions and methods for treatment of patients with cancer. In particular, the invention relates to an anti-cancer cytotoxic therapeutic agent, metformin, a neu-1 sialidase inhibitor and aspirin for use in an anti cancer treatment regimen in a patient with cancer.

BACKGROUND OF THE INVENTION

Cancers of epithelial origin account for 90% of cancer deaths worldwide.

The standard treatment of most potentially curable solid tumors is surgical removal often followed by chemotherapy. For the major cancer killers such as lung, breast, and colorectal cancer, the administration of chemotherapy after the tumour is surgically removed may eradicate micrometastatic disease (disease undetectable using conventional imaging technologies) in those patients who still harbor residual cancer cells after surgery. However, this treatment is often unsuccessful.

The resistance of any given cancer cell to conventional medical treatments may not primarily result from the possession or acquisition of specific point mutations but instead largely reside in a distinct cancer cell subpopulation of cancer stem cells. In addition to being relatively resistant to conventional medical therapies, cancer stem cells are also capable of metastasis and tissue colonization. As few as 200 cancer cells displaying the stem cell phenotype can form tumours in animal models, while 20,000 cancer cells without the stem cell phenotype fail to form tumours. These cells are therefore particularly relevant to cancer metastasis and recurrence and treatment resistance.

O'Shea et al. (O' Shea et al., 2014) disclose that Oseltamivir phosphate disabled the cancer cell survival mechanism(s) and reversed the epithelial-mesenchymal transition characteristic of the phenotypic E-cadherin to N-cadherin changes associated with resistance to drug therapy. The authors concluded that targeting of Neu1 sialidase with oseltamivir phosphate disables the intrinsic signaling platform for cancer cell survival in human pancreatic cancer with acquired chemoresistance.oseltamivir phosphate (Tamiflu) can be a potential therapeutic agent for pancreatic cancer resistant to drug therapy.

Zhang and Gao review combinational strategies of metformin and chemotherapy in cancers and summarize a number of molecular mechanisms underlying anticancer effects of metformin. The authors discuss clinical trials regarding metformin in combination of chemotherapeutic drugs and the imitation related to the potential role of metformin in cancer treatment is also discussed.

Thus, there is a need for new therapeutic strategies in treating patients with cancer.

SUMMARY OF THE INVENTION

The invention is set out in the appended set of claims.

BRIEF DESCRIPTION OF THE DRAWINGS

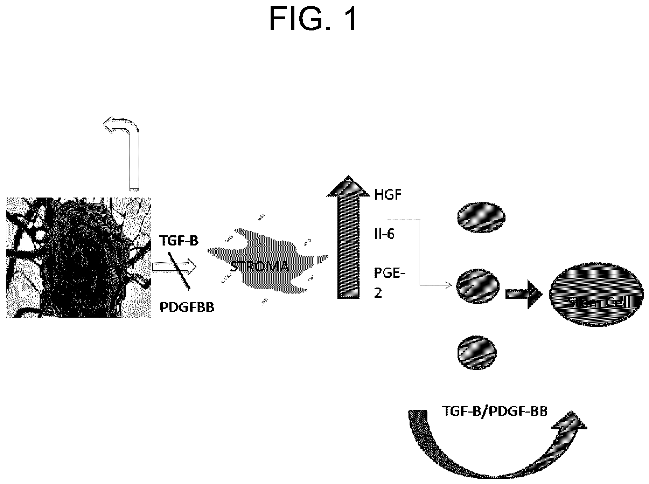

Figure 1 depicts the expression of cytokines after tumour removal. Tumour removal is shown to lead to a drop in TGF-beta and PDGFBB levels, which triggers increased stromal secretion of HGF, PGE-2 and IL-6. TGF-beta and PDGFBB levels will rapidly rise to baseline and in concert with HGF, IL-6, and PGE-2 facilitate cellular proliferation and stem cell enrichment in a residual cancer cell population.

Figure 2 depicts scatterplots of flow cytometry experiments from enriched circulating tumour cells stained with an antibody containing CD44 following treatment with various cytokines and cytokine cocktails.

Figure 3 depicts scatterplots of flow cytometry from enriched circulating tumour cells stained with an antibody containing CD133 following treatment with various cytokines and cytokine cocktails.

Figure 4 depicts cell proliferation of various cell subpopulations in the HCT -15 cell line after exposure to various cytokines and cytokine cocktails.

Figure 5 depicts cell proliferation of various cell subpopulations in the SW 620 cell line after exposure to various cytokines and cytokine cocktails.

Figure 6 depicts cell proliferation of various cell subpopulations in cultured circulating tumour cells after exposure to various cytokines and cytokine cocktails. Cells were stained using an antibody containing CD44 PE.

Figure 7 depicts cell proliferation of various cell subpopulations in cultured circulating tumour cells after exposure to various cytokines and cytokine cocktails. Cells were stained using an antibody containing CD133 PE.

Figure 8 depicts enriched circulating tumour cells cultured with or without cytokines, including IL-6, IL-8 and PDGF-BB. The addition of IL-6 significantly increased (p <0.05) the subpopulation of EpCAM+CD133- cells as compared to control.

Figure 9 depicts flow cytometry scatterplots of enriched circulating tumour cells stained with EpCAM A488, CD133PE and Lgr5-PE-Vio770 following treatment with IL-6, IL-8 and PDGFBB.

Figure 10 depicts the percentage of CD44+CD133- cells following treatment with various cytokine and cytokine cocktails.

Figure 11 depicts the percentage of CD44+CD133- cells following treatment with various cytokines and Irinotecan.

Figure 12 depicts the percentage of CD44+CD133- cells following treatment with various cytokine cocktails and Irinotecan.

Figure 13 depicts the effect of treatment of various cytokines on cellular apoptosis.

Figure 14 depicts the effect of treatment of various cytokines and Irinotecan on cellular apoptosis.

Figure 15 depicts the effect of treatment of various cytokine cocktails and Irinotecan on cellular apoptosis.

Figure 16 depicts the percentage of CD44-CD133+ cells following treatment with various cytokines and cytokines cocktails.

Figure 17 depicts the percentage of CD44-CD133+ cells following treatment with various cytokines and Irinotecan.

Figure 18 depicts the percentage of CD44-CD133+ cells following treatment with various cytokine cocktails and Irinotecan.

Figure 19 depicts the percentage of CD44+CD133+ cells following treatment with various cytokine and cytokine cocktails.

Figure 20 depicts the percentage of CD44+CD133+ cells following treatment with various cytokines and Irinotecan.

Figure 21 depicts the percentage of CD44+CD133+ cells following treatment with various cytokine cocktails and Irinotecan.

Figure 22 depicts the treatment protocol for cohort 4 of Example 7.

Figure 23 depicts tumor volume after treatment with gemcitabine alone or under combinatorial treatment strategy.

Figure 24 depicts the upregulation of E cadherin after treatment with Oseltamivir Phosphate and Aspirin.

Figure 25 depicts the reversal of EMT and inhibition of angiogenesis in drug resistant cancer cell lines triggered by Oseltamivir Phosphate.

Figure 26 depicts the reversal of markers of EMT and angiogenesis in pancreatic cancer using Oseltamivir Phosphate.

Figure 27 depicts the Neuraminidase-1 (Neu1) and matrix metalloproteinase-9 (MMP9) cross-talk in alliance with G protein-coupled receptor(s) (GPCR), regulating receptor tyrosine kinases (RTKs).

Figure 28 depicts metastatic burden abrogated with combination of OP and celecoxib in cohorts 3 and 6.

DETAILED DESCRIPTION

In the following description, numerous specific details are set forth to provide a thorough understanding of the invention. However, it is understood that the invention may be practiced without these specific details.

The references to methods of treatment in the subsequent paragraphs of this description are to be interpreted as references to the compounds, pharmaceutical compositions and medicaments of the present invention for use in a method for treatment of the human (or animal) body by therapy.

The invention is defined in the appended claims. Any technical solutions which may be referred to herein but not falling under the claims is to be understood as described herein for information purposes only.

**Cancer Stem Cells and Metastatic Cancer**

In contrast to the proven ability of chemotherapy to cure micrometastatic cancer in some patients, clinically evident metastatic cancer is generally incurable. Given the emerging evidence of the importance of cancer stem cells in drug resistance and metastatic efficiency, the eradication of this cancer cell subpopulation may be critical to achieve cancer cure.

Differentiated cancer cells may dedifferentiate to a cancer stem cell phenotype, either spontaneously, or, after certain triggering mechanisms. After an initial treatment against cancer, such as surgery, chemotherapy, or radiation, tissue damage induced by that treatment will trigger the release of specific inflammatory molecules fostering the induction of a partial epithelial-mesenchymal transition (EMT) in the remnant cancer cell population and reversion to a cancer stem cell phenotype. The present inventor provides evidence that these same signaling pathways foster cancer stem cell self-renewal as a highly conserved response to tissue damage. The net result of this process is the rapid emergence of a stem cell enriched residual cancer cell population.

The present disclosure provides methods and compositions for inhibiting metastasis or recurrence of a cancer and the development of therapeutic resistance in a patient.

There are three steps to this methodology within any treatment cycle or period of time. For the sake of understanding this technology a treatment cycle is defined as comprising seven days or one week.

1) The first step is the administration of a conventional anti-cancer therapeutic. This step may be referred to as a "primary treatment". In one embodiment, this therapeutic can be any class of cytotoxic medications effective in killing cancer cells. Examples of such agents include alkylators, topoisomerase inhibitors, anti-metabolites, proteasome inhibitors, monoclonal antibodies, etc. This drug is administered on Day One of each treatment cycle.

2) This primary treatment is followed by the administration of a secondary drug effective against cancer stem cells for 24-96 hours (days 2,3,4,5) starting on Days 2 of each treatment cycle. Examples of such a drug include drugs such as metformin or novel small molecule inhibitors that target stem cell self-renewal. Certain conventional anti-cancer therapies, such as alkylating agents, will also be effective if started during this time.
Per Examples 1-3, this time frame is the period when a residual cancer stem cell population will be cycling in response to the highly conserved tissue repair response triggered by the tissue damage induced by the anti-cancer therapy given on Day 1. This cycling renders the normally treatment resistant surviving cancer stem cell population acutely vulnerable to anti-cancer therapies at approximately 18-24 hours after initial chemotherapy on Day 1. These secondary drug or drugs should be started 18-24 hours after the initial treatment and given on at least day 2, and can be considered effective up to 96 hours after initial chemotherapy (days 3, 4, 5).

3) The third arm of this therapeutic methodology, disclosed herein, is the administration of drugs that disrupt the influence of the acute inflammatory response on a surviving cancer cell population. These drugs act to prevent, disrupt, or ameliorate the downstream effects of tissue repair signaling cascades induced by treatments that damage a cancerous tumor. These tissue repair cascades can serve to facilitate regrowth of a cancerous tumor by activating stem cell self-renewal and EMT. These processes in turn will facilitate regrowth of the cancer and a stem cell enriched residual cancer cell population. As detailed further below, examples of these molecules include hepatocyte growth factor, MMP-9, IL-6, Il-8, PDGF-BB, Prostaglandin E2, and TGF-beta. It is also disclosed herein that examples of medications that can disrupt these signaling cascades include monoclonal antibodies that specifically block these ligands or their receptors; or more broad based blockers such as, but not limited to, the neu-1 sialidase inhibitors, including, but not limited to, oseltamivir phosphate and analogues thereof that prevent the dimerization of many of these receptors after being activated by their respective ligands.

In addition, aspirin can also be used to mitigate the influence of this inflammatory response on a residual cancer cell population.

This tissue repair response is evidenced to be maximally upregulated approximately 24 hours after day one of chemotherapy and will last for approximately 72 to 96 hours before returning to baseline. Thus, in one embodiment, these anti-inflammatory therapies are started approximately 24 hours after initial chemotherapy and continued for a minimum of 96 hours. As evidenced in the examples, by blocking this response at the time it is upregulated, one can substantially limit the ability of a cancerous tumor to repair itself and mitigate the development of a drug resistant phenotype, rendering the surviving cancer cell population vulnerable to the same cycle of treatment for a much longer period of time. Moreover, by starving the dividing cells of the inputs it needs to activate the transcriptional machinery essential to cell division, apoptosis may be upregulated, fostering cancer cell death.

In addition to the three-steps methodology, or as an alternative to one or more of the three-steps in this methodology, immunotherapies, particularly blockers of the Programmed Death PD-1/PD-L1 pathway, is administered to enhance antitumor responses from cytotoxic T-lymphocytes. In one embodiment, the immunotherapy is the primary treatment, per the treatment regimen described above. Immunotherapies can be effective treatment modalities for a variety of cancers in a minority of patients who are eligible for treatment with these novel therapeutic compounds (Sharma et al., 2015). The checkpoint signaling pathway involves the programmed death 1 (PD-1) receptor and its ligands (PD-L1/2). This pathway is critical in triggering immune suppression of cytotoxic T cells and thereby preventing immune destruction of cancer cells. Blocking this pathway, either the receptor or its ligands by using checkpoint inhibitors, makes antitumor responses from cytotoxic T-lymphocytes more likely, thereby providing a basis for developing chemotherapeutic immunotherapies. However, challenges chemotherapeutic immunotherapies include, only a minority of patients will respond to these therapies, and those who respond initially will often develop resistance after initial response. Resistance to these therapies can be primary or acquired. Primary resistance refers to initial resistance to these therapies; acquired resistance is the development of resistance after responding initially to the treatments. The biological mechanisms that underlie resistance to these novel immunotherapies are the subject of research, hence finding ways to make tumors more susceptible to potential immunotherapy treatments represent greater chemotherapy options for cancer patients.

Tumor cells interact closely with the stromal cells, immune cells, and extracellular matrix that is part of the tumor microenvironment (TME). This TME can play an important role in limiting the ability of immune cells to detect and eradicate cancer cells. Within this TME a specific transcriptome, also referred to as an innate anti-PD-1 resistance signature or IPRES signature, can predict for resistance to immunotherapy (Hugo et al., 2016). Some of the genes that are upregulated and are part of the IPRES signature include mesenchymal transition genes such as AXL, WNT5A, LOXL2, TWIST2, FAP), angiogenesis genes, wound healing genes, as well as immunosuppressive genes.

The term "cancer", as used herein, may mean a malignant neoplasm that has undergone characteristic anaplasia with loss of differentiation, increased rate of growth, invasion of surrounding tissue, and is capable of metastasis. Residual cancer is cancer that remains in a subject after any form of treatment given to the subject to reduce or eradicate a cancer and recurrent cancer is cancer that recurs after such treatment. Metastatic cancer is a cancer at one or more sites in the body other than the site of origin of the original (primary) cancer from which the metastatic cancer is derived. In the case of a metastatic cancer originating from a solid tumor, one or more (for example, many) additional tumor masses can be present at sites near or distant to the site of the original tumor. In an aspect, the cancer originates from a solid tumour.

The term "tumor", as used herein, refers to a neoplasm or an abnormal mass of tissue that is not inflammatory, which arises from cells of pre-existent tissue. A tumor can be either benign (noncancerous) or malignant (cancerous). Tumors can be solid or hematological. Examples of hematological tumors include, but are not limited to: leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelogenous leukemia, and chronic lymphocytic leukemia), myelodysplastic syndrome, and myelodysplasia, polycythemia vera, lymphoma, (such as Hodgkin's disease, all forms of non-Hodgkin's lymphoma), multiple myeloma, Waldenstrom's macroglobulinemia, and heavy chain disease. Examples of solid tumors, such as sarcomas and carcinomas, include, but are not limited to: fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, lung cancer, ovarian cancer, prostate cancer, benign prostatic hyperplasia, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, epithelial tumors (e.g., cervical cancer, gastric cancer, skin cancer, head and neck tumors), testicular tumor, bladder carcinoma, melanoma, brain tumors, and CNS tumors (such as a glioma, astrocytoma, medulloblastoma, craniopharyogioma, ependymoma, pinealoma, hemangioblastoma, acoustic neuroma, oligodendroglioma, menangioma, meningioma, neuroblastoma and retinoblastoma).

In some aspects, the tumour is a malignant solid tumour.

As used herein, the term "metastasis" refers to the growth of a cancerous tumor in an organ or body part, which is not directly connected to the organ of the original cancerous tumor. Metastasis may be understood to include micrometastasis, which is the presence of an undetectable amount of cancerous cells in an organ or body part which is not directly connected to the organ of the original cancerous tumor. Metastasis can also be defined as several steps of a process, such as the departure of cancer cells from an original tumor site, or primary tumour, and migration and/or invasion of cancer cells to other parts of the body.

In some aspects, metastasis refers to the subsequent growth or appearance of a cancerous tumour in a different location to an original tumour after treatment of the original tumour.

As used herein, the terms "recurrence" and grammatical variants thereof, refer to further growth of neoplastic or cancerous cells after diagnosis of cancer or a primary tumour. Particularly, recurrence may occur when further cancerous cell growth occurs in the cancerous tissue at the site of the original cancer. The cancer may come back to the same place as the original cancer/primary tumor or to another place in the body.

In some aspects, recurrence refers to a cancer that has reappeared at the site of an original cancer or primary tumour after treatment of that original cancer or primary tumour, after a period of time during which the cancer or tumour could not be detected.

The term "treatment" as used herein generally means obtaining a desired physiologic effect. The effect may be prophylactic in terms of completely or partially preventing a disease or condition or symptom thereof and/or may be therapeutic in terms of a partial or complete cure for an injury, disease or condition and/or amelioration of an adverse effect attributable to the injury, disease or condition and includes arresting the development or causing regression of a disease or condition.

In a broad aspect, "primary treatment", as used herein, means any treatment of any kind or means intended to or having the effect of partially or completely removing, destroying, damaging, excising, reducing in size, rendering benign or inhibiting the growth of, a cancer or tumour, and may include one or more such treatments. For example, primary treatment may include one or more of endocrine therapy, chemotherapy, radiotherapy, hormone therapy, surgery, gene therapy, thermal therapy, and ultrasound therapy.

In one aspect, the primary treatment is chemotherapy. In one embodiment, primary treatment refers to the administration of one or more chemotherapeutics on day 1 of a chemotherapy cycle.

"Cancer stem cells", as used herein, are defined and functionally characterized as a small subset of cells from a tumor that can grow indefinitely in vitro under appropriate conditions (i.e., possess the ability for self-renewal), and are able to form tumors in vivo using only a small number of cells. Other common approaches to characterize cancer stem cells involve morphology and examination of cell surface markers, transcriptional profile, and drug response.

"Stem cell enrichment", as used herein, means the increase in size or proportion or concentration of a population of cancer stem cells locally at the site of a cancer or tumour in a patient or in a location distal to the cancer or tumour. Stem cell enrichment may, in some aspects, include cancer stem-cell self-renewal, partial or complete induction of epithelial-mesenchymal transition in a cancer cell, or cancer stem cell proliferation.

## Methods, Compounds and Compositions

In one aspect, there is provided methods and compositions for inhibiting metastasis, treatment resistance or recurrence of a cancer in a patient after a primary treatment of the patient. In one embodiment, the method comprises administering a therapeutically effective amount of a composition as described herein for inhibiting stem cell enrichment in any surviving cancer cell population.

As used herein in one embodiment, a cancer patient refers to a mammal with cancer, in one embodiment, a human patient diagnosed with cancer.

As used herein, "therapeutically effective amount" refers to an amount effective, at dosages and for a particular period of time necessary, to achieve the desired therapeutic result. A therapeutically effective amount of the pharmacological agent may vary according to factors such as the disease state, age, sex, and weight of the individual, and the ability of the pharmacological agent to elicit a desired response in the individual. A therapeutically effective amount is also one in which any toxic or detrimental effects of the pharmacological agent are outweighed by the therapeutically beneficial effects. In respect of a therapeutic agent that disrupts a downstream effect of a tissue repair signaling cascade induced by primary treatment, "therapeutically effective amount", may mean a level that inhibits or prevents the upregulation or the activity of one of more cytokines associated with stem cell enrichment.

As used herein, "therapeutic agent" means any chemical or biological material, and may be a compound or composition, suitable for administration by methods known to those in the art, which induces a desired biological or pharmacological effect. The effect may be local or it may be systemic.

The first therapeutic agent may be any chemotherapeutic agent that targets the machinery of cell division. In one embodiment, the first therapeutic agent is cytotoxic chemotherapy, provided that if the primary treatment is chemotherapy, then the first therapeutic agent is different than the cytoxic chemotherapy used in primary treatment. Such chemotherapeutic agents , include but are not limited to alkylators (including busulphan/carmustine/carboplatin/chlorambucil/cyclophosphamide/cisplatin/dacarbazine/e stramustine/lomustine/melphalan/thiotepa/treosulphan); cytotoxic antibiotics, such as anthracyclines (including doxorubicin/idarubicin/epirubicin/aclarubicin/mitozantrone); topoisomerase inhibitors 1 and 2 (including doxorubicin/irinotecan/etoposide/topotecan); taxanes (including docetaxel/paclitaxel/abraxane); vinca alkaloids (including vincristine/vinblastine/vindesine/vinorelbine); and antimetabolites (including 5-FU/Gemcitabine/cladribine/Cytarabine/fludarabine/mercaptopurine/methotexate/Pemetrex ed/pentostatin/tioguanine).

In one embodiment, chemotherapy treatment comprises immunotherapeutic chemotherapy agents. In some embodiments, immunotherapeutic chemotherapy agents comprise checkpoint inhibitors, including but not limited to: ipilimumab, pembrolizumab, nivolumab, atezolizumab, avelumab, and durvalumab. Optionally, immunotherapeutic chemotherapy agents are used in combination with at least one of a neuraminidase 1 inhibitor and a cyclo-oxygenase inhibitor.

In one embodiment, the first therapeutic agent comprises a nanoparticle that generates heat upon electromagnetic stimulation, optionally a golden nanorod, conjugated to an antibody that recognizes a cancer stem cell surface molecule and the patient is further subjected to nanoparticle-mediated thermal therapy within 12 to 120 hours post-primary treatment. Certain cancer stem cells surface markers are known e.g. CD44 for breast cancer; CD133 for colon cancer; or MET.

Signalling cascades crucial to cancer stem cell self-renewal including Wnt/β-catenin, hedgehog, Notch, NF-κB, and Bcl-2 may be targeted using therapies such as monoclonal antibodies or small molecule inhibitors against these specific cascades. Cancer stem cell kill may be amplified by targeting these signalling cascades at the time stem cells would be most dependent on them for self-renewal and survival.

In one embodiment, compounds or compositions as described herein suitably comprise an inhibitor of one or more cytokines associated with stem cell enrichment. "Inhibitor" includes, but is not necessarily limited to, an antibody, a soluble cytokine binding protein (e.g. a soluble cytokine receptor) and a receptor antagonist. These can also comprise inhibitors of downstream molecules activated by the receptor-ligand interaction of the identified cytokines.

The effectiveness of an anti-cancer treatment such as a chemotherapeutic drug may, for example, be time and concentration dependent. This may be due to the drug being removed from the body through physiological processes such as hepatic or renal clearance. The time frame a given drug dosage is effective may vary, but in respect of certain therapeutics, may not be longer than 24 hours, requiring additional dosages over time to maintain therapeutically effective concentrations in the patient undergoing treatment. As some chemotherapeutics may have a narrow therapeutic index (i.e., dose limiting toxicity may be found at levels necessary for therapeutic effectiveness), daily dosing of chemotherapy may not possible over an extended period of time.

In one aspect the therapeutic agents as described herein are administered to maintain an effective level for at least 96 to 120 hours post primary treatment. In one embodiment, administration then ceases such that circulating levels decline after 96 to 120 hours post primary treatment.

In some embodiments, administration is at the time of primary treatment or within 6 hours, 12 hours, 18 hours, 24 hours, 30 hours, 36 hours, 42 hours, 48 hours, 54 hours, 60 hours, 66 hours, 72 hours, 78 hours, 84 hours, 90 hours, or 96 hours after primary treatment or administering the treatment that induces the population of cancer stems cells in the patient to proliferate. In other embodiments, within 120 hours or within 1 week. In some embodiments, for example, the cancer therapy is administered before about 24 hours after the primary treatment or administering the treatment that induces the population of cancer stems cells in the patient to proliferate. In some embodiments, for example, the cancer therapy is administered from about 24 to about 96 hours after the primary therapy or administering the treatment that induces the population of cancer stems cells in the patient to proliferate. In some embodiments, the method increases the efficacy of cancer therapies.

In still another aspect, the composition is administered within 24 hours +/- 12 hours after primary treatment.

In still another aspect, the compound or composition is administered immediately after or simultaneous with the primary treatment.

After primary treatment, distinct cytokines, including, but not necessarily limited to, TGF-beta, Hepatocyte Growth Factor (HGF), Interleukin 6 (IL-6), prostaglandin E2 (PGE-2), Matrix metallopeptidase 9 (MMP-9), Monocyte Chemoattractant Protein 1 (MCP-1), Platelet derived growth factor BB (PDGF-BB) and placental growth factor (PGF), may be released at a predictable time frame after treatment, particularly after any treatment that damages a cancerous tumor. These cytokines may facilitate self-renewal of normally dormant cancer stem cells, and/or may facilitate the dedifferentiation of more differentiated cancer cells to a stem cell phenotype via induction of a partial EMT. Targeting this cytokine signaling network induced by cancer treatments at the time these signaling networks are upregulated can limit the ability of a residual cancer cell population to repair itself after any treatment that has damaged it, including radiation therapy, cytotoxic chemotherapy, and surgery. The same signaling pathways that trigger cancer stem cell proliferation may also facilitate stem cell enrichment by facilitating the molecular reprogramming of more differentiated cancer cells via a partial EMT and transition to the stem cell phenotype. By targeting this highly conserved signaling pathway at the time it is upregulated a novel medical treatment against cancer is provided.

TGF-beta is secreted by the cancer cell as a latent complex stored in the ECM. Myofibroblasts release bioactive TGF-beta from the latent complex through proteolytic and non-proteolytic mechanisms. Without wishing to be bound by a theory, the present inventor has observed that TGF-beta decreases at 24 hours after primary treatment, and then rapidly increases to normal or above baseline levels; it is postulated that this sudden drop causes the remaining cancer cell population to be more sensitive to the effects of acute inflammatory mediators, including IL-6, HGF, PGE-2, PGF, PDGFBB, MCP-1 and MCP-9, and other known inflammatory mediators. Given the pleiotropic nature of TGF-beta, its sudden drop after initial cancer treatments followed by rapid increase may serve as an initial molecular trigger that facilitates the transition to a stem-cell enriched residual cancer cell population. It has been observed that PDGF-BB decreases and then increases over the same time period in a similar manner to TGF-beta and therefore may also be a part of this molecular trigger. Inventor has documented that both TGF-beta and PDGF-BB, after initial drop, will rapidly return to baseline or above. This fluctuation is predicted to sensitize a residual cancer cell population to the effects of an increase in cytokines such as Il-6 and HGF among others. In concert with these upregulated cytokines and the return to normal or higher levels of PDGF-BB and TGF-beta, an environment is created conducive to stem cell proliferation, EMT, and residual cancer cell proliferation.

In various embodiments, a second therapeutic agent inhibits at least one, at least two, at least three, at least four, at least five, at least six, at least seven, and preferably all of HGF, IL-6, PGE-2, MCP-1, MMP-9, TGF-beta, PDGF-BB, and PGF. In one embodiment, the second therapeutic agent inhibits at least HGF and IL-6.

The second therapeutic agent may comprise therapeutically effective amounts of one or more antibodies specific for at least one of the group consisting of: TGF-beta, HGF, IL-6, PGE-2, MCP-1, MMP-9, PDGF-BB, and PGF. In one embodiment, one or more of an antibody specific for HGF, IL-6, PGE-2, MCP-1, MMP-9 and PGF. In one embodiment, it includes an antibody specific for IL-6 and HGF. In one embodiment, it inhibits HGF, IL-6, TGF-beta, PGE-2, and PDGF-BB, and PGF.

The terms "antibody", "antibodies" and "immunoglobulin", as used herein, refer broadly to any immunological binding agent or molecule that comprises a human antigen binding domain, including polyclonal and monoclonal antibodies. Depending on the type of constant domain in the heavy chains, whole antibodies are assigned to one of five major classes: IgA, IgD, IgE, IgG, and IgM. Several of these are further divided into subclasses or isotypes, such as IgG1, IgG2, IgG3, IgG4, and the like. The heavy-chain constant domains that correspond to the difference classes of immunoglobulins are termed $\alpha, \delta, \varepsilon, \gamma$ and $\mu$, respectively. The subunit structures and three-dimensional configurations of different classes of immunoglobulins are well known. As will be understood by those in the art, the immunological binding reagents encompassed by the term "antibody" extend to all human antibodies and antigen binding fragments thereof, including whole antibodies, dimeric, trimeric and multimeric antibodies; bispecific antibodies; chimeric antibodies; recombinant and engineered antibodies, and fragments thereof. The term "antibody" is thus used to refer to any human antibody-like molecule that has an antigen binding region, and this term includes antibody fragments that comprise an antigen binding domain such as Fab', Fab, F(ab')$_2$, single domain antibodies (DABs), T and Abs dimer, Fv, scFv (single chain Fv), dsFv, ds-scFv, Fd, linear antibodies, minibodies, diabodies, bispecific antibody fragments and the like.

In some embodiments, the second therapeutic agent includes antibodies against one or more of IL-6, HGF, PGE-2, PGF, TGF-beta, PDGF-BB, MCP-1 and MMP-9 or their receptors. In another embodiment, it is or includes a neu-1 sialidase inhibitor such as oseltamivir phosphate that can prevent receptor dimerization triggered by these ligand-receptor interactions and hence prevent downstream activation. In other embodiments, the compound or composition is or includes a small molecule inhibitor of the transcriptional activators triggered by these distinct ligand-receptor interactions, including inhibitors of transcriptional activators such as NF-kb and Stat-3, among others. Other therapeutic agents that may be employed to disrupt the effects of these distinct ligand receptor interactions are miRNA therapeutics that disrupt the post-transcriptional activity of target genes upregulated by the distinct ligand-receptor interactions described above.

Methods for preparing antibodies to IL-6 are known and are disclosed, for example in US5,670,373, US5,866,689, CA2,700,498, CA2,763,039 CA2,632628, US6,235,28, US5,959085, US7,482,436.

Methods for preparing antibodies to HGF are known and are disclosed, for example in US7,718,174, CA2,472,383, CA2,524329, US6,099,84.

Methods for preparing antibodies to PGE-2 are known and are disclosed, for example in CA2,812,756, CA2,664,763, US8,624,002.

Methods for preparing antibodies to PGF are known and are disclosed, for example in CA2,607,471, US7,482,004, CA2,601267.

Methods for preparing antibodies to MCP-1 are known and are disclosed, for example in CA2,609,349, US7,342,106 EP1,888,114, US7687,606.

Methods for preparing antibodies to MMP-9 are known and are disclosed, for example in US8,013,125, US9,120,863, US8,999,332, 2,379,373, US8,008,445.

The antibody may be modified by attachment with various molecules such as an enzyme, a fluorescent material, a radioactive material and a protein. The modified antibody may be obtained by chemically modifying the antibody. This modification method is conventionally used in the art. Also, the antibody may be obtained as a chimeric antibody having a variable region derived from a non-human antibody, and a constant region derived from a human antibody, or may be obtained as a humanized antibody including a complementarity-determining region derived from a non-human antibody, and a framework region (FR) and a constant region derived from a human antibody. Such an antibody may be prepared by using a method known in the art.

In some embodiments, a composition is provided comprising at least one inhibitor of one or more cytokines associated with stem cell enrichment; a therapeutically effective amount of a cytotoxic chemotherapy that targets stem cells; and optionally a non-steroidal anti-inflammatory drug, and a pharmaceutically acceptable carrier.

The compositions of the present disclosure can be administered in any manner which enables inhibition of the effects of the molecules inducing cancer stem cell self-renewal and EMT pathways in cancer cells. The composition may be injected in a pharmaceutically acceptable liquid carrier directly to the site of injury. Alternatively, the composition may be administered intravenously or orally. Depending on the isolation of the primary tumour, other modes of administration may be appropriate.

As used herein, "pharmaceutically acceptable carrier" means any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the pharmacological agent. Pharmaceutically acceptable carriers must be compatible with both the components of the composition and the patient. Other examples of non-aqueous solvents include propylene glycol and other glycols, metabolizable oils, aqueous carriers including water and alcoholic/aqueous solutions, and emulsions or suspensions (eg. saline and buffered media).

Suitable dosage ranges may be readily ascertained by those of skill in the art.

In some embodiments, for example, the cancer may be leukemia, lung cancer, prostate cancer, colorectal cancer, breast cancer, or ovarian cancer.

In some embodiments, for example, the cancer is metastatic. In some embodiments, the primary treatment may be for treatment of a cancer that metastasized.

**Monitoring of Cytokine Levels Post Primary Treatment**

Acute inflammatory mediators and cytokine levels important in physiologic wound repair, EMT induction, and stem cell activation may be evaluated immediately before and after initial treatment against cancer. Post-treatment sampling can suitably be taken at any or all of 24, 48, 72, and 96 hours after primary treatment or at any point therebetween.

In one embodiment, the at least one cytokine is one or more of HGF, IL-6, PGE-2, MMP-9, PDGF-BB, TGF-beta, MCP-1, and PGF.

Persistent upregulation of TGF-beta and PDGFBB above baseline predicts residual micrometastatic disease and hence benefit of adjuvant systemic therapies.

In an aspect, the level is determined in a patient sample selected from the group consisting of: blood sample, serum sample, tissue sample and tumour sample. In an aspect, the level is determined by mRNA or protein analysis.

In some embodiments, a "wound healing pattern" may be detectable in the serum or plasma of patients after primary treatment. This pattern may reveal upregulated expression of growth factors/cytokines with established roles in EMT induction including one or more of TGF-beta, HGF, IL-6, PGE-2, PGF, PDGF-BB, MCP-1 and MMP-9.

In some aspects, the activity levels are determined by mRNA level or protein level analysis. In some aspects, at least one cytokine is selected from the group consisting of: TGF-beta, HGF, IL-6, PGE-2, MMP-9, PDGF-BB, MCP-1 and PGF. In other aspects, the reference activity profile is that of a patient who does not have metastasis or recurrence of the cancer as measured at a predetermined period of time after primary treatment. The sample may be one of a blood sample, tumour sample, serum sample and tissue sample of the patient.

In an embodiment, determining the cancer stem cell proliferation profile comprises determining a concentration of markers in the patient sample indicative of cancer stem cell proliferation. In some embodiments, for example, the markers comprise cellular receptors that are indicative of cancer stem cell proliferation or self-renewal. In some embodiments, the markers comprise cytokines that are indicative of cancer stem cell proliferation, self-renewal or shift into EMT. In some embodiments, the cytokines are selected from TGF-beta, IL-6, HGF, PGE-2, PGF, PDGF-BB, MCP-1 and MMP-9. In one embodiment, IL-6 and HGF and, optionally, one or more of TGF-beta, PGE-2, PGF, PDGF-BB, MCP-1 and MMP-9; in one embodiment, IL-6, HGF, TGF-beta, PGE-2, and PDGF-BB.

In a further embodiment, determining a cancer stem cell proliferation profile comprises determining the extent of proliferation of cancer stem cells in the patient sample. In some embodiments, for example, proliferation may be determined by running the sample in a fluorescence activated cell sorter (FACS) and measuring the fraction of cells proliferating by measuring DNA content as a surrogate for cells undergoing cellular division. In some embodiments, for example, circulating cancer cells retrieved from a patient after surgery are cultured, and the retrieved cells' ability to be maintained in culture and to be passaged may be used to identify the extent of the stem cells within the circulating tumor cell population, as it is the stem cell fraction cells that would enable the persistence of the culture line.

In some embodiments, the patient sample is obtained at one or more of the group consisting of: prior to primary treatment, at the time of primary treatment, within 1 hour after primary treatment, within 6 hours after primary treatment, within 12 hours after primary treatment, within 18 hours after primary treatment, within 24 hours after primary treatment, within 30 hours after primary treatment, within 36 hours after primary treatment, within 42 hours after primary treatment, within 48 hours after primary treatment, within 54 hours after primary treatment, within 60 hours after primary treatment, within 66 hours after primary treatment, within 72 hours after primary treatment, within 78 hours after primary treatment, within 84 hours after primary treatment, within 90 hours after primary treatment and within 96 hours after primary treatment. In other embodiments, within 120 hours or within 1 week.

Modes or administration of therapeutic agents identified herein are known to those of skill in the art. In one embodiment, one or more of the therapeutic agents may be administered by IV and/or orally. Further, one or more therapeutic agents may be combined and administered as a single composition or may be co-administered.

While determining therapeutic dosages of individual agents may be within the purview of a person of skill in the art, the inventor has found the following dosage regimen effective: initial (primary) anti-cancer treatment: surgery, chemotherapy, radiation (example: Gemcitabine); 18 hours later: anti-cancer stem cell treatment lasting for 48-96 hours (example: Metformin [2000 mg po twice daily]); 24 hours later :blocking tissue (tumor) repair response using combination of drugs, aspirin [81 mg po daily], oseltamivir phosphate [4 mg/kg IV infusion daily] and continue for at least 72-96 hours.

It will be understood that numerous modifications thereto will appear to those skilled in the art. Accordingly, the above description and accompanying drawings should be taken as illustrative of the invention and not in a limiting sense. It will further be understood that it is intended to cover any variations, uses, or adaptations of the invention following, in general, the principles of the invention and including such departures from the present disclosure as come within known or customary practice within the art to which the invention pertains and as may be applied to the essential features herein before set forth, and as follows in the scope of the appended claims.

The embodiments of the invention described above are intended to be exemplary only. The scope of the invention is therefore intended to be limited solely by the scope of the appended claims.

EXAMPLES

Example 1

***Delaying Chemotherapy After Tumour Removal Will Lead to Increase in Cancer Stem Cells***

After potentially curative surgical removal of a primary tumour and no radiographic evidence of metastatic disease any surviving cancer cells will be within immature tissue micrometastatic deposits or the circulation. The surgical removal of a variety of different tumours in animal models was shown to have a growth stimulating effect on cancer cells within evident metastatic deposits in seminal studies by Fisher and colleagues.

The increase in proliferation was noted at approximately 24 hours after surgery and lasted for approximately five to seven days. This increase varied from cancer type, but all cancers showed a significant increase from baseline after tumour removal. After this transient increase, cancer cell proliferation fell to the levels noted prior to surgical removal of the primary tumour.

Stem cells, whether cancer stem cells or tissue specific stem cells, are generally dormant or non-proliferating. When they are proliferating, they can divide asymmetrically (one stem cell and one daughter cell) or symmetrically (two stem cells).

After tumour removal an increased number of cancer cells in extant metastatic foci transited into a cycling mode. The percentage of cells transiting into a cycling mode was not increased by administering higher doses of serum from animals with removed tumours. The present inventor is therefore of the view that a fixed number of cells in these foci are able to respond to the growth factor of factor(s) released after tumour removal.

Both normal and neoplastic cells can spontaneously dedifferentiate to a stem cell phenotype. This phenotypic transformation permits these cells to behave like stem cells. In the case of cancer stem cells, these behaviors include resistance to conventional medical treatments and a heightened ability to metastasize and colonize tissue. Neoplastic cells appear particularly susceptible to this de-differentiation process as a result of the genetic instability inherent in the neoplastic phenotype.

Cells that have been experimentally manipulated to undergo the process of epithelial-mesenchymal transition or EMT can also revert to a stem cell phenotype. The process of wound healing/tissue repair has been shown to release a number of growth factors that can trigger an EMT like process in normal and neoplastic epithelial cell populations under various physiologic states. Because of the known association between partial EMT induction and wound healing, systemic tissue repair response triggered by any cancer treatment, including primary tumour removal, will accelerate the observed spontaneous rate of dedifferentiation of a cancer cell population to a stem cell phenotype. Both chemotherapy treatment and radiation treatment will foster stem cell enrichment of a residual cancer cell population rapidly after treatment. The enrichment for cancer stem cells in response to chemotherapy or radiation therapy is the result of tissue damage signals inducing both stem cell proliferation and the induction of a partial EMT in a residual cancer cell population. A similar response will be triggered after the surgical removal of the primary tumour.

The present inventor modelled the growth of a residual cancer cell population after surgery.

Assume that the cancer cell population remaining after surgery is 100,000 cancer cells, which is the number of cells estimated to reside in a tiny one millimetre micrometastatic deposit.

*NO = Population of cancer cells remaining after surgical removal of the primary tumour (One hundred thousand cancer cells).*

This cancer cell population will come under the influence of the inflammatory events triggered by primary tumour removal as discussed above. In the experiments by Fisher the fraction of the cancer cell population transiting into a cycling mode reached approximately 40% in some of the tumour types studied. We denote this as $r_1$=40%. Cell cycle time for cancer cells undergoing cell division is approximately 24 hours. The duration of time for the acute increase in proliferation was approximately six days (t*=6; days 1-6). We denote the total number of cancer cells at time t as N (t), note this includes all the cancer cells, including the stem cell like or non-stem cell like cancer cells. Hence we have the following equation for the growth of a residual cancer cell population during the initial six days following surgical resection of the primary tumour:

$$N\,(t) = N_0\,(1+r_1)^t, \quad t \leq t^*$$

From day 7 to day 30 the rate of proliferation can be expected to fall to normal values for micrometastases. It is difficult to precisely quantify this rate, which varies according to tumour type, but an average estimate would be a tumour doubling time of 30 days and therefore the fraction of cells proliferating during this time would be approximately 2.33% ($r_2$=0.0233). Assuming the same cycle time a similar equation can be used to estimate the growth of this cellular population from day 7 to day number 30.

$$N\,(t) = N_6\,(1+r_2)^{t-6}, \quad t > t^*$$

Second, we model the growth of the stem cell fraction. We assume symmetrical division of a residual cancer stem cell population rapidly after surgery based on the observation that tissue damage triggers symmetrical stem cell division of normal tissue stem cells acutely after tissue injury. We choose day one to day six for symmetrical stem cell division based on the increased proliferation period noted above. Similarly, we assume asymmetrical stem cell division from day seven to day thirty as the proliferation of an extant cancer cell population falls to normal levels after the initial acute increase noted after surgery.

Finally, the rate of dedifferentiation of more differentiated cancer cells to a cancer stem cell phenotype can be approximated. The spontaneous conversion rate of the non-neoplastic population was .0170 per cellular division, while it was much higher in the neoplastic population of .0025. For the sake of our model we will use the lower conversion rate of .0170 from day seven to day thirty and the higher conversion rate of .0025 per cell division acutely after surgery (six days).

Given these assumptions, the growth of a residual cancer cell population after surgery can be expressed mathematically within these two simultaneous equations:

$$N'\,(t) = N'\,(t\text{-}1)\,(1+r_1) \quad + \mu_1\,[N\,(t\text{-}1)\text{-}N'\,(t\text{-}1)], \quad t \leq t^*$$

$$N'\,(t) = N'\,(t\text{-}1)\,(1+r_2/2) \quad + \mu_2\,[N\,(t\text{-}1)\text{-}N'\,(t\text{-}1)] \quad t > t^*$$

(N' (t) is the number of cancer stem cell at day t. The two terms in the equation are from self-renewal of stem cells and the dedifferentiation of more differentiated cancer cells to a stem cell phenotype; $\mu_1$ =0.017 and $\mu_2$ =0.0025 are the conversion rates of more differentiated cancer cells to a stem cell phenotype during stage 1 (day 1 to 6) and stage 2 (day 7 to 30) respectively).

The increase in the stem cell fraction of the residual cancer cell population after surgery can be expressed as: *F(t) =100 N' (t)/N (t)*.

Similarly, the absolute increase in the number of cancer stem cells N' (t) and more differentiated cancer cells N (t) is presented in Table 2.

Table 2

| Day | N' (t) | N(t)-N'(t) | N(t) | F(t) |
|---|---|---|---|---|
| Day 0 | 1000 | 99000 | 100000 | 1 |
| Day 1 | 1839 | 138161 | 140000 | 1.313071 |
| Day 3 | 4923 | 191077 | 196000 | 2.511413 |
| Day 4 | 10140 | 264260 | 274400 | 3.695203 |
| Day 5 | 18688 | 365472 | 384160 | 4.864618 |
| Day 6 | 32376 | 505447 | 537824 | 6.019833 |
| Day 7 | 53919 | 699034 | 752953 | 7.161021 |
| Day 10 | 62107 | 744879 | 806986 | 7.696139 |
| Day 15 | 77504 | 828290 | 905794 | 8.556441 |
| Day 20 | 95354 | 921346 | 1016700 | 9.378803 |
| Day 25 | 116000 | 1025186 | 1141186 | 10.1649 |
| Day 30 | 139828 | 1141085 | 1280913 | 10.91632 |
| N' (t) is equal to number of cancer stem cells and N (t) is equal to total number of cancer cells after surgery. | | | | |

Given the assumptions of our model, an exponential increase in a residual cancer cell population for cancer stem cells will occur rapidly after surgical removal of the primary tumour and well before the time chemotherapy is now conventionally started.

Chemotherapy given rapidly after primary tumour removal could limit stem cell repopulation dramatically by killing off the bulk of the more differentiated cancer cells remaining after surgery. This would limit the number of cells capable of dedifferentiating to a stem cell phenotype, a process that is most responsible in our model for the increase in cancer stem cells after surgery.

Further, based on the model above, a residual cancer stem cell population is predicted to be actively proliferating within 24-48 hours of surgery under the influence of a systemic tissue repair response, thereby being more susceptible to many chemotherapy treatments.

The model predicts that a residual cancer stem cell population will grow exponentially during the time before the start conventional chemotherapy.

The rapid increase in treatment resistant cancer cells after surgery is due to a molecular plasticity inherent in cancer cells populations rendering them highly responsive to environmental perturbations induced by our clinical interventions timing of medical treatment after surgical removal of the primary tumour is critical for inhibiting disease recurrence and metastasis. Optimization of the potentially curative medical treatment of cancer requires that treatment be started rapidly after the primary tumour is removed to prevent repopulation of drug resistant and metastatically competent cancer stem cells.

Example 2

***The Distinct Cytokine Response After Surgical Removal of Tumours***

Specimens for cytokine testing were collected by aseptic technique into EDTA tubes. Specimens from surgical patients were collected at eight intervals - before surgery, after surgery (while the patient was in recovery), at 24 hours, 48 hours, 72 hours, 1 week, 2 weeks and 4 weeks after surgery.

EDTA samples were centrifuged within 30 minutes of collection, plasma was removed and then recentrifuged. Plasma was then aliquoted into cryotubes and stored in a -80°C freezer. On the morning of testing, cryotubes containing an aliquot of plasma from designated patients were placed into the 4°C refrigerator to thaw, then vortexed and recentrifuged for 5 minutes at 10,000g. Testing was performed immediately after. Levels of various cytokines were measured at the indicated time points.

Results of tests performed using EMD Millipore kits were read on the Luminex 200 analyzer. This flow cytometer based instrument integrates key detection components, such as lasers, optics, advanced fluidics and high speed digital signal processors. The multiplex technology is capable of performing a variety of bioassays including immunoassays on the surface of fluorescent coded magnetic beads known as MagPlex ™_ C microspheres. Results are quantified based on fluorescent reporter signals.

The surgical removal of three different tumour types - Colorectal, Breast, Prostate - triggers a statistically significant decline after surgery in the levels of TGF-beta and PDGF-BB at twenty four (24) hours after surgery. This is followed by a rapid increase back to baseline or even increased levels of TGF-beta and PDGF-BB. This, in turn is associated with a reciprocal statistically significant upregulation in the levels of IL-6, HGF, MCP-1, MMP-9 and PGF. This upregulation occurs at approximately the 24 hour mark after surgery and tends back to baseline within approximately one week of surgery.

As furthered detailed in Example 3, applying these distinct cytokines/growth factors alone or in combination to cancer cells in culture at levels detectable in the serum of patients after surgery, either from established cancer cell lines or acquired from cultured circulating tumour cells, triggers an increase in the fraction of the cells that are proliferating; facilitates epithelial-mesenchymal transition; and demonstrates an increase in the stem cell fraction as compared to cell lines not exposed to these molecules. This evidences that the upregulation in these distinct molecules after surgical removal of a primary tumour can facilitate an increase in a residual cancer cell population for cancer stem cells rapidly after surgical removal of a primary tumour and within one week of surgery. Blocking these upregulated molecules at the time they are predicted to be upregulated may prevent stem cell reproduction of a surviving cancer cell population after surgery.

Table 3 shows that IL-6 is significantly upregulated after surgery, and at Day 1 and Day 2 after surgery.

Table 3: significant upregulation of IL-6 after surgery, at Day 1 and Day 2 after surgery.

| IL-6 | Before | After | D1 | D2 | D3 | D7 | D14 | D28 |
|---|---|---|---|---|---|---|---|---|
| Patient 1 | 3.33 | 2.77 | 4.13 | 2.14 | 1.48 | 27.66 | 1.7 | 1.45 |
| Patient 2 | 2.21 | 28.97 | 46.71 | 28.64 | 30.86 | 38.56 | 29.18 | 12.19 |
| Patient 3 | 60.45 | 78.18 | 97.91 | 71.85 | 69.74 | 45.97 | 43.51 | 49.12 |
| Patient 4 | 7.73 | 5.29 | 8.71 | 6.27 | 4.09 | 3.99 | 4.37 | 4.06 |
| Patient 6 | 3.27 | 4.71 |  | 7 | 4.79 | 5.11 | 20.83 | 1.96 |
| Patient 7 | 0.67 | 17.77 | 28.82 | 9.35 | 4.73 | 4.23 | 1.74 | 1.92 |
| Patient 8 | 7.29 | 12.35 | 60.27 | 26.16 | 10.97 | 33.81 | 102.1 | 9.61 |
| Patient 9 | 44.3 | 94.42 | 119.96 | 204.65 | 90.32 | 27.73 | 35.48 | 35.77 |
| Patient 10 | 2.48 | 1.88 | 4.3 | 2.8 | 2.59 | 1.85 | 2.25 | 1.33 |
| Patient 11 | 10.56 | 2.18 | 2.04 | 1.2 | 0.43 | 0.42 | 0.51 | 0.38 |
| Patient 13 | 21.25 | 47.33 | 129.83 | 25.7 | 9.17 | 12.05 | 4.93 | 24.87 |
| Patient 14 | 1.91 | 3.97 | 6.43 | 19.92 | 21.38 | 7.56 | 1.75 | 3.88 |
| Patient 15 | 0.44 | 23.96 | 35.72 | 21.74 | 10.94 | 4.9 | 0.84 | 0.77 |
| Patient 16 | 0.92 | 6.75 | 8.59 | 2.45 | 1.43 | 1.33 | 1.09 | 1.31 |
| Patient 17 | 31.85 | 22.26 | 7.01 | 6.47* | 6.17* | 41.88 | 27.82 | 14.23 |
| Patient 19 | 57.37 | 62.4 | 210.85 | 90.83 | 31.88 | 18.9 | 74.59 | 57.16 |
| Patient 20 | 1.82 | 5.06 | 6.52 | 6.76 | 3.66* | 3.12 | 1.67 | 1.67 |
| Patient 21 | 17.21 | 71.16 | 104.72 | 38.45 | 16.26 | 25.07 | 24.41 | 35.51 |
| Patient 22 | 8.89 | 48.77 | 49.85 | 31.68 | 44.45 | 34.89 | 12.62 | 9.07 |
| Patient 23 | 3.34 | 2.67 | 2.09 | 5.1 | 1.91 | 2.3 | 1.49 | 0.79 |
| Patient 25 | 4.37 | 12.36 | 13.1 | 169.19 | 99.28 | 32.04 | 11.4 | 1.24 |
| Patient 26 | 15.64 | 8.73 | 17.03 | 11.42 | 6.48 | 9.04 | 9.09 | 7.62 |
| Patient 27 | 5.21 | 36.36 | 97.24 | 19.75 | 8.96 | 1.73 | 1.2 | 3.55 |
| Patient 29 | 1.72 | 76.16 | 40.94 | 18.94 | 7.97 | 1.45 | 3.3 | 1.51 |
| Patient 30 | 1.07 | 1.48 | 2.87 | 1.82 | 1.41 | 1.18 | 1.129 | 1.05 |
| Patient 31 | 3.17 | 4.24 | 3.5 | 3.63 | 2.42 | 2.79 | 2.97 | 2.64 |
| Patient 33 | 1.04 | 2.17 | 35.45 | 10.53 | 6.87 | 1.12 | 7.69 | 7.32 |

(continued)

| IL-6 | Before | After | D1 | D2 | D3 | D7 | D14 | D28 |
|---|---|---|---|---|---|---|---|---|
| Patient 34 | 22.23 | 31.49 | 23.99 | 27.81 | 22.37 | 13.89 | 17.06 | 18.28 |
| Patient 35 | 6.83 | 18.8 | 77.08 | 40.2 | 14.3 | 10.51 | 9.55 | 8.79 |
| | | | | | | | | |
| P values IL-6, n=29 (p<0.05 = statistically significant (*)): after surgery vs. before surgery = 0.00073* (up); Day 1 vs. before surgery = 0.000174* (up); Day 2 vs. before surgery = 0.009* (up); Day 3 vs. before surgery = 0.07517; Day 7 vs. before surgery = 0.21122; Day 14 vs. before surgery = 0.16019; Day 28 vs. before surgery = 0.20538 | | | | | | | | |

Table 4 shows that HGF is significantly upregulated after surgery and at Day 1, Day 2, Day 3 and Day 7 after surgery.

Table 4. Significant upregulation of HGF after surgery and at Day 1, Day 2, Day 3 and Day 7 after surgery.

| HGF | Before | After | D1 | D2 | D3 | D7 | D14 | D28 |
|---|---|---|---|---|---|---|---|---|
| Patient 1 | 57.08 | 60.35 | 32.26 | 65.45 | 94.61 | 53.92 | 112.74 | 94.61 |
| Patient 2 | 113.81 | 145.37 | 251.71 | 60.02 | 116.5 | 91.29 | 96.04 | 77.86 |
| Patient 3 | 175.38 | 262.14 | 277.62 | 259.95 | 363.18 | 382.47 | 288.82 | 284.32 |
| Patient 4 | 151.78 | 34.62 | 175.38 | 74.48 | 65.45 | 151.78 | 103.44 | 67.2 |
| Patient 5 | 28.9 | 18.35 | 20.07 | 39.64 | | 37.08 | 43.66 | 34.62 |
| Patient 6 | 358.23 | 379.64 | | 465.75 | 514.95 | 415.16 | 445.89 | 342.39 |
| Patient 7 | 119.22 | 266.28 | 326.75 | 319.01 | 374.26 | 454.37 | 164.51 | 148.47 |
| Patient 8 | 218.56 | 222.48 | 3557 | 841.2 | 431.85 | 363.55 | 503.26 | 226.45 |
| Patient 9 | 234.51 | 271.19 | 841.2 | 1091 | 424.05 | 201.08 | 144.16 | 118.73 |
| Patient 10 | 239.48 | 194.38 | 194.38 | 295.59 | 221.49 | 232.19 | 251.71 | 263.84 |
| Patient 11 | 15.4 | 17.54 | 21.49 | 15.4 | 19.85 | 12.81 | 15.4 | 15.4 |
| Patient 12 | 94.16 | 225.04 | 2979 | 118.73 | 349.81 | | | |
| Patient 13 | 52.07 | 69.48 | 962.84 | 629.84 | 497.46 | 43.84 | 25.95 | 36.46 |
| Patient 14 | 317.46 | 409.33 | 870.03 | 797.05 | 1132 | 814.42 | 526.17 | 296.6 |
| Patient 15 | 136.21 | 149.61 | 322.77 | 372.05 | 407.12 | 518.26 | 182 | 123.4 |
| Patient 16 | 195.79 | 94.91 | 77.06 | 83.95 | 70.48 | 68.89 | 70.48 | 55.45 |
| Patient 17 | 112.76 | 134.58 | 151.14 | 119.08 | 112.76 | 123.4 | 156.06 | 151.14 |
| Patient 18 | 146.3 | 271.62 | 415.61 | 373.62 | 392.38 | 64.23 | 146.3 | 82.2 |
| Patient 19 | 70.48 | 80.47 | 269.7 | 240.55 | 153.59 | 112.76 | 176.64 | 231.19 |
| Patient 20 | 260.63 | 203.06 | 767.9 | 243.34 | 222.64 | 193.69 | 167.19 | |
| Patient 21 | 112.76 | 256.68 | 469.44 | 347.48 | 336.76 | 382.47 | 178.67 | 112.23 |
| Patient 22 | 140.53 | 164.4 | 687.26 | 2307 | 2311 | 689.9 | 215.99 | 167.19 |
| Patient 23 | 215.99 | 262.61 | 351.8 | 209.46 | 219.3 | 190.63 | 187.6 | |
| Patient 25 | 82.2 | 193.69 | 343.18 | 1803 | 894.51 | 369.23 | 105.75 | |
| P values HGF, n=24 (p<0.05 = statistically significant (*)): after surgery vs. before surgery = 0.0228 (up); Day 1 vs. before surgery = 0.00724* (up); Day 2 vs. before surgery = 0.00591* (up); Day 3 vs. before surgery = 0.00742* (up); Day 7 vs. before surgery = 0.00775* (up); Day 14 vs. before surgery = 0.05204; Day 28 vs. before surgery = 0.41475 | | | | | | | | |

Table 5 shows that MCP-1 is significantly upregulated after surgery and at Day 2, and Day 3 after surgery.

Table 5. Significant upregulation of HGF after surgery and at Day 2 and Day 3 after surgery.

| MCP-1 | Before | After | D1 | D2 | D3 | D7 | D14 | D28 |
|---|---|---|---|---|---|---|---|---|
| Patient 1 | 346.89 | 386.79 | 338.11 | 658.54 | 400.59 | 495.15 | 377.2 | 452.69 |
| Patient 2 | 245.05 | 278.48 | 222.31 | 326.91 | 319.9 | 263.51 | 261.16 | 223.16 |
| Patient 3 | 720.15 | 988.56 | 733.58 | 773.2 | 693.77 | 1093 | 773.36 | 660.41 |
| Patient 4 | 399.35 | 424.88 | 335.95 | 417.92 | 385.25 | 390.62 | 521.83 | 490.32 |
| Patient 5 | 791.02 | 601.99 | 384.1 | 885.88 | 1438 | 1443 | 988.78 | 1008 |
| Patient 6 | 353.11 | 848.83 |  | 358.2 | 820.34 | 413.02 | 900.29 | 391.19 |
| Patient 7 | 221.63 | 281.45 | 336.88 | 426.41 | 287.8 | 292.87 | 268.97 | 256.05 |
| Patient 8 | 1247 | 1065 | 1234 | 1039 | 911.14 | 879 | 1095 | 1218 |
| Patient 9 | 579.35 | 826.58 | 548.48 | 783.86 | 477.02 | 370.25 | 346.55 | 496.7 |
| Patient 10 | 471.54 | 487.21 | 232.01 | 572.48 | 473.6 | 373.75 | 357.58 | 394.98 |
| Patient 11 | 96.18 | 38.65 | 81.3 | 107.47 | 75.94 | 76.05 | 68.32 | 71.83 |
| Patient 12 | 261.6 | 453.35 | 270.28 | 286.96 | 1688 |  |  |  |
| Patient 13 | 397.11 | 445.47 | 713.19 | 494.76 | 374.2 | 450.79 | 406.99 | 469.9 |
| Patient 14 | 480.3 | 296.73 | 398.17 | 1105 | 737.71 | 419.47 | 370.87 | 391.7 |
| Patient 15 | 268.82 | 417.46 | 169.04 | 234.65 | 258.49 | 366.84 | 246.1 | 278.35 |
| Patient 16 | 284.55 | 381.46 | 302.93 | 328.38 | 286.67 | 313.49 | 376.19 | 371.72 |
| Patient 17 | 351.37 | 385.26 | 300.66 | 336.36 | 320.92 | 300.58 | 397.48 | 362.3 |
| Patient 18 | 801.55 | 786.31 | 781.41 | 906.76 | 969.83 | 842.96 | 856.74 | 819.62 |
| Patient 19 | 446.7 | 558.04 | 557.59 | 747.84 | 448.57 | 551.95 | 565.65 | 504.45 |
| Patient 20 | 598.71 | 761.82 | 389.14 | 572.5 | 557.08 | 406.69 | 477.34 |  |
| Patient 21 | 379.56 | 530.44 | 929.07 | 452.14 | 340.1 | 835.14 | 391.5 | 379.87 |
| Patient 22 | 371.72 | 367.97 | 398.92 | 467.43 | 589.8 | 389.9 | 338.33 | 377.84 |
| Patient 23 | 425.74 | 364.69 | 780.98 | 841.53 | 406.13 | 343.35 | 304.04 |  |
| Patient 25 | 142.38 | 95.91 | 148.38 | 706.81 | 423.22 | 189.78 | 180.08 |  |
| Average | 445.0575 | 503.0554 | 464.2383 | 576.2913 | 570.1696 | 500.0504 | 472.6239 | 480.954 |

P values MCP-1, n=24 ($p < 0.05$ = statistically significant (*)): after surgery vs. before surgery = 0.03966* (up); Day 1 vs. before surgery = 0.35371; Day 2 vs. before surgery = 0.00142* (up); Day 3 vs. before surgery = 0.0423* (up); Day 7 vs. before surgery = 0.15314; Day 14 vs. before surgery = 0.27265; Day 28 vs. before surgery = 0.14261

Table 6 shows that MMP-9 is significantly upregulated at after surgery and at Day 1 after surgery.

Table 6. Significant upregulation of MMP-9 after surgery and at Day 1 after surgery.

| MMP-9 | Before | After | D1 | D2 | D3 | D7 | D14 | D28 |
|---|---|---|---|---|---|---|---|---|
| Patient 1 | 30334 | 22839 | 20301 | 40973 | 14577 | 32027 | 32401 | 26151 |
| Patient 2 | 45241 | 90898 | 136613 | 30004 | 42378 | 35080 | 34161 | 26296 |
| Patient 3 | 71190 | 113651 | 111192 | 118515 | 42018 | 108063 | 64622 | 41137 |
| Patient 4 | 44246 | 98711 | 94154 | 39469 | 21053 | 59246 | 66047 | 37687 |
| Patient 5 | 131755 | 80150 | 97816 | 127227 |  | 134722 | 163760 | 86542 |
| Patient 6 | 74472 | 135561 |  | 74110 | 138191 | 73375 | 123812 | 78098 |

(continued)

| MMP-9 | Before | After | D1 | D2 | D3 | D7 | D14 | D28 |
|---|---|---|---|---|---|---|---|---|
| Patient 7 | 59144 | 208787 | 100312 | 85512 | 114732 | 45942 | 65442 | 80702 |
| Patient 8 | 18591 | 33530 | 11658 | 7767 | 12198 | 34206 | 104099 | 32356 |
| Patient 9 | 16188 | 70639 | 15865 | 67304 | 14463 | 41384 | 31427 | 16144 |
| Patient 10 | 43867 | 81341 | 79670 | 32528 | 40120 | 44362 | 23861 | 24900 |
| Patient 11 | 15912 | 70977 | 48904 | 26991 | 37247 | 24229 | 19088 | 25812 |
| Patient 12 | 85221 | 260288 | 30219 | 17246 | 83729 | | | |
| Patient 13 | 92784 | 242844 | 161006 | 45267 | 51178 | 4864 | 46121 | 83203 |
| Patient 14 | 87265 | 106362 | 47663 | 109945 | 85300 | 122075 | 97364 | 48542 |
| Patient 15 | 47770 | 61415 | 48046 | 31258 | 38451 | 50022 | 33181 | 20721 |
| Patient 16 | 40533 | 69646 | 26255 | 20285 | 20804 | 31413 | 25980 | 15726 |
| Patient 17 | 92684 | 144133 | 122538 | 74234 | 75484 | 76795 | 164105 | 110434 |
| Patient 18 | 101130 | 146579 | 29451 | 38215 | 77079 | 121520 | 76035 | 58381 |
| Patient 19 | 13268 | 23670 | 20954 | 12659 | 19223 | 16815 | 16891 | 14275 |
| Patient 20 | 104610 | 117682 | | 59977 | 67947 | 87903 | 66984 | |
| Patient 21 | 46298 | 181529 | 60010 | 29982 | 54875 | 121762 | 62572 | 48526 |
| Patient 22 | 30655 | 91081 | 35622 | 22039 | 60140 | 38225 | 37163 | 44286 |
| Patient 23 | 91006 | 158650 | 179892 | 64994 | 62357 | 55323 | 78385 | |
| Patient 25 | 37102 | 147097 | 78888 | 50225 | 103001 | 47110 | 54059 | |
| Average | 59219.42 | 114919.2 | 70774.05 | 51113.58 | 55501.96 | 64194.04 | 64676.52 | 45995.95 |
| P values MMP-9, n=24 (p<0.05 = statistically significant (*)): after surgery vs. before surgery = 2.03767E-05* (up); Day 1 vs. before surgery = 0.066421* (up); Day 2 vs. before surgery = 0.09712; Day 3 vs. before surgery = 0.46457; Day 7 vs. before surgery = 0.10848; Day 14 vs. before surgery = 0.16279; Day 28 vs. before surgery = 0.03068* (down) | | | | | | | | |

Table 7 shows that PGF levels are upregulated after surgery and at Day 1, Day 2, Day 3, Day 7, Day 14 and Day 28 after surgery.

Table 7. Significant upregulation of of PGF levels at Day 1 after surgery and at Day 1, Day 2, Day 3, Day 7, Day 14 and Day 28 after surgery.

| PGF | Before | After | D1 | D2 | D3 | D7 | D14 | D28 |
|---|---|---|---|---|---|---|---|---|
| Patient 1 | 2.82 | 2.91 | 2.01 | 2.23 | 4.09 | 4.69 | 4.42 | 3.31 |
| Patient 2 | 4.31 | 5.35 | 1.23 | 3.54 | 6.35 | 4.78 | 4.15 | 4.62 |
| Patient 3 | 1.15 | 1.28 | 2.91 | 2.27 | 2.62 | 2.86 | 1.95 | 4.69 |
| Patient 4 | 2.38 | 0.61 | 2.62 | 2.16 | 1.65 | 5.22 | 3.08 | 2.16 |
| Patient 5 | 1.44 | 1.56 | 2.91 | 5.52 | | 3.22 | 3.54 | 1.62 |
| Patient 6 | 12.28 | 12.74 | | 13.94 | 15.7 | 14.03 | 16.92 | 11.73 |
| Patient 7 | 609 | 3.39 | 9.03 | 7.63 | 3.54 | 4.94 | 4.86 | 9.66 |
| Patient 8 | 9.12 | 14.03 | 16.82 | 11.55 | 13.38 | 22.56 | 18.79 | 15.7 |
| Patient 9 | 11.73 | 11.92 | 16.08 | 12.19 | 8.65 | 11.17 | 7.65 | 6.44 |
| Patient 10 | 2.54 | 1.39 | 0.73 | 2.86 | 3.74 | 2.19 | 7.46 | 6.94 |
| Patient 11 | 0.72 | 0.72 | 0.99 | 0.91 | 1.11 | 1.08 | 1.02 | 0.86 |

(continued)

| PGF | Before | After | D1 | D2 | D3 | D7 | D14 | D28 |
|---|---|---|---|---|---|---|---|---|
| Patient 12 | 3.65 | 6.44 | 3.99 | 16.39 | 69.47 | | | |
| Patient 13 | 13.75 | 13.26 | 18.55 | 14 | 13.02 | 12.94 | 6.35 | 9.98 |
| Patient 14 | 2.7 | 3.46 | 6.35 | 18.92 | 12.7 | 6.83 | 5.71 | 7.03 |
| Patient 15 | 4.96 | 7.76 | 18.64 | 12.86 | 10.31 | 17.52 | 11.53 | 16.18 |
| Patient 16 | 14.65 | 6.52 | 15.79 | 7.83 | 9.62 | 10.43 | 9.4 | 13.68 |
| Patient 17 | 4 | 3.38 | 6.35 | 4.07 | 5.92 | 4.61 | 2.94 | 5.13 |
| Patient 18 | 9.18 | 6.52 | 13.9 | 12.95 | 4.61 | 13.17 | 13.46 | 14.57 |
| Patient 19 | 5.44 | 3.16 | 14.35 | 10.91 | 12.51 | 10.43 | 9.4 | 12.66 |
| Patient 20 | 7.56 | 8.89 | 9.86 | 10.88 | 8.53 | 8.71 | 11.09 | |
| Patient 21 | 0.76 | 0.72 | 2.22 | 1.95 | 1.35 | 1.95 | 0.85 | 0.85 |
| Patient 22 | 6.36 | 4.38 | 15.35 | 16.69 | 19.06 | 18.73 | 11.52 | 11.09 |
| Patient 23 | 8.99 | 6.21 | 3.53 | 9.86 | 8.26 | 7.48 | 10.26 | |
| Patient 25 | 8.44 | 15.89 | 5.91 | 28.7 | 11.96 | 16.69 | 16.13 | |
| Average | 6.0425 | 5.937083 | 8.266087 | 9.617083 | 10.78913 | 8.966522 | 7.933913 | 7.945 |
| P values PGF, n=24 (p<0.05 = statistically significant (*)): after surgery vs. before surgery = 0.43143; Day 1 vs. before surgery = 0.00596* (up); Day 2 vs. before surgery = 0.00394* (up); Day 3 vs. before surgery = 0.06693; Day 7 vs. before surgery = 0.00423 * (up); Day 14 vs. before surgery = 0.02303* (up); Day 28 vs. before surgery = 0.012592* (up) | | | | | | | | |

Table 8 shows that TGF-beta is significantly down-regulated at Day 1 after surgery.

Table 8. Significant down-regulation of TGF-beta at Day 1 after surgery.

| TGF-B | Before | After | D1 | D2 | D3 | D7 | D14 | D28 |
|---|---|---|---|---|---|---|---|---|
| Patient 1 | 3744 | 6471 | 3612 | 4602 | 4448 | 5411 | 4696 | 3793 |
| Patient 2 | 4101 | 9574 | 2555 | 6351 | 5191 | 4031 | 3461 | 3391 |
| Patient 3 | 5110 | 20387 | 13771 | 11451 | 6777 | 24502 | 14167 | 11457 |
| Patient 4 | 7370 | 8144 | 7014 | 8676 | 9775 | 6433 | 4626 | 4415 |
| Patient 5 | 8656 | 7232 | 4415 | 7814 | No spec | 15497 | 13421 | 8592 |
| Patient 6 | 13630 | 25299 | | 9438 | 15326 | 12677 | 20029 | 16594 |
| Patient 7 | 6028 | 4890 | 1401 | 1782 | 2089 | 2871 | 22252 | 9313 |
| Patient 8 | 2089 | 2953 | 1341 | 1627 | 2239 | 4303 | 8684 | 4281 |
| Patient 9 | 5164 | 5472 | 1807 | 7755 | 2027 | 4575 | 4082 | 1670 |
| Patient 10 | 3410 | 4735 | 4818 | 6188 | 7141 | | 5587 | 2239* |
| Patient 11 | 3678 | 4287 | 6232 | 4033 | 3281 | 2393 | 2675 | 3239 |
| Patient 12 | 4209 | 6096 | 3270 | 2027 | 2531 | | | |
| Patient 13 | 2139 | 2365 | 1783 | 1550 | 2738 | 5725 | 2701 | 5697 |
| Patient 14 | 5078 | 6147 | 1670 | 15923 | 4511 | 5500 | 8718 | 6552 |
| Patient 15 | 6547 | 3873 | 3078 | 1424 | 1997 | 4547 | 6372 | 4691 |
| Patient 16 | 5482 | 9694 | 3545 | 3667 | 4008 | 7427 | 4547 | 3730 |
| Patient 17 | 2029 | 2563 | 2214 | 2273 | 2764 | 1739 | 2728 | 2108 |
| Patient 18 | 7556 | 8084 | 1793 | 2789 | 6253 | 22060 | 17609 | 9217 |

(continued)

| TGF-B | Before | After | D1 | D2 | D3 | D7 | D14 | D28 |
|---|---|---|---|---|---|---|---|---|
| Patient 19 | 9402 | 11680 | 3793 | 12276 | 6337 | 8348 | 11030 | 12377 |
| Patient 20 | 5156 | 11213 | 8202 | 7606 | 10103 | 8658 | 7945 | 6196 |
| Patient 21 | 15937 | 6275 | 3493 | 2695 | 6226 | 21404 | 13383 | 8973 |
| Patient 22 | 9433 | 7789 | 5790 | 1949 | 3186 | 3146 | 5437 | 8488 |
| Patient 23 | 14817 | 10878 | 8056 | 8475 | 8938 | 5666 | 4562 | 5670 |
| Patient 25 | 4298 | 2441 | 1495 | 3156 | 6160 | 9128 | 5100 | 4209 |
| Patient 26 | 4512 | 6241 | 3320 | 5041 | 5546 | 5951 | 6266 | 7050 |
| Patient 27 | 3951 | 3236 | 2206 | 4904 | 4160 | 8310 | 3937 | 5593 |
| Patient 29 | 1508 | 3009 | 1828 | 716.1 | 566.59 | 1835 | 1610 | 2065 |
| Patient 30 | 10961 | 9165 | 5861 | 8274 | 8779 | 6180 | 3800 | 6176 |
| Patient 31 | 4552 | 12159 | 5721 | 5985 | 5106 | 4649 | 3483 | 7481 |
| Patient 33 | 12832 | 8633 | 2448 | 9966 | 11200 | 10371 | 11339 | 8215 |
| Patient 34 | 4785 | 2894 | 4356 | 2994 | 3571 | 8839 | 10484 | 6394 |
| Patient 35 | 3588 | 2756 | 714.53 | 1296 | 3378 | 1520 | 1995 | 1366 |
| | 6304.75 | 7394.844 | 3922.662 | 5459.566 | 5374.922 | 7789.867 | 7636.323 | 6299.767 |
| P values TGF-beta, n=32 (p<0.05 = statistically significant (*)): after surgery vs. before surgery = 0.0942; Day 1 vs. before surgery = 0.00251* (down); Day 2 vs. before surgery = 0.013586; Day 3 vs. before surgery = 0.06321; Day 7 vs. before surgery = 0.10053; Day 14 vs. before surgery = 0.08699; Day 28 vs. before surgery = 0.39017 | | | | | | | | |

Table 9 shows that PDGFBB levels are down-regulated at Day 1 after surgery.

Table 9. Significant down-regulation of PDGFBB levels at Day 1 after surgery.

| PDGFBB | Before | After | D1 | D2 | D3 | D7 | D14 | D28 |
|---|---|---|---|---|---|---|---|---|
| Patient 1 | 990.23 | 2865 | 1125 | 3465 | 1388 | 1160 | 1639 | 1023 |
| Patient 2 | 1125 | 3047 | 237.65 | 1309 | 837.88 | 456.84 | 320 | 516.83 |
| Patient 3 | 2424 | 16265 | 10458 | 9518 | 3139 | 25550 | 9585 | 7567 |
| Patient 4 | 1778 | 2620 | 2252 | 2753 | 2664 | 1798 | 958.37 | 958.37 |
| Patient 5 | 5115 | 3251 | 1685 | 3936 | | 14389 | 11141 | 6959 |
| Patient 6 | 7163 | 15197 | | 3304 | 12581 | 5933 | 13607 | 9532 |
| Patient 7 | 2367 | 1994 | 186.04 | 229.34 | 320 | 449.01 | 15393 | 3547 |
| Patient 8 | 365.06 | 723.74 | 139.91 | 123.79 | 176.12 | 826.07 | 5773 | 1155 |
| Patient 9 | 7197 | 5177 | 380.95 | 9167 | 652.67 | 3251 | 1600 | 586.33 |
| Patient 10 | 2402 | 4266 | 3632 | 5791 | 9205 | 867 | 4379 | 7154 |
| Patient 11 | 2452 | 2208 | 3033 | 1714 | 549.72 | 341.17 | 436.31 | 549.72 |
| Patient 12 | 1783 | 3882 | 490.61 | 170.29 | 341.17 | | | |
| Patient 13 | 299.82 | 839.96 | 197.74 | 87.62 | 1668 | 2280 | 262.28 | 2828 |
| Patient 15 | 6449 | 2256 | 1875 | 386.59 | 648.06 | 3426 | 4317 | 3137 |
| Patient 16 | 2787 | 7799 | 1088 | 1138 | 1919 | 4357 | 2132 | 1243 |
| Patient 17 | 340.67 | 1536 | 458.94 | 706.52 | 903.52 | 281.4 | 340.67 | 903.52 |
| Patient 18 | 2617 | 5585 | 263.43 | 781.12 | 2925 | 21090 | 7208 | 7015 |

(continued)

| PDGFBB | Before | After | D1 | D2 | D3 | D7 | D14 | D28 |
|---|---|---|---|---|---|---|---|---|
| Patient 19 | 9563 | 11867 | 2009 | 10745 | 2617 | 7899 | 13652 | 18985 |
| Patient 20 | 2105 | 5366 | 3483 | 2661 | 4585 | 3592 | 2888 | 2228 |
| Patient 21 | 10485 | 2339 | 646.68 | 456.88 | 1654 | 13233 | 4725 | 3702 |
| Patient 22 | 7763 | 5681 | 3924 | 333.71 | 1130 | 1990 | 1728 | 5294 |
| Patient 25 | 986.02 | 440.13 | 268.72 | 1526 | 3924 | 5318 | 1014 | 1092 |
| Patient 26 | 2209 | 3114 | 1366 | 2621 | 2325 | 2423 | 2462 | 2742 |
| Patient 27 | 1635 | 1635 | 348.28 | 2344 | 1834 | 5231 | 1330 | 2762 |
| Patient 29 | 857.99 | 2581 | 857.99 | 211.87 | 155.63 | 1689 | 831.39 | 1671 |
| Patient 30 | 11639 | 6949 | 3475 | 6212 | 5667 | 3891 | 1559 | 3605 |
| Patient 31 | 3128 | 10285 | 2500 | 3831 | 4183 | 3831 | 4678 | 6528 |
| Patient 33 | 9895 | 5331 | 561.17 | 5226 | 6048 | 5331 | 6268 | 2583 |
| Patient 34 | 2583 | 1545 | 1894 | 644.53 | 1545 | 7841 | 7960 | 1672 |
| Patient 35 | 1624 | 1153 | 254.42 | 142.46 | 2527 | 142.46 | 198.98 | 152.71 |
| Average | 3737.593 | 4593.261 | 1692.811 | 2717.857 | 2693.544 | 5133.343 | 4427.103 | 3713.499 |
| P values PDGFBB, n=30 (p<0.05 = statistically significant (*)): after surgery vs. before surgery = 0.13346; Day 1 vs. before surgery = 0.00455* (down); Day 2 vs. before surgery = 0.05306; Day 3 vs. before surgery = 0.07732; Day 7 vs. before surgery = 0.13632; Day 14 vs. before surgery = 0.24104; Day 28 vs. before surgery = 0.45003 | | | | | | | | |

Other cytokines were tested and the results are summarized here:

- IL-8 was statistically unchanged after surgery.
  P values IL-8, n=32 (p<0.05 = statistically significant (*)): after surgery vs. before surgery = 0.49204; Day 1 vs. before surgery = 0.18784; Day 2 vs. before surgery = 0.46249; Day 3 vs. before surgery = 0.36836; Day 7 vs. before surgery = 0.37866; Day 14 vs. before surgery = 0.1813; Day 28 vs. before surgery = 0.04077

- TNF-alpha levels were down-regulated at Day 1 and Day 2 after surgery.
  P values TNF-A, n=24 (p<0.05 = statistically significant (*)): after surgery vs. before surgery = 0.06717; Day 1 vs. before surgery = 0.0393* (down); Day 2 vs. before surgery = 0.05497* (down); Day 3 vs. before surgery = 0.2551; Day 7 vs. before surgery = 0.30798; Day 14 vs. before surgery = 0.63612; Day 28 vs. before surgery = 0.03266* (Down)

- FGF-2 levels were down-regulated at Day 1 after surgery.
  P values FGF-2, n=18 (p<0.05 = statistically significant (*)): after surgery vs. before surgery = 0.30268; Day 1 vs. before surgery = 0.01792* (down); Day 2 vs. before surgery = 0.06712; Day 3 vs. before surgery = 0.13529; Day 7 vs. before surgery = 0.41027; Day 14 vs. before surgery = 0.16193; Day 28 vs. before surgery = 0.16036

- HIGF-2 levels were down-regulated after surgery and at Day 1, Day 2, Day 3 and Day 7 after surgery.
  P values HIGF-1, n=23 (p<0.05 = statistically significant (*)): after surgery vs. before surgery = 0.00598* (down); Day 1 vs. before surgery = 0.02042* (down); Day 2 vs. before surgery = 0.00206* (down); Day 3 vs. before surgery = 0.00044* (down); Day 7 vs. before surgery = 0.00234* (down); Day 14 vs. before surgery = 0.28478; Day 28 vs. before surgery = 0.41181

- IL1B levels were down-regulated at Day 1 and Day 2 after surgery.
  P values IL1B, n=32 (p<0.05 = statistically significant (*)): after surgery vs. before surgery = 0.24645; Day 1 vs. before surgery = 0.0514* (down); Day 2 vs. before surgery = 0.04013* (down); Day 3 vs. before surgery = 0.29054; Day 7 vs. before surgery = 0.28257; Day 14 vs. before surgery = 0.219; Day 28 vs. before surgery = 0.1794

- IL1A levels were down-regulated after surgery and at Day 1, Day 2 and Day 3 after surgery.
  P values IL1A, n=22 (p<0.05 = statistically significant (*)): after surgery vs. before surgery = 0.01151* (down); Day 1 vs. before surgery = 0.01105* (down); Day 2 vs. before surgery = 0.00731* (down); Day 3 vs. before surgery = 0.009* (down); Day 7 vs. before surgery = 0.09992; Day 14 vs. before surgery = 0.29876; Day 28 vs. before surgery = 0.42089

- VEGF levels were down-regulated after surgery and at Day 1, Day 2, Day 3, Day 7, Day 14 and Day 28 after surgery. P values VEGF, n=30 ($p < 0.05$ = statistically significant (*)): after surgery vs. before surgery = 0.07035* (down); Day 1 vs. before surgery = 0.0816* (down); Day 2 vs. before surgery = 0.0288* (down); Day 3 vs. before surgery = 0.05952* (down); Day 7 vs. before surgery = 0.06271* (down); Day 14 vs. before surgery = 0.08025* (down); Day 28 vs. before surgery = 0.1007

- EGF levels were down-regulated at Day 1 and Day 2 after surgery. P values EGF, n=24 ($p < 0.05$ = statistically significant (*)): after surgery vs. before surgery = 0.0857; Day 1 vs. before surgery = 0.04162* (down); Day 2 vs. before surgery = 0.05083* (down); Day 3 vs. before surgery = 0.20491; Day 7 vs. before surgery = 0.07448; Day 14 vs. before surgery = 0.13942; Day 28 vs. before surgery = 0.07131

- MCP-2 levels are down-regulated at Day 1 and Day 2 after surgery. P values MCP-2, n=23 ($p < 0.05$ = statistically significant (*)): after surgery vs. before surgery = 0.24955; Day 1 vs. before surgery = 8.59162E-05* (down); Day 2 vs. before surgery = 0.00421* (down); Day 3 vs. before surgery = 0.05392; Day 7 vs. before surgery = 0.11152; Day 14 vs. before surgery = 0.45277; Day 28 vs. before surgery = 0.23071

- SDF1AB levels were down-regulated at Day 1 after surgery. P values SDF1AB, n=24 ($p < 0.05$ = statistically significant (*)): after surgery vs. before surgery = 0.22973; Day 1 vs. before surgery = 8.0633E-05* (down); Day 2 vs. before surgery = 0.29111; Day 3 vs. before surgery = 0.40954; Day 7 vs. before surgery = 0.29029; Day 14 vs. before surgery = 0.45502; Day 28 vs. before surgery = 0.38572

- PDGFAA levels were down-regulated at Day 1 and Day 3 after surgery. P values PDGFAA, n=32 ($p < 0.05$ = statistically significant (*)): after surgery vs. before surgery = 0.13228; Day 1 vs. before surgery = 0.00187* (down); Day 2 vs. before surgery = 0.26105; Day 3 vs. before surgery = 0.54894* (down); Day 7 vs. before surgery = 0.06234; Day 14 vs. before surgery = 0.26536; Day 28 vs. before surgery = 0.45594

In a limited number of patients, although not followed longitudinally, preliminary data exists to suggest that PGE-2 is upregulated over a similar time period to IL-6, HGF, PDGF, MCP-1 and MMP-9.

An additional 25 patients undergoing surgery for breast, colorectal, and prostate cancer were enrolled. Research efforts for these patients was focussed on the expression patterns of IL-6, HGF, and TGF-beta and a similar statistically significant change from baseline was observed as in the above data set.

Example 3

### *Inflammatory Cytokines Facilitate Cell Proliferation and EMT in Cancer Stem Cell Populations*

Specific cytokines or cytokine cocktails capable of inducing cell proliferation of cancer stem cell populations and/or triggering stem cell enrichment via Epithelial Mesenchymal Transition (EMT) and/or stem cell self renewal were identified.

Cell proliferation of cancer and/or cancer stem cell populations with selected cytokine and cytokine cocktails on serum starved colorectal carcinoma cell lines. Prior to incubation cells were stained with a cell proliferation dye, which binds stoichiometrically to DNA. Intensity of the dye decreased upon cell division thereby signifying cell proliferation.

For specific cytokine combinations, there was an enrichment of cells with cancer stem cell (CSC) phenotype. Imaging flow cytometry data show that cells incubated with cytokines were able to resume cell division in the absence of serum. Moreover, specific cytokine combinations achieved a high percentage of stem cell population of the total events collected. Data from enriched Circulating Tumour Cells (CTCs) from a patient and from cell lines support this observation.

### **Cell Proliferation Assays with Cytokine Cocktails**

Two authenticated colorectal carcinoma cell lines, HCT-15 and SW-620, were used to test the effect of different cytokine and cytokine cocktails, including IL-6, HGF, PGE-2, TGF-beta and MMP-9 alone or in a cocktail, on cell proliferation, EMT and cancer stem cell populations.

Table 21 shows the cytokine and cytokine cocktail treatments used in cell proliferation assays.

Table 21: Cytokine and Cytokine Cocktail Treatments for Cell Proliferation Assay

| Source of Cells | 2 Component Cocktail | 3 Component Cocktail | 5 Component Cocktail |
|---|---|---|---|
| SW620 | IL6+HGF | IL6+HGF+PGE2 | IL6+HGF+PGE2+TGF+ MMP9 |
| | IL6+PGE2 | IL6+HGF+TGF | |
| | IL6=MMP9 | IL6+PGE2+TGF | |
| | | IL6+PGE2+MMP9 | |
| | | MMP9+HGF+TGF | |

In order to evaluate stem cell populations, cells were synchronized by serum starvation for 72 hours before seeding in 24-well ultra low attachment plates at a density of 200,000 cells/well. Cells were then detached using trypsin EDTA and washed twice with PBS (without Calcium or Magnesium). A cell proliferation dye, eFluor 450 was used to label cells. Cells were grown with DMEM with high glucose and 2mM Gluatmine without FBS in the presence of selected cytokines and or cytokine cocktails over a period of 72 hrs.

Cells that were grown in 5% serum were stained with the following antibody cocktails: CD24FITC, CD44PE and CD24FITC, CD133PE. Cells were also stained with a fixable live/dead efluor dye 506 to exclude dead cells. In parallel, cells were grown in the presence of 5% serum to evaluate CSC markers.

Cells were fixed in 1% Paraformaldehyde for 15 minutes at room temperature and run on Flowsight using 2 lasers (488 nm at 60mW and 405nm at 30mW). For cell lines, 100,000 events were collected whilst for enriched CTCs all events were collected until the sample finished.

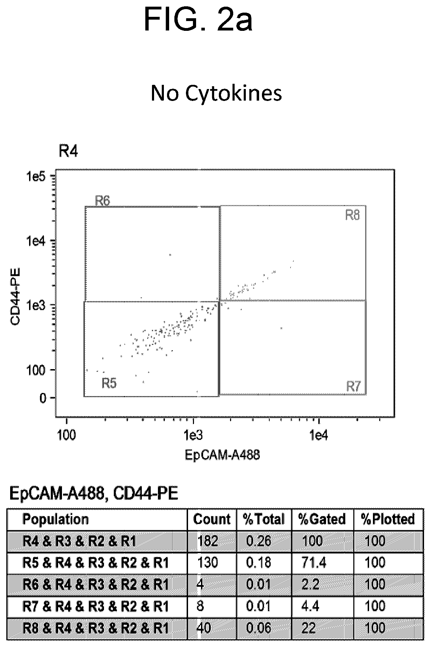

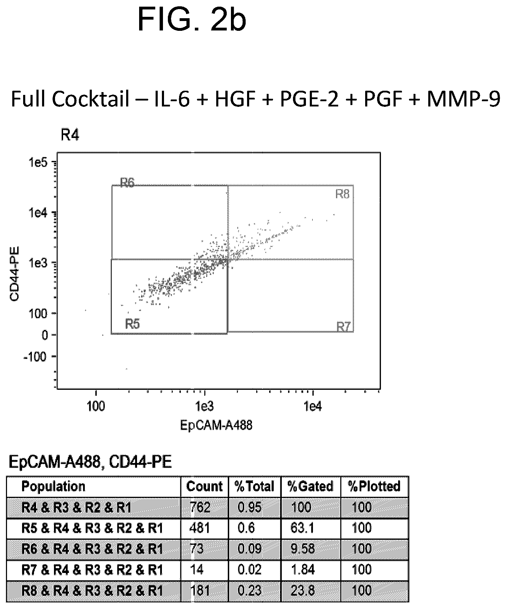

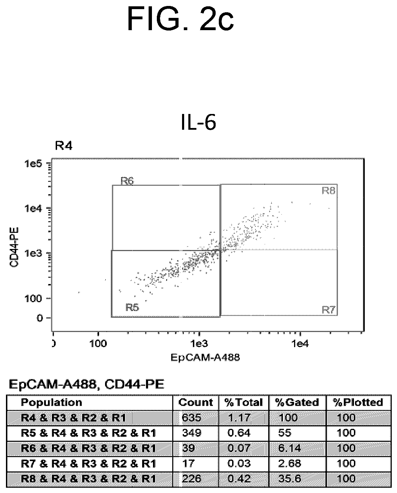

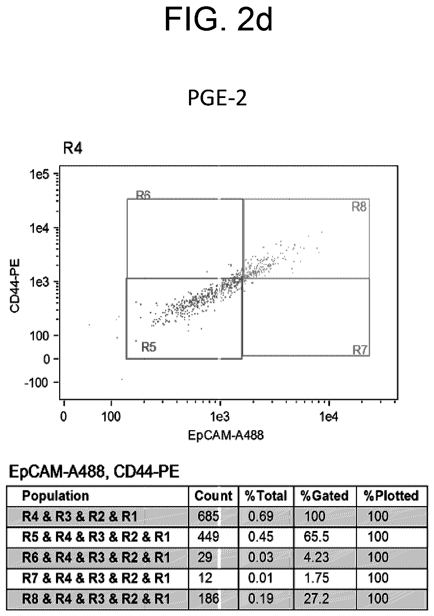

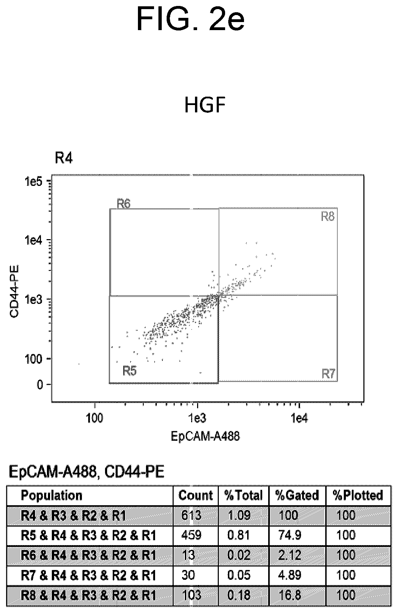

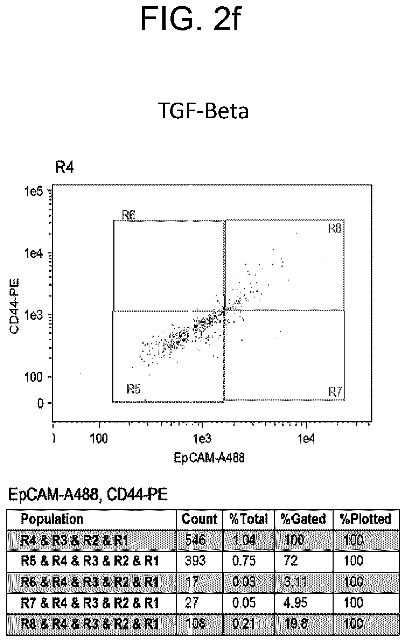

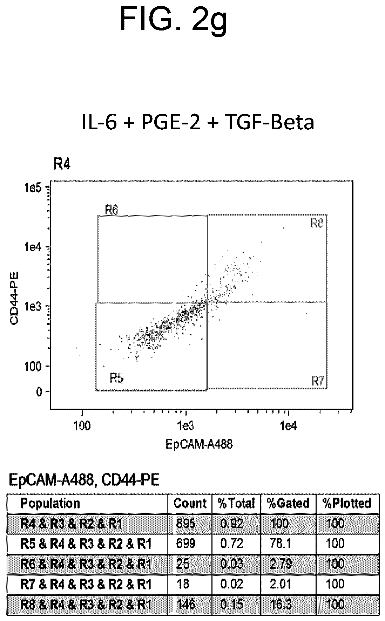

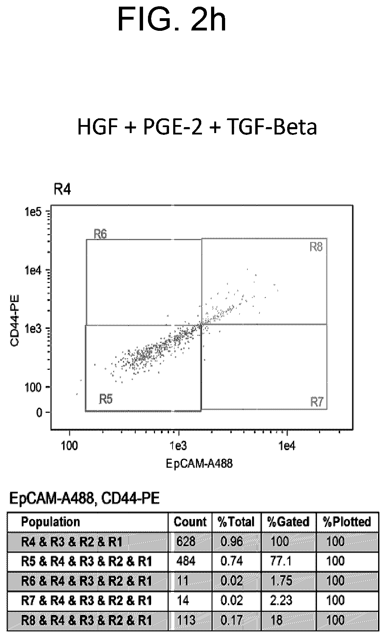

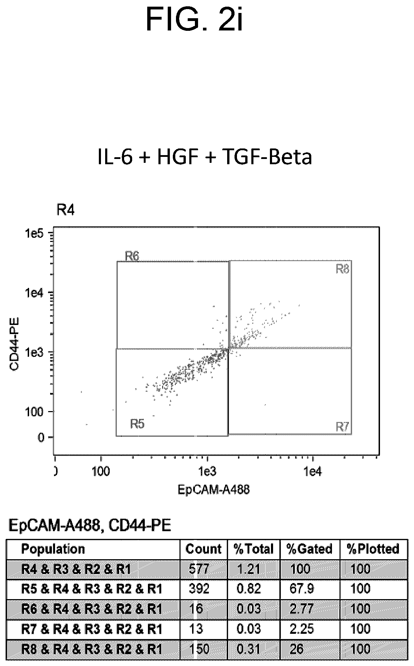

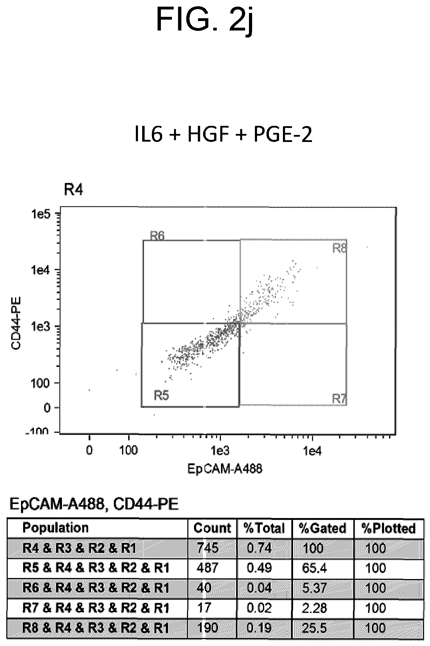

Flow cytometry data was analysed using IDEAS software. Double events, out of focus events, CD45PercPCy5.5 positive cells (in case of enriched CTCs), dead cells and non-nucleated events were eliminated from the fluorescence analysis of the cell population. Figure 2 depicts scatterplots of flow cytometry experiments showing the enriched CTCs stained with an antibody containing CD44 after treatment with various cytokines and cytokine cocktails. Hoescht 33258 dye was used to stain nuclei in the case of the HCT-15 cell line and enriched CTCs. All experiments were stained with a fixable live/dead dye eFluor 506 to exclude dead cells.

**CTC Enrichment and Cell Culture with Cytokine Cocktails**

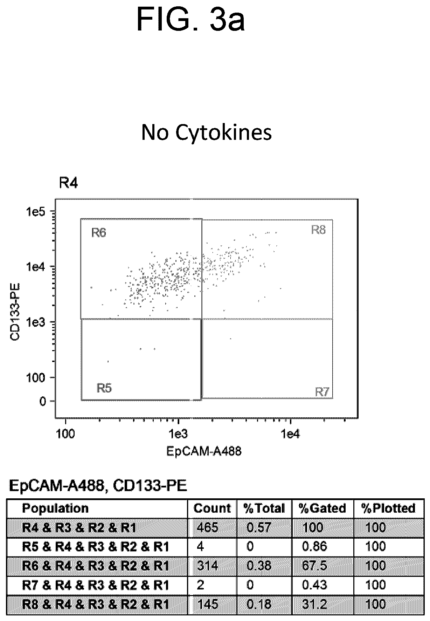

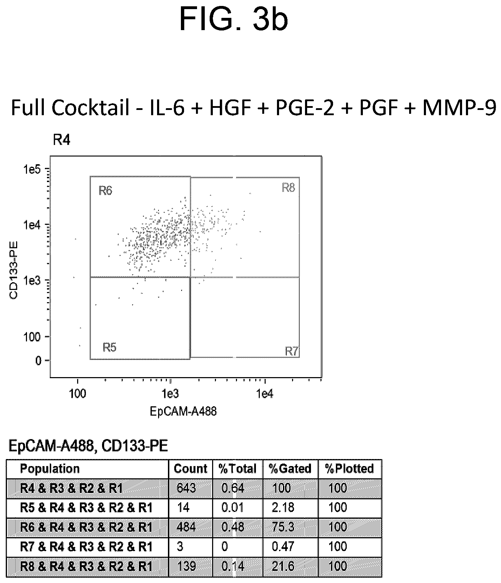

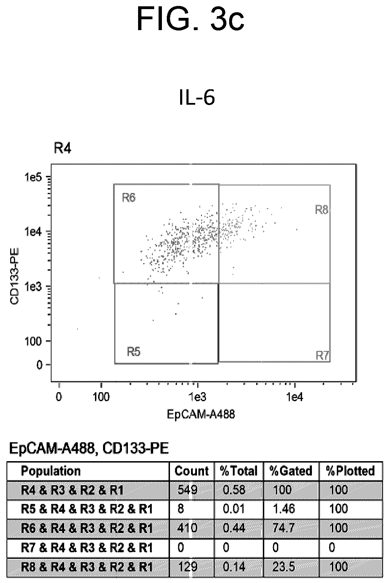

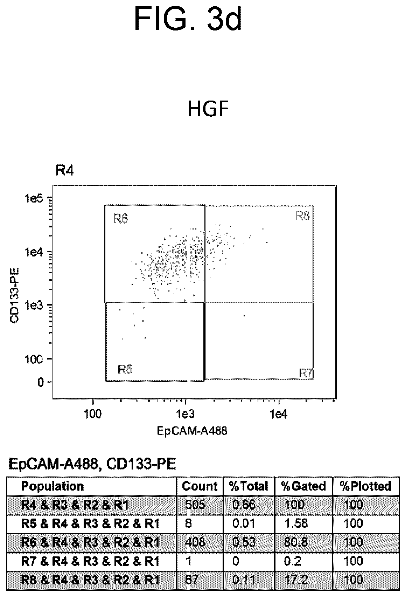

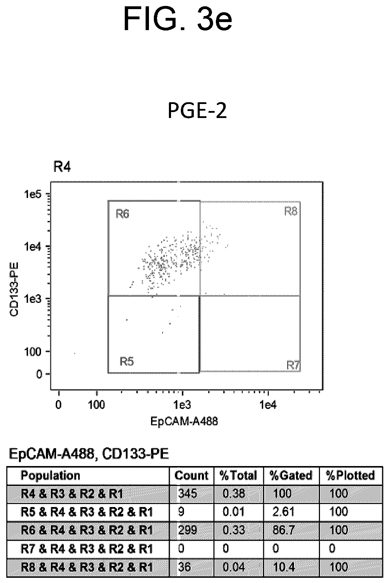

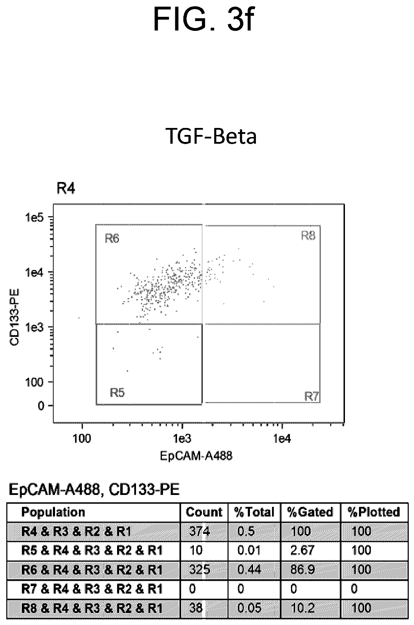

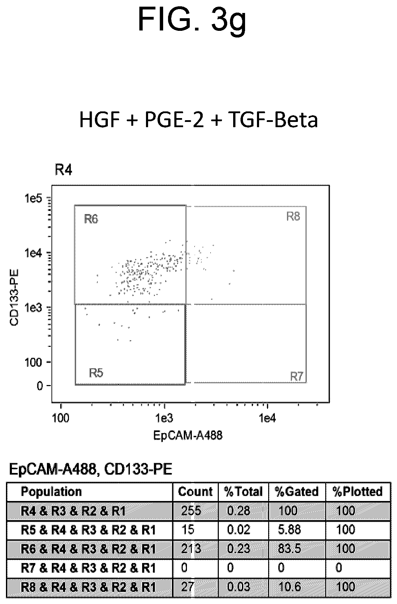

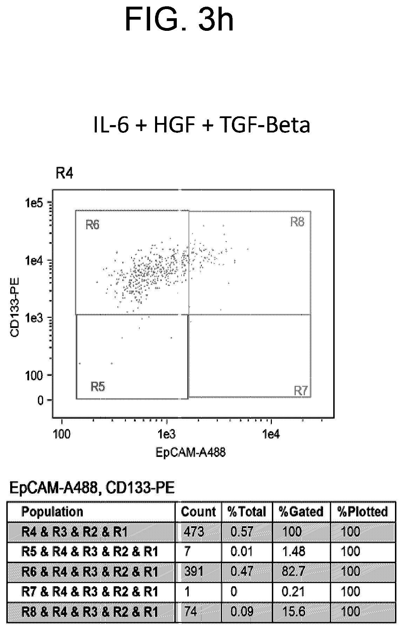

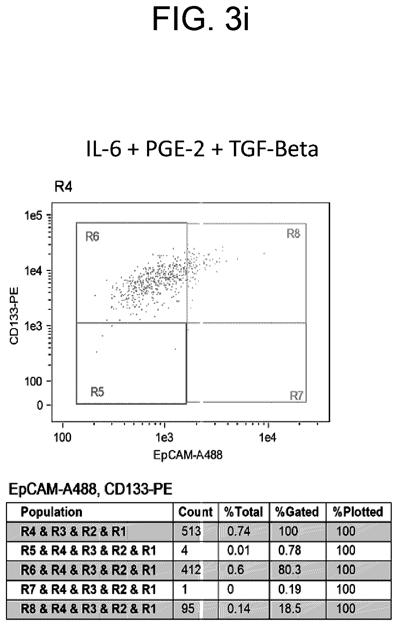

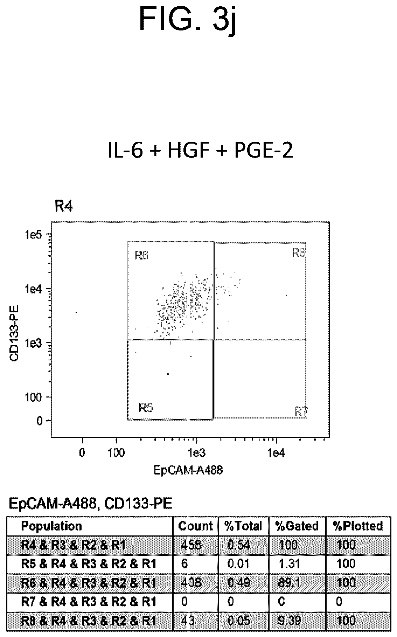

In order to determine if selected cytokines influence cell proliferation of circulating tumour cells (CTCs), whole blood was collected from a patient with metastatic prostate cancer. Circulating tumour cells were enriched from whole blood using RosetteSep CTC Enrichment cocktail. Briefly, samples of whole blood were incubated with RosetteSep (anti CD 56 for 20 minutes at room temperature). Phosphate buffered saline containing 2% Fetal Bovine Serum was added to the samples and layered on a Ficoll Paque gradient. After centrifugation, the supernatant which contained CTCs was washed twice with PBS containing 2% FBS. Equal volumes of the cell suspension were seeded into a 24-well ultra-low attachment polystyrene plate. Enriched CTCs were cultures in RPMI 1640 medium with 2mM glutamine and no serum over a period of 8 days. Different cytokines and/or cytokine cocktails were added to the medium as shown in Table 22. Figure 3 depicts scatterplots of flow cytometry experiments enriched CTCs stained with an antibody containing CD133 after treatment with various cytokines and cytokine cocktails.

Table 22: Cytokine and Cytokine Cocktail Treatments for Enriched CTCs

| Source of Cells | 1 Component Cocktail | 3 Component Cocktail | 4 Component Cocktail |
|---|---|---|---|
| Peripheral Blood | IL6 | IL6+HGF+PGE2 | IL6+HGF+PGE2+TGF |
| | HGF | IL6+HGF+TGF | |
| | PGE2 | IL6+PGE2+TGF | |
| | TGF | HGF+PGE2+TGF | |

Single component, 2-, 3-, 4- and 5-component cytokine cocktails were evaluated with respect to cell proliferation and expression of cancer stem cell-like markers. These investigations were performed in both cell lines and in circulating tumour cells enriched from whole blood of a patient with metastatic prostate cancer.

Results show that some cytokine cocktails encourage cell proliferation. Results on cell proliferation in cell lines and enriched CTCs are presented. In both cases, cells were grown in serum-free media. Results are also presented showing the effect the cytokine cocktails on cell lines and cultured CTCs with respect to cancer stem cell-like phenotypes.

For these experiments, cells were grown in media supplemented with 5% FBS.

**Cell Proliferation in the HCT-15 Cell Line**

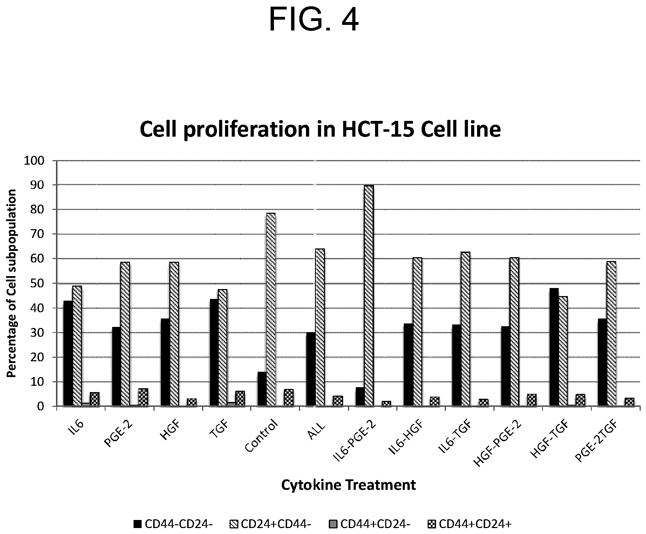

To evaluate which cocktail combinations were most likely involved in cell proliferation of presumed quiescent stem cells or de-differentiated stem cell-like cells, four cytokines, IL-6, HGF, PGE-2 and TGF-beta were added singly or in pairs to HCT-15 cells. Results of combinations that influenced cell proliferation of HCT-15 cells in culture are presented. Table 23 shows cell subpopulations after exposure to various cytokines and cytokine combinations. Figure 4 depicts cell proliferation of subpopulations in the HCT-15 cell line after exposure to various cytokines and cytokine cocktails.

Table 23: Cell Subpopulations after exposure to cytokine combinations

|  | CD44-CD24- | CD24+CD44- | CD44+CD24- | CD44+CD24+ |
|---|---|---|---|---|
| IL6 | 43.2 | 49.1 | 1.35 | 5.63 |
| PGE-2 | 32.4 | 58.7 | 0.61 | 7.3 |
| HGF | 35.9 | 58.6 | 0.31 | 3.29 |
| TGF | 43.8 | 47.7 | 1.51 | 6.26 |
| Control | 14.1 | 78.5 | 0.14 | 6.9 |
| ALL | 30.3 | 64 | 0.09 | 4.25 |
| IL6-PGE-2 | 7.85 | 89.7 | 0 | 2.17 |
| IL6-HGF | 33.9 | 60.4 | 0.26 | 3.96 |
| IL6-TGF | 33.4 | 62.7 | 0.06 | 2.91 |
| HGF-PGE-2 | 32.8 | 60.4 | 0.37 | 5.09 |
| HGF-TGF | 48.3 | 44.8 | 0.43 | 4.89 |
| PGE-2-TGF | 35.8 | 58.9 | 0.28 | 3.45 |

The CSC markers CD44 and CD24 have been used to characterize cancer stem cells among others as various cancer stem cell markers in various cell lines including HCT-15 and SW-620 (Muraro et al. 2012). In their studies, Muraro and coworkers showed that 94.87% of HCT-15 cells were double negative when stained with similar antibodies (CD44 and CD24). The cytokine combination of IL-6 and PGE-2 appears to influence significantly the expression of CD-24 as shown in Figure 4. A 2-component cytokine combination of IL-6 and PGE-2 increased significantly the CD24+ CD44- population compared with the control where no cytokines were added.

**Cell Proliferation in the SW-620 Cell Line**

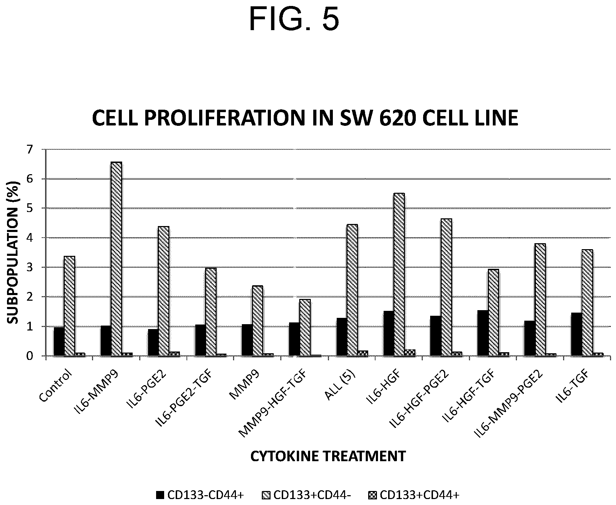

To determine cell proliferation upon the addition of cytokines, SW-620 cells were synchronized by serum starvation for 72 hours. The cells were harvested and stained with a cell proliferation dye before being exposed to selected cytokine combinations. Cells were grown over a period of 72 hours without serum to assess the specific contribution of cytokines in cell proliferation. Results are presented to show which sub-populations of cells show cell proliferation. Table 24 shows cell proliferation of cell subpopulations after exposure to various cytokines and cytokine cocktails. Figure 5 depicts cell proliferation of subpopulations in the SW 620 cell line after exposure to various cytokines and cytokine cocktails.

Table 24: Cell Proliferation in Subpopulations after cytokine treatment

|  | CD133-CD44- | CD133-CD44+ | CD133+CD44- | CD133+CD44+ |
|---|---|---|---|---|
| Control | 95.3 | 0.99 | 3.38 | 0.1 |
| IL6-MMP9 | 92 | 1.04 | 6.58 | 0.11 |
| IL6-PGE2 | 94.3 | 0.92 | 4.39 | 0.15 |
| IL6-PGE2-TGF | 95.7 | 1.08 | 2.97 | 0.08 |
| MMP9 | 96 | 1.1 | 2.37 | 0.09 |

(continued)

|  | CD133-CD44- | CD133-CD44+ | CD133+CD44- | CD133+CD44+ |
|---|---|---|---|---|
| MMP9-HGF-TGF | 96.7 | 1.15 | 1.93 | 0.04 |
| ALL (5) | 93.9 | 1.29 | 4.45 | 0.18 |
| IL6-HGF | 92.5 | 1.53 | 5.51 | 0.23 |
| IL6-HGF-PGE2 | 93.5 | 1.37 | 4.65 | 0.14 |
| IL6-HGF-TGF | 95.1 | 1.55 | 2.94 | 0.13 |
| IL6-MMP9-PGE2 | 94.5 | 1.2 | 3.81 | 0.09 |
| IL6-TGF | 94.4 | 1.49 | 3.6 | 0.11 |
| Subpopulations are expressed in as a percentage of gated cells | | | | |

Cells that were not exposed to cytokines show the least proliferation in the stem cell sub-population fraction (0.99% gated cells). The highest proliferation with the 72 hour period was in cells treated with the following cytokine cocktail:

1. IL-6, HGF, TGF (1.55% gated cells)

2. IL-6, HGF (1.55% gated cells)

3. IL-6, TGF (1.49% gated cells)

4. IL-6, HGF, PGE2 (1.37% gated cells)

Selected cytokines were applied to enriched CTCs in culture. Results from these experiments showed cell proliferation with some cytokine combinations.

**Cell Proliferation in Enriched CTCs**

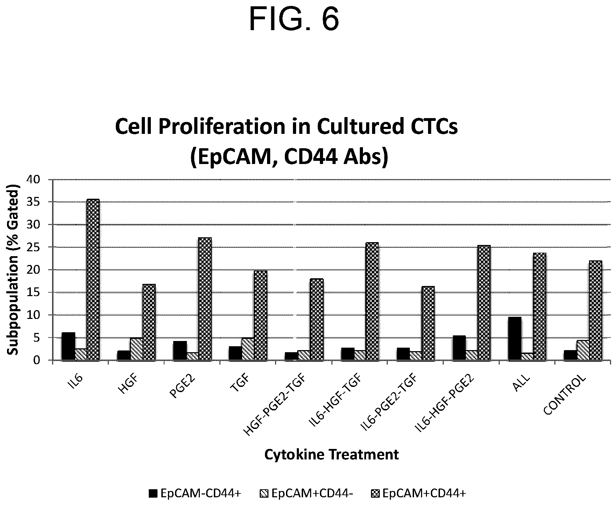

Cells stained with CD44PE antibody cocktail, analysed using flow cytometry had four possible fluorescence staining outcomes: EpCAM positive, CD44 positive, EpCAM and CD44 positive (double positive) and double negative cells (cells negative for both EpCAM and CD44). Table 25 shows cell subpopulations exhibiting cancer stem cell marker after treatment with various cytokines and cytokine cocktails. Figure 6 depicts cell proliferation of subpopulations in cultured CTCs after exposure to various cytokines and cytokine cocktails. Cells were stained using an antibody containing CD44 PE.

Table 25: Cell populations exhibiting cancer stem cell markers after treatment with different cytokines. Values are expressed as a percentage of gated cells.

|  | EpCAM-CD44- | EpCAM-CD44+ | EpCAM+CD44- | EpCAM+CD44+ |
|---|---|---|---|---|
| IL6 | 55 | 6.14 | 2.66 | 35.6 |
| HGF | 74.9 | 2.12 | 4.89 | 16.8 |
| PGE2 | 65.5 | 4.23 | 1.75 | 27.2 |
| TGF | 72 | 3.11 | 4.95 | 19.8 |
| HGF-PGE2-TGF | 77.1 | 1.75 | 2.23 | 18 |
| IL6-HGF-TGF | 67.9 | 2.77 | 2.25 | 26 |
| IL6-PGE2-TGF | 78.1 | 2.79 | 2.01 | 16.3 |
| IL6-HGF-PGE2 | 65.4 | 5.37 | 2.28 | 25.5 |
| ALL | 63.1 | 9.58 | 1.64 | 23.8 |

(continued)

| | EpCAM-CD44- | EpCAM-CD44+ | EpCAM+CD44- | EpCAM+CD44+ |
|---|---|---|---|---|
| CONTROL | 71.4 | 2.2 | 4.4 | 22 |

Numbers represent percentages of gated subpopulations. An antibody cocktail with EpCAM A488, CD133 PE and CD45 PerCPCy5.5 was used. No cell stained positive for CD45 **PerCPCy5.5**.

CD44 is a marker of stemness in various cancer cells including prostate cancer. Cells treated with all four cytokines had the high cell proliferation of EpCAM-CD44+ cell sub-population during the cell culture period (8 days).

The highest proliferation for this sub-population were in cells which were treated with:

1. all four cytokines: IL-6, HGF, PGE-2 and TGF-beta (9.58% gated)

2. IL-6 (6.14% gated)

3. IL-6, HGF, PGE2 (5.37% gated)

4. PGE2 (4.23% gated)

Deletion of IL-6 from the cocktail reduced significantly the EpCAM-CD44+ sub-population (1.75% gated compared to 2.2% in cells with no cytokines). This strongly suggests that IL-6 as well as a full cocktail of cytokines is important in cell proliferation of the stem-cell subpopulation (EpCAM-CD44+) of enriched CTCs.

The percentage of cells which stained positive for both EpCAM and CD44 were the highest in cells treated with IL-6. This again strongly suggests that IL-6 is important in cell proliferation of CD44+ cells.

A large number of cells did not stain positive for any antibody (Table 25). These cells were not leucocytes as observed by negative staining of CD45PerCP Cy5.5. To explain this, it is important to look at results of cells from the same samples which were stained with a different antibody cocktail.

It is worth noting that enriched CTCs had a lower counter of EpCAM positive cells as compared to cell lines which had more than 99% EpCAM positive cells.

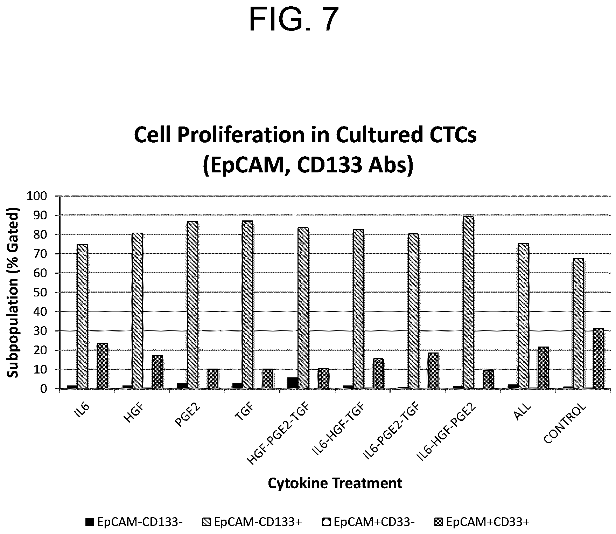

Similarly, cells stained with CD133 PE antibody cocktail, analysed using flow cytometry had four possible fluorescent staining outcomes: EpCAM positive, CD133 positive, EpCAM and CD133 positive (double positive) and double negative (cells negative for both EpCAM and CD133. The different cell populations within a cell population treated by a specific cytokine or cocktail of cytokines. Table 26 shows cell subpopulations exhibiting cancer stem cell marker after treatment with various cytokines and cytokine cocktails. Figure 7 depicts cell proliferation of subpopulations in cultured CTCs after exposure to various cytokines and cytokine cocktails. Cells were stained using an antibody containing CD133 PE.

Table 26: Cell populations exhibiting Cancer Stem Cell Marker after Treatment with different Cytokines.

| | EpCAM-CD133- | EpCAM-CD133+ | EpCAM+CD133- | EpCAM+CD133+ |
|---|---|---|---|---|
| IL6 | 1.46 | 74.7 | 0 | 23.5 |
| HGF | 1.58 | 80.8 | 0.2 | 17.2 |
| PGE2 | 2.61 | 86.7 | 0 | 10.4 |
| TGF | 2.67 | 86.9 | 0 | 10.2 |
| HGF-PGE2-TFG | 5.88 | 83.5 | 0 | 10.6 |
| IL6-HGF-TGF | 1.48 | 82.7 | 0.21 | 15.6 |
| IL6-PGE2-TGF | 0.78 | 80.3 | 0.19 | 18.5 |
| IL6-HGF-PGE2 | 1.31 | 89.1 | 0 | 9.39 |
| ALL | 2.18 | 75.3 | 0.47 | 21.6 |
| CONTROL | 0.86 | 67.5 | 0.43 | 31.2 |

Numbers represent percentages of gated subpopulations. An antibody cocktail with EpCAM A488, CD133 PE and CD45 PerCPCy5.5 was used. No cell stained positive for CD45 PerCPCy5.5.

CD133 is a marker for stemness in prostate cancer.

The highest proliferation of cells were observed in EpCAM-CD133+ subpopulation. The cytokine cocktail of IL-6, HGF and PGE-2 had the highest EpCAM-CD133+ subpopulation (89.1% gated) and control cells had the lowest gated subpopulation (67.5% gated). The full cocktail surprisingly had a lower subpopulation of EpCAM-CD133+ cell subpopulation. It was expected that the percentages of gated cell subpopulation for EpCAM-CD133+ cell subpopulations would be lower than observed; at least comparable to CD44+ subpopulations. However, CD133 is considered a prominent cancer stem cell marker for prostate cancer whilst CD44 is a cancer stem cell marker for various cancer types.

Moreover in the CD44 cocktail, there was a very high percentage of the double negative subpopulation which could mean that these were CD133+ cells.

Moreover, CTCs appear to respond to cytokine exposure differently from cell lines. One reason could be that CTCs might already be going through the EMT process. The results show that in cell lines more than 99% of cells stained positive for EpCAM whilst in enriched CTCs only about 30% stained positive for EpCAM.

Surprisingly, the highest cell population of double positive was in control cells. Whilst this may be surprising, it may also mean that the absence of cytokines in culture arrested cells in the state of EMT where cells could not progress into cancer stem cells (EpCAM-CD133+).

A very small subpopulation were double negative. This suggests an active EMT process in enriched CTCs.

The cytokine cocktail of IL-6, HGF and PGE-2 appears to enhance cell proliferation both in cell lines and in enriched CTCs. The effect of these cytokines on expression of cancer stem cell markers has varied according to the source of the cells. For cell lines, IL-6-HGF and TGF-beta appeared to be more important whilst in enriched CTCs, IL-6-PGE-2 combinations appeared to be more important.

Example 4

***Evidence of EMT in a Patient with Colorectal Cancer***

A blood sample was collected from a patient with colorectal cancer six days after surgery. Blood samples were enriched for CTCs using RosetteSep CD 56 kit according to standard protocol. Prior to cell culture, cells were stained with the following antibody cocktail: EpCAM A488, CD133PE, CD 45 PerCP Cy5.5, and Lgr5-PE-Vio770. Cells were either cultured in low attachment well plates without cytokines (control cells) or with three different cytokines (IL-6, IL-8 or PDGFBB).

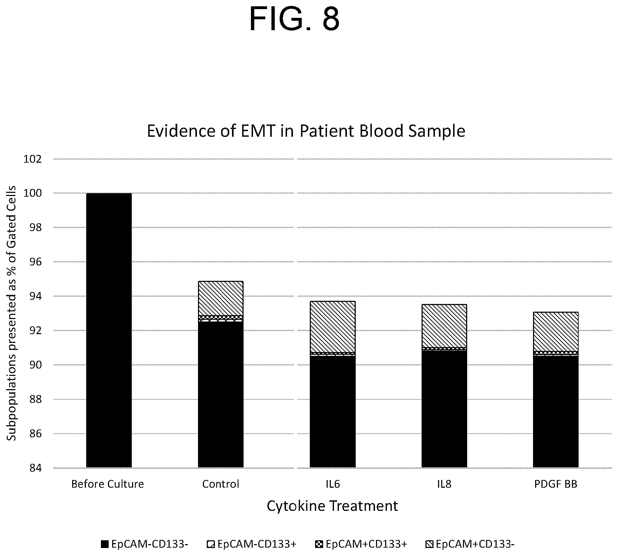

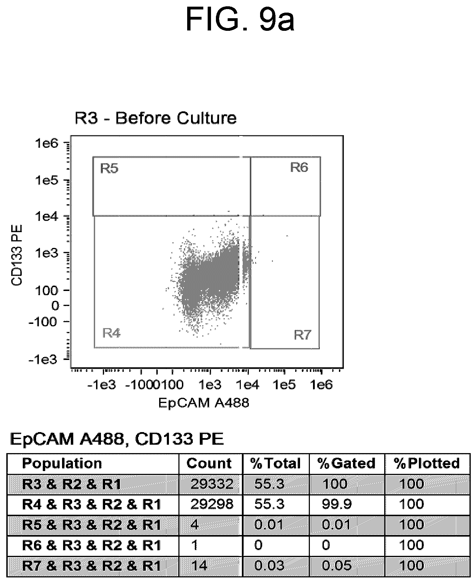

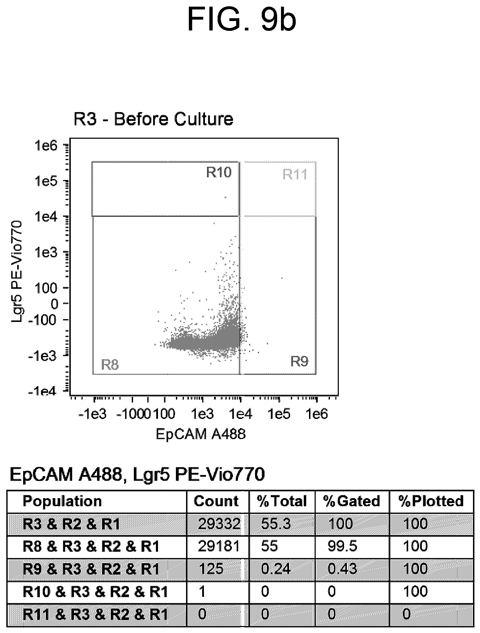

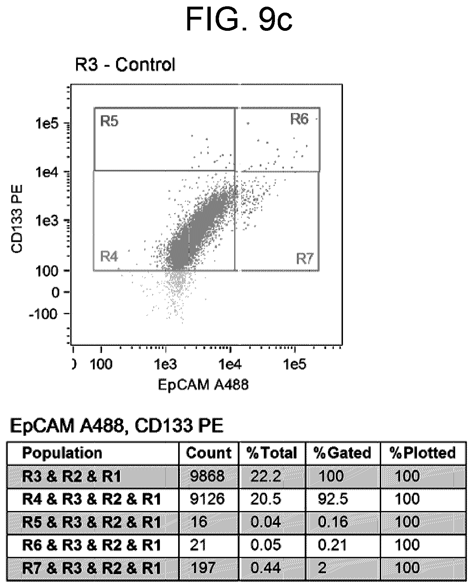

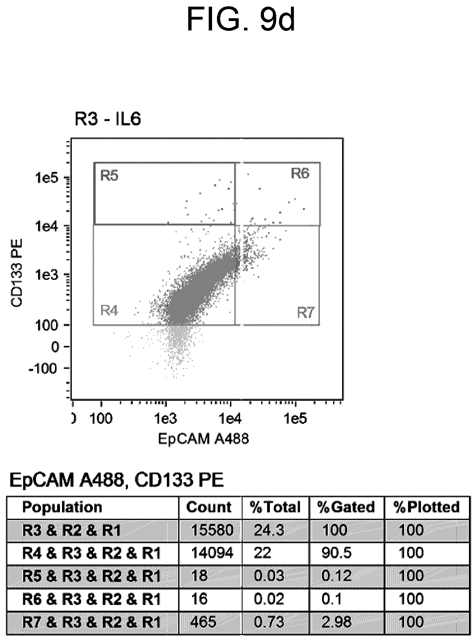

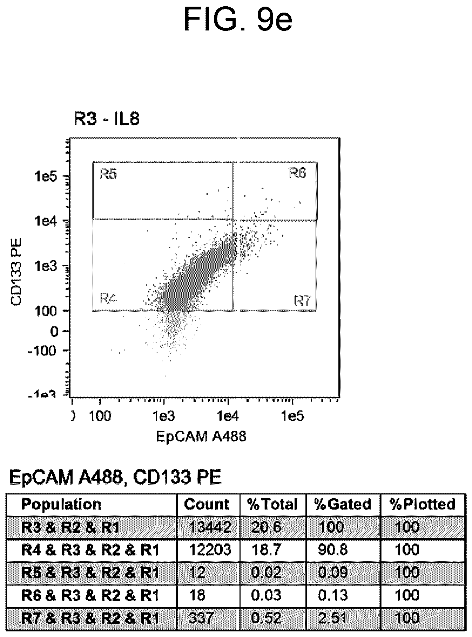

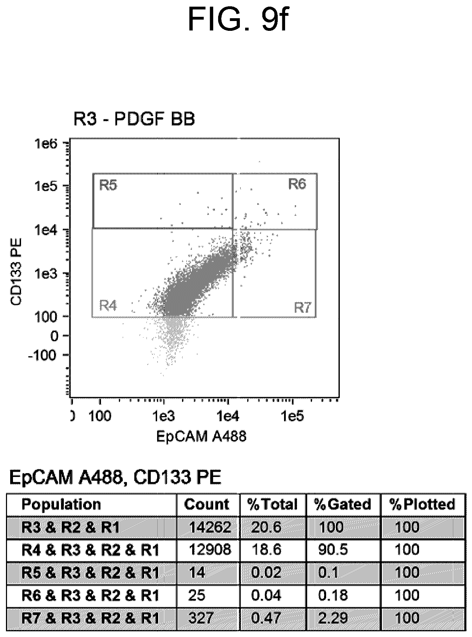

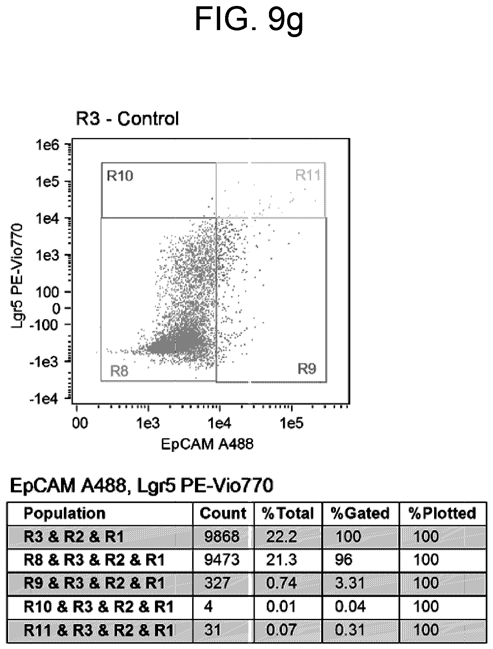

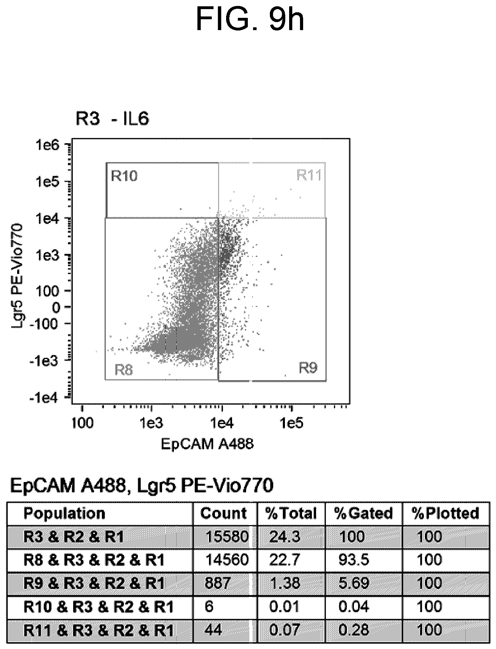

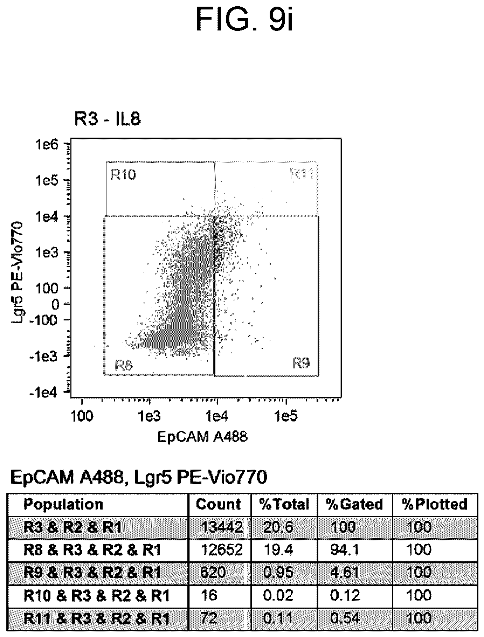

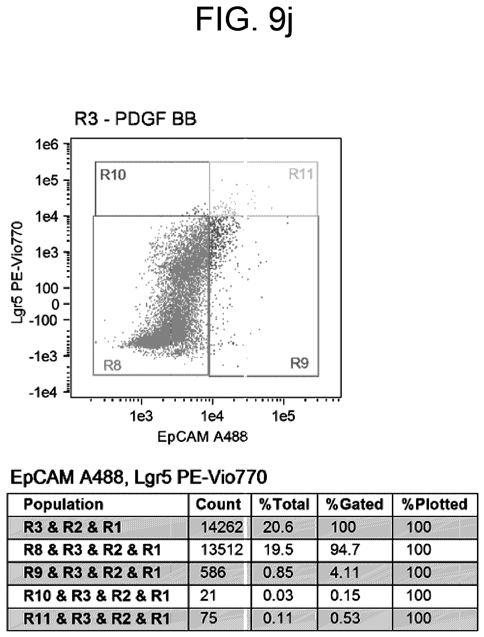

Results show that prior to culture, the subpopulation of EpCam positive cells constituted 99.9% of gated cells. During culture, the subpopulation of EpCAM positive cells dropped to 92.5% in the control cells and 90.5-90.8% in cytokine treated cells as shown in Table 27. Figure 8 shows the effect of three cytokines (IL-6, IL-8 and PDGFBB) on enriched CTCs. The cancer stem cell fraction (the EpCAM+CD133- subpopulation) increased from 0.05% before culture to 2% of gated cells in the control and 2.29-2.98 % of gated cells in cytokine treated cells (See Table 27) This increase corresponds to more than an order of magnitude. The increase in the EpCAM+CD133- subpopulation following the addition of IL-6 was statistically significant ($p < 0.05$), Additionally, the EpCAM+CD133+ subpopulation is observed at higher percentages in control cells than in cytokine treated cells. This subpopulation is absent in cells before culture (Table 27). This subpopulation may act as an intermediate subpopulation before the cells advance to a full cancer stem cell subpopulation (EpCAM+CD133-) . Figure 9 depicts flow cytometry scatterplots of cells stained with EpCAM A488, CD133PE and Lgr5-PE-Vio770 following treatment with IL-6, IL-8 and PDGFBB.

Flow cytometry data was analysed using IDEAS software. Double events, out of focus events, CD45PercPCy5.5 positive cells, dead cells and non-nucleated events were eliminated from the fluorescence analysis of the cell population. Hoescht 33258 dye was used to stain nuclei of enriched CTCs. All experiments were stained with a fixable live/dead dye eFluor 506 to exclude dead cells.

Table 27: Cell populations of cultured circulating tumour cells exhibiting cancer stem cell marker after treatment with different cytokines.

|  | **EpCAM-CD133-** | **EpCAM-CD133+** | **EpCAM+CD133+** | **EpCAM+CD133-** |
|---|---|---|---|---|
| Before Culture | 99.9 | 0.01 | 0 | 0.05 |
| Control | 92.5 | 0.16 | 0.21 | 2 |
| IL-6 | 90.5 | 0.12 | 0.1 | 2.98 |
| Il-8 | 90.8 | 0.09 | 0.13 | 2.51 |

(continued)

|  | EpCAM-CD133- | EpCAM-CD133+ | EpCAM+CD133+ | EpCAM+CD133- |
|---|---|---|---|---|
| PDGFBB | 90.5 | 0.1 | 0.18 | 2.29 |
| Numbers represent percentages of gated subpopulations. An antibody cocktail with EpCAM A488, CD133 PE and CD45 PerCPCy5.5 was used. No cell stained positive for CD45 PerCPCy5.5. | | | | |

Example 5

***Applying Distinct Cytokine Cocktails to Cancer Cells will Increase Cell Proliferation and EMT***

Data suggests that applying anti-cancer therapies to cancer cell populations rapidly after exposure to HGF in isolation can improve therapeutic effectiveness against cancer stem cells. This is believed to be caused by this cytokine triggering cellular proliferation in both non-stem and stem cell populations and rendering these dividing cells more vulnerable to anti-cancer therapies during a narrow window of time. This effect appeared to be limited to HGF alone as IL-6, PGE-2, and TGF-beta exposure enriched irinotecan treated cells for cancer stem cells above non-cytokine treated controls. However, delaying therapy too long after exposure to these cytokines renders most conventional anti-cancer therapies less effective, presumably because many of these cells will have transitioned to the stem cell phenotype and shifted back into dormancy.

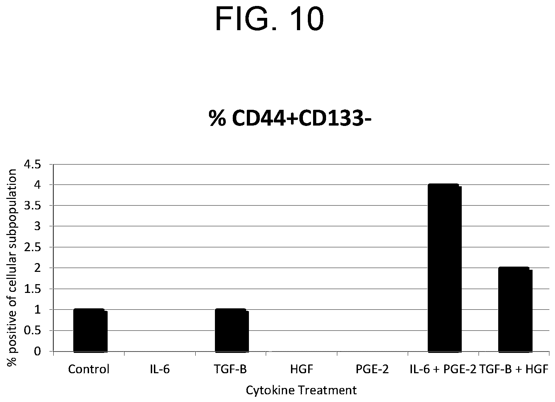

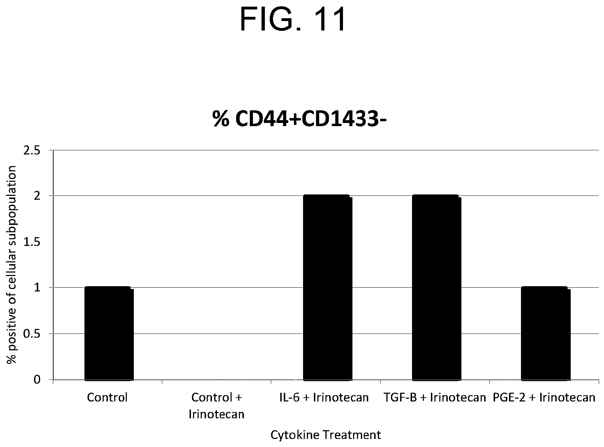

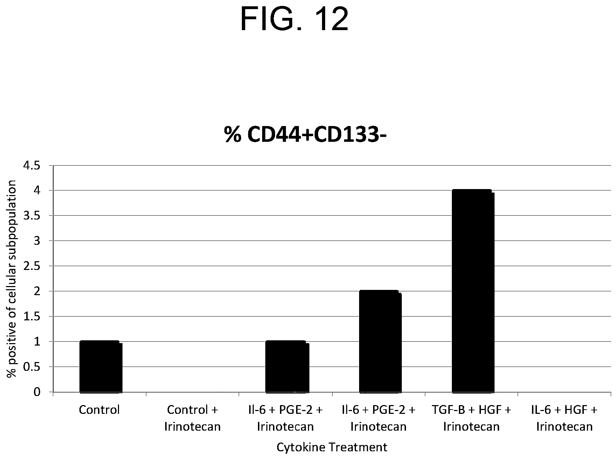

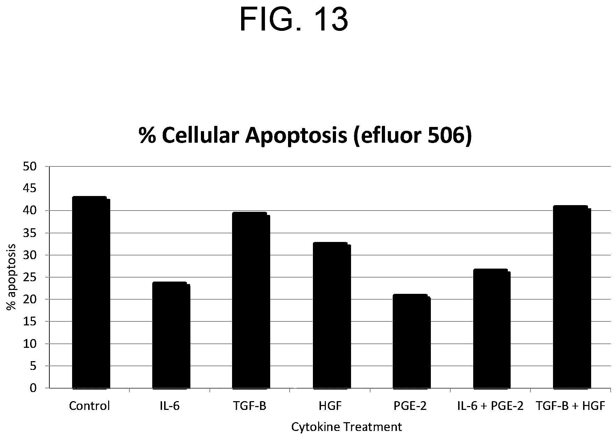

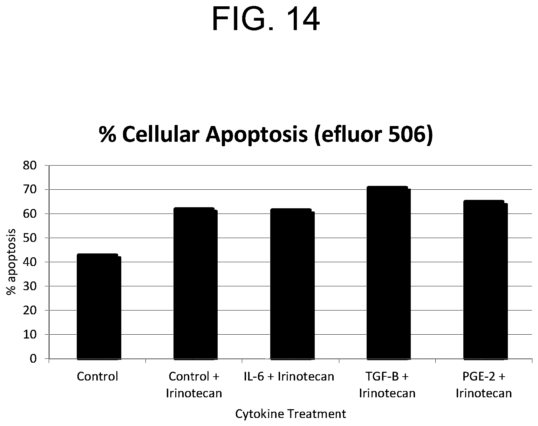

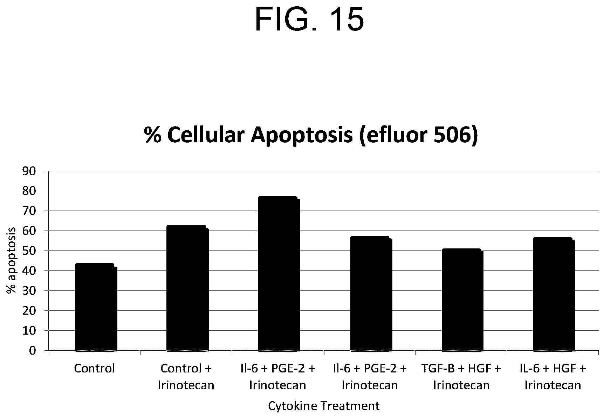

SW-620 cells, a human colorectal adenocarcinoma cell line, were treated with cytokines alone or in combination including IL-6, TGF-beta, HGF, PGE-2, IL-6 + PGE-2 and TGF-beta + HGF for 24 hours and compared to untreated SW-620 cells as control. Some treated and untreated SW-620 cells were then exposed to $2\mu$M Irinotecan for 36 hours at which point cells were harvested for flow cytometry to assess for stem cell markers and markers of apoptosis. The percentage of CD44+CD133- cells following treatment with various cytokine and cytokine cocktails is depicted in Figure 10. Compared to non-cytokine treated cells, combinations with IL-6 + PGE-2, and TGF-beta + HGF increased the number and percentage of cells that were CD44+CD133- as shown in Figure 10 (stem cells). Figure 11 depicts the percentage of CD44+CD133- cells following treatment with various cytokines and Irinotecan. Treatment with IL-6 + Irinotecan and TGF + Irinotecan increased the percentage of CD44+CD133- stem cells to 2% (from zero in non-treated control cells) as shown in Figure 11. Figure 12 depicts the percentage of CD44+CD133- cells following treatment with various cytokine cocktails and Irinotecan. A combination of TGF + HGF + Irinotecan increased the percentage of CD44+CD133- stem cells to 2% (from zero in non-treated control cells) as shown in Figure 12. Cells treated with HGF + Irinotecan resulted in a log kill ratio of 100% (no viable cells after treatment). Additionally, treatment with IL-6 and PGE-2 appears to have a cytoprotective effective as evidenced by an increased percentage of stem cells that survived treatment as shown in Figure 13. The effect of treatment of various cytokines and Irinotecan on cellular apoptosis is depicted in Figure 14. The effect of treatment of various cytokine cocktails and Irinotecan on cellular apoptosis is depicted in Figure 15.

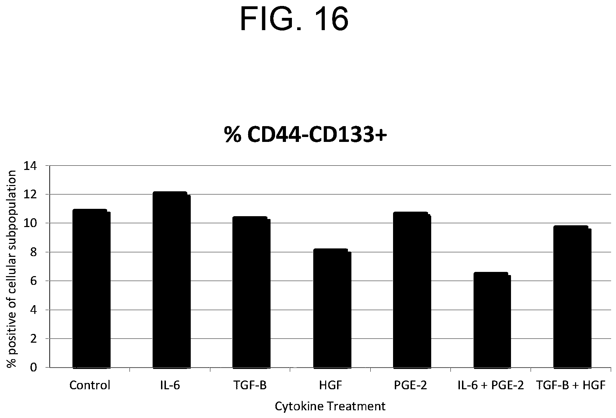

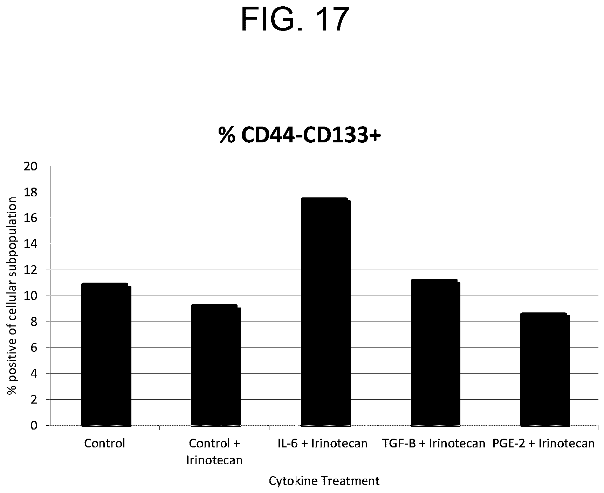

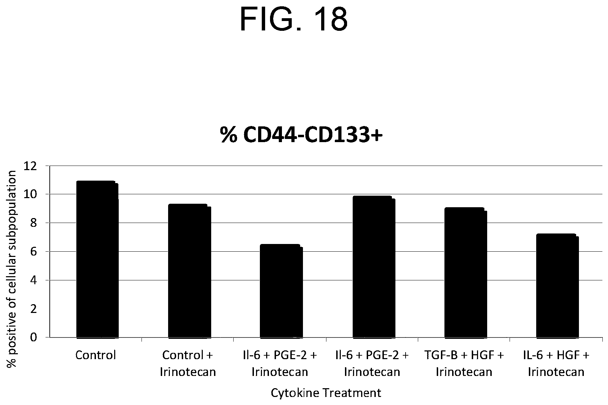

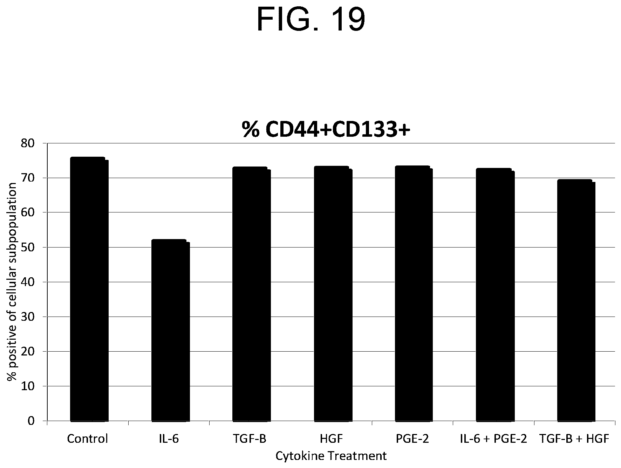

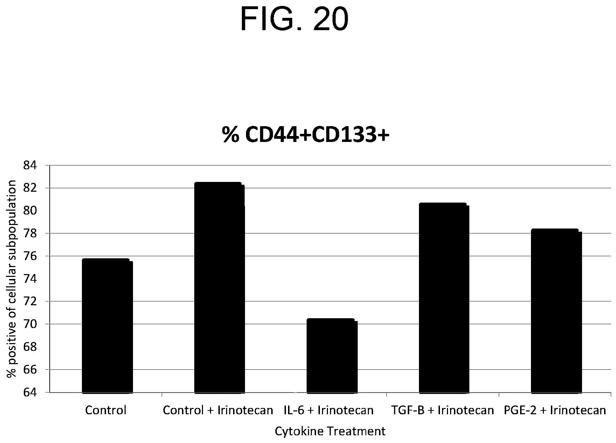

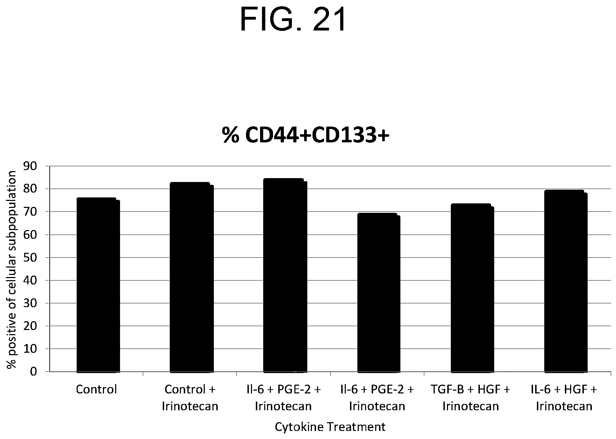

Additionally, the percentage of CD44-CD133+ cells following treatment with various cytokines and cytokines cocktails was determined as depicted in Figure 16. The percentage of CD44-CD133+ cells following treatment with various cytokines and Irinotecan was measured as depicted in Figure 17. The percentage of CD44-CD133+ cells following treatment with various cytokine cocktails and Irinotecan was measured as depicted in Figure 18. The percentage of CD44+CD133+ cells following treatment with various cytokine and cytokine cocktails is depicted in Figure 19. The percentage of CD44+CD133+ cells following treatment with various cytokines and Irinotecan is depicted in Figure 20. The percentage of CD44+CD133+ cells following treatment with various cytokine cocktails and Irinotecan is depicted in Figure 21.

*The Effects of HGF on Chemotherapy*

Given the protective nature for stem cell survival afforded by IL-6, PGE-2, and TGF-beta after exposure to chemotherapy, and potentially PDGF-BB given its role in fostering stem cell enrichment as shown in Table 27, and given that HGF in isolation rendered cancer cells highly sensitive to the effects of chemotherapy as detailed above, it is conceptualized that by blocking the influence of these specific cytokines, including IL-6, PGE-2, PGF, TGF-beta, PDGF-BB, MCP-1 and MMP-9 at the time they are upregulated (ligand/receptor/downstream actors) while allowing the predicted upregulation of HGF to occur after tumor removal, a surviving cancer cell population may become very vulnerable to the effects of cytotoxic chemotherapy, including a surviving cancer stem cell population. The chemotherapeutic agents that would work most effectively during this time would be those agents that target the machinery of cell division, given the effect of HGF in isolation triggering cell proliferation and hence vulnerability to agents that disrupt the fidelity of cell division.

Example 6

### The Distinct Cytokine Response After Chemotherapy and Radiation

Given the highly conserved nature of a wound healing response, a similar tissue (tumour) repair response will be triggered not only by surgery, but also by chemotherapy and radiation therapy. By blocking this response at the time it is predicted to be upregulated one should also be able to prevent the emergence of a stem cell enriched residual cancer cell population and therefore mitigate the development of a drug resistant phenotype.

The influence of radiation treatment and chemotherapy treatment was studied on patients with either prostate, breast, or colorectal cancers (N=6). Specimens for cytokine testing were collected by aseptic technique into EDTA tubes. Specimens from chemotherapy patients were collected before chemotherapy, 48 hours after chemotherapy and at one week after chemotherapy. Specimens from radiation patients were collected before radiation, and at 24 hours, 48 hours, 72 hours and at one week after radiation.

EDTA samples were centrifuged within 30 minutes of collection, plasma was removed and then recentrifuged. Plasma was then aliquoted into cryotubes and stored in a -80°C freezer. On the morning of testing, cryotubes containing an aliquot of plasma from designated patients were placed into the 4°C refrigerator to thaw, then were vortexed and recentrifuged for 5 minutes at 10,000g. Testing was performed immediately after this.

Results of tests performed using EMD Millipore kits were read on the Luminex 200 analyzer. This flow cytometer based instrument integrates key detection components, such as lasers, optics, advanced fluidics and high speed digital signal processors. The multiplex technology is capable of performing a variety of bioassays including immunoassays on the surface of fluorescent coded magnetic beads known as MagPlex TM-C microspheres. Results are quantified based on fluorescent reporter signals.

TGF-beta levels dropped in concert with PDGFBB levels acutely after treatment in 4/6 of the patients. IL-6, HGF and IL-8 levels rose acutely in 4/6 patients after treatment, as did IL-8.

The results of this study are ongoing and with future enrollment, the results will be determined longitudinally for longer than one week in duration.

Example 7

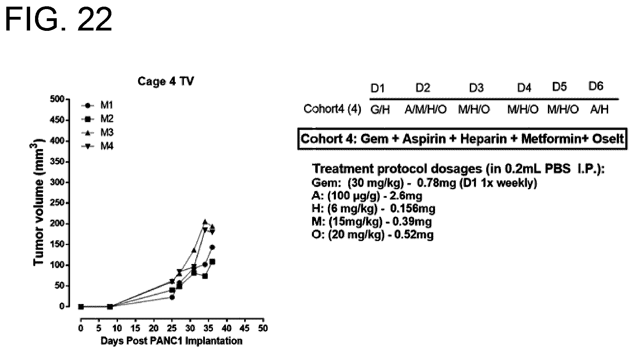

Nude mice were injected with PANC1 human pancreatic cancer cells. Treatment was initiated when the size of the tumors exceeded 100 mm$^3$. They were divided up into 4 cohorts. Cohort one was untreated. Cohorts 2 and 4 received treatments in line with the combinatorial strategy outlined above. They both received chemotherapy on day number one with gemcitabine, an antimetabolite. They also received low molecular weight heparin on days 1-6; aspirin on days 2 and days 6; and metformin, an anti-stem cell drug, on days 2-5. Cohort 4 also received oseltamivir on days 2-5 as well and is listed below in Figure 22.

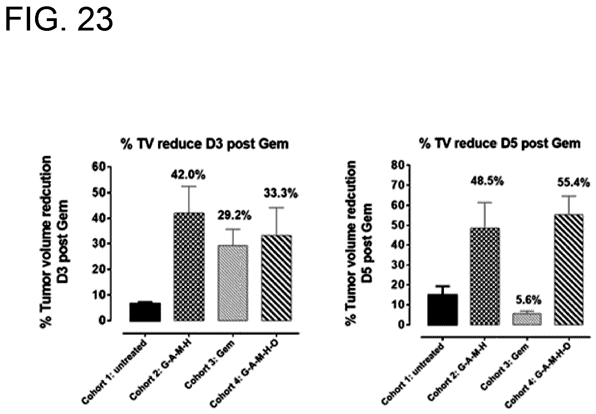

As one can see in Figure 23, the cohorts that received treatment according to the combinatorial methodology listed above saw the greatest reduction in tumor volume from baseline and this reduction persisted to the start of the next cycle of treatment. In contrast, the cohort that received the conventional treatment strategy of chemotherapy in isolation saw an initial reduction in tumor volume by day 3 but essentially a return to baseline by day 5. The present inventor maintains that this return to pretreatment volume after chemotherapy is inevitable sooner or later in any non-curative cancer treatment that does not disrupt the highly conserved tissue repair response that will be induced by the initial cancer treatment. Such a repair response, if left unchecked, is certain to foster the repair of a surviving cancer cell population.

The present inventor has also recognized that the foregoing example could also be extrapolated to other anti-cancer therapies, such as radiation therapy or surgical removal of a primary tumor, where a similar tissue repair response is predicted to be engaged after treatment damaging the tumor.

Example 8 (reference example, not forming part of the invention)

### Reversing IPRES Signature Using Inhibitors of Neuraminidase 1 in Combination with COX Inhibitors as Method to Sensitize Tumours to Treatment with Checkpoint Inhibitors

One way to reverse a mesenchymal/IPRES tumor signature towards a molecular genetic signature sensitive to the effects of immunotherapeutic agents such as the checkpoint inhibitors comprises the use of a chemotherapy cocktail together with checkpoint inhibitors. The present inventor has shown that tumors from mice with human PANC-1 cancer cells implanted heterotopically can be manipulated towards an epithelial rather than mesenchymal phenotype using a cocktail comprising a neuraminidase 1 inhibitor (Oseltamivir phosphate) in combination with a cyclooxygenase (COX) inhibitor such as celecoxib or aspirin.

In some embodiments, the cocktail comprise a non-steroidal anti-inflammatory drug (NSAID) that inhibits COX-1 and/or COX-2. In some embodiments the NSAID is ketorolac, flurbiprofen, suprofen, ketoprofen, indometacin, aspirin, naproxen, tolmetin, ibuprofen, ampyrone, fenoprofen, zomepirac, niflumic acid, sodium salicilate, diflunisal, piroxicam, tomoxiprol, meclofenamate, sulindac, diclofenac, nimesulide, celecoxib, meloxicam, etodaloc, or rofecoxib. In some embodiments the NSAID is a COX-1 selective inhibitor. In other embodiments, the NSAID is a COX-2 selective inhibitor. In preferred embodiments the NSAID is celecoxib or aspirin.

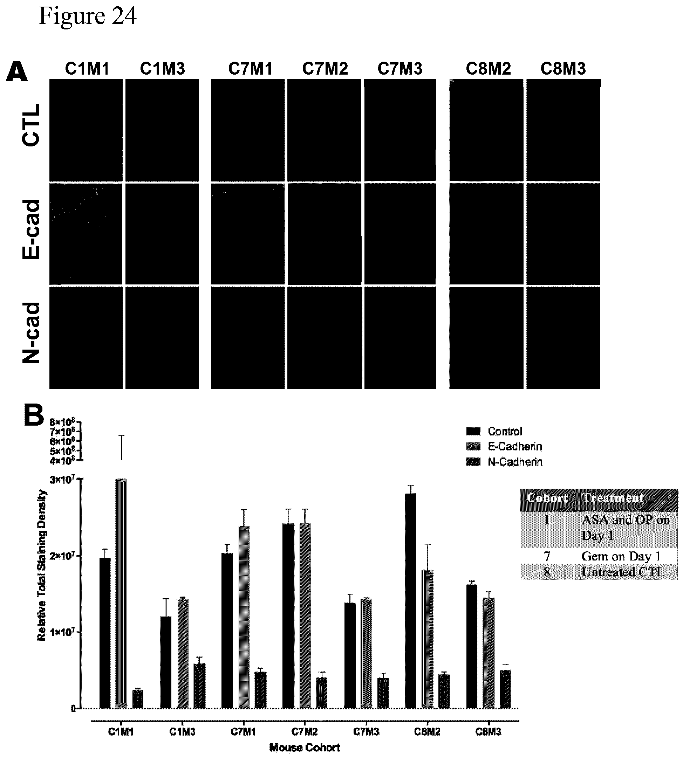

Using this cocktail with chemotherapy prevented drug induced reversion to a mesenchymal phenotype during chemotherapy treatment while treatment with chemotherapy by itself led to the downregulation of E-cadherin. Specifically, mice treated with a combination of Oseltamivir phosphate, celecoxib or aspirin, and chemotherapy had upregulated expression of E-Cadherin (marker of epithelial phenotype) relative to treatment with chemotherapy alone (downregulation of E-cadherin) (see Figure 24).

Figure 24 shows fluorescence immunohistochemical detection of E-cadherin and N-cadherin expression in paraffin-embedded tumor tissues from xenograft tumors of PANC-1 cells growing in immunocompromised mice. Paraffin-embedded tumor sections (5 µm) on glass slides were processed from immunohistochemistry using conjugated E-cadherin and N-cadherin antibodies (see A of Figure 24). Background control (CTL) sections were prepared without the antibodies. Images are representative of at least five fields of view from two tumor sections. (B). Each bar represents the mean ($\pm$ standard error of the mean) corrected density of tumor staining. Abbrev.: C = cohort; M = mouse number. E- and N-cadherin expression in paraffin-embedded xenograft tumors of PANC-1 cells in immunocompromised mice using conjugated E- and N-cadherin antibodies was detected by fluorescence immunohistochemistry. Animals treated with a combination of ASA, Met and OP with Gem showed an increased E-cadherin (E-cad) expression compared to Gem only and untreated controls. Ncadherin levels remained consistent regardless of treatment. Images are representative of at least five fields of view from two tumor sections, and each bar represents the mean $\pm$ standard error of the mean corrected density of tumor staining.

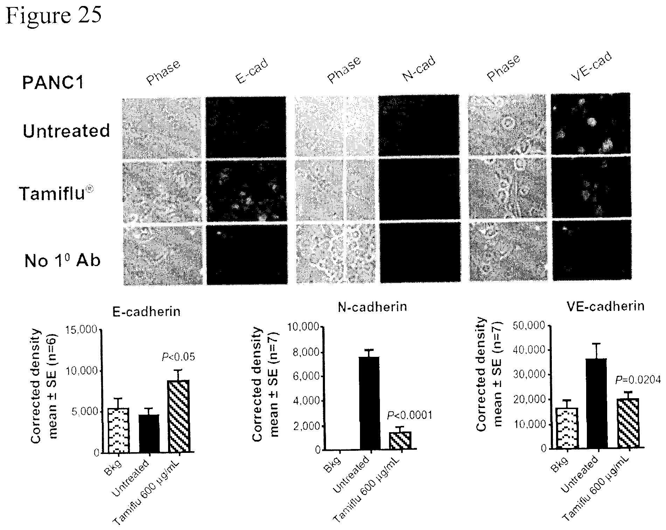

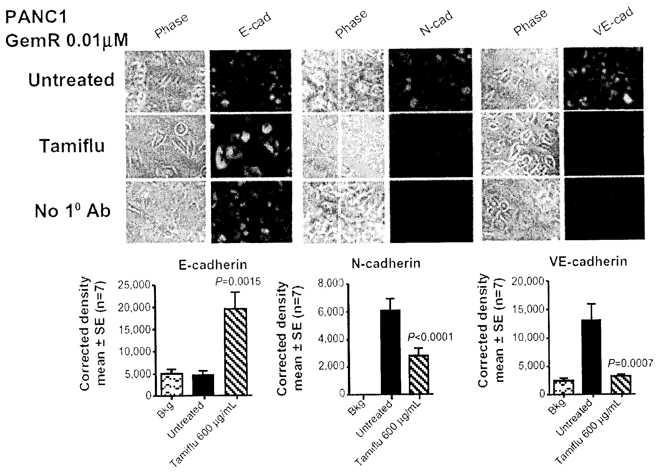

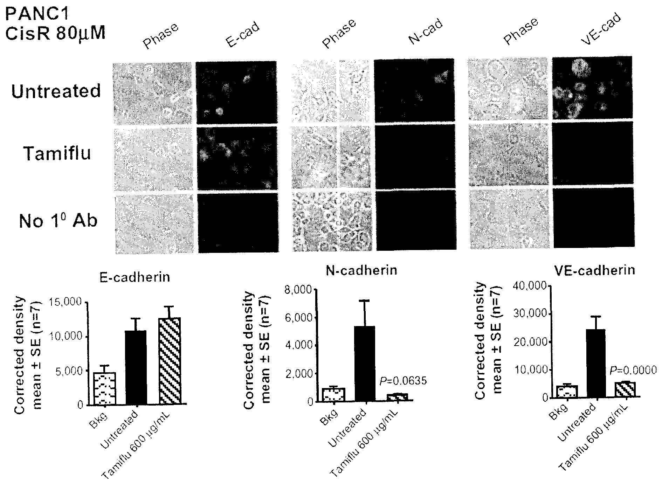

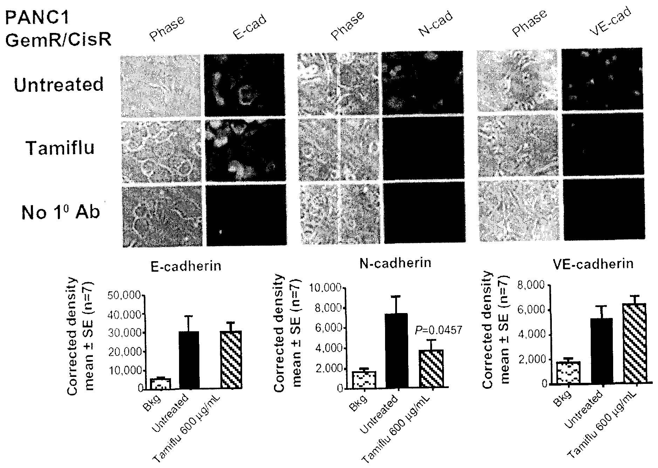

Turning to Figure 25, oseltamivir phosphate by itself was able to reverse a partial EMT in human PANC1 cancer cell lines, including drug resistant cancer cell lines as evidenced by an upregulation in the epithelial marker E-cadherin and a downregulation in the mesenchymal marker N-cadherin. Expression of e-cadherin, n-cadherin, and Ve-cadherin on the surface of Panc1, Panc1-gemr, Panc1-cisr, and Panc1-gemr/cisr cells were determined following treatment with Tamiflu® 600 µg /mL for 24 hours. Immunocytochemistry was performed on fixed, nonpermeabilized cells. The indicated primary antibodies for Ecadherin, N-cadherin, and Ve-cadherin were used, followed by alexa Fluor® 594 (life Technologies inc, Burlington, ON, Canada) secondary antibody for the primary antibody against n-cadherin and Dylight™ 488 (santa cruz Biotechnology, inc, santa cruz, ca, Usa) secondary antibody for primary antibodies against e-cadherin and Ve-cadherin. The background controls had no primary antibody during the staining procedure. The stained cells were visualized after 24 hours using a Zeiss M2 Imager fluorescence microscope (Carl Zeiss ag, Oberkochen, germany) at 400× magnification. Images are representative of at least four fields of view in three separate trials. Quantitative analysis was done by assessing the density of cell staining corrected for background in each panel using corel Photo Paint 8.0 software (Corel Corporation, Ottawa, ON, Canada). each bar in the figures represents the mean ( $\pm$ standard error of the mean) corrected density of culture cell staining for equal cell density (5 $\times$ 105 cells) within the respective images. P-values represent significant differences at 95% confidence using Dunnett's multiple comparison test compared with untreated control group. (Abbreviations: 1° ab = primary antibody; Panc1-gemr = Panc1 cells with established chemoresistance to 0.01 µM gemcitabine; Panc1-gemr/cisr = Panc1 cells with established chemoresistance to a combination of 0.01 µM gemcitabine and 80 µM cisplatin; cad = cadherin; Bkg = background; se = standard error.)

Downregulation of N cadherin was seen in all cell lines tested while there was a statistically significant upregulation in the epithelial marker E-Cadherin in all 4 cell lines tested with the exception of the double resistant pancreatic cell line (see Cisplatin/Gemcitabine of Figure 25). These results show that treatment with Oseltamivir Phosphate (Tamiflu) was able to reverse a partial EMT phenotype even in cell lines that were drug resistant. The mesenchymal phenotype is a hallmark of the IPRES signature predicting resistance to immunotherapy.

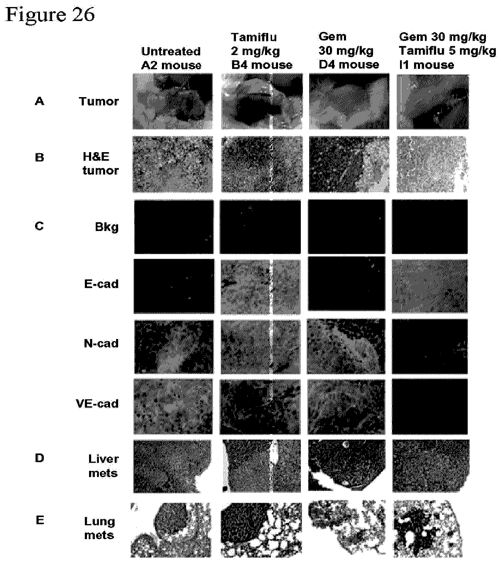

A similar result was obtained in vivo. Tumor tissue obtained from mouse xenografts heterotopically implanted with human PANC-1 cancer cells showed preservation of an epithelial phenotype given treatment with Oseltamivir phosphate (Tamiflu). Specifically, tumor tissue obtained from mice treated with a combination of Oseltamivir Phosphate with the chemotherapy drug Gemcitabine showed a significant downregulation of the mesenchymal marker N-Cadherin and a significant upregulation in the epithelial marker E-cadherin (see Figure 26). Treatment with chemotherapy alone showed an upregulated expression of N-cadherin and a downregulated expression of E-cadherin.

With reference to Figure 26, Fluorescence immunohistochemical detection of E-cadherin, N-cadherin, and VE-cadherin expression was performed in paraffin-embedded tumor tissues archived from xenograft tumors of PANC 1 cells growing in RAGxCγ double mutant mice. Mice were implanted with 1 $\times$ 106 PANC 1 cells cutaneously on the rear flank and treatment began at 22-23 days post implantation when tumors reached 100-200 mm³ as described above for Figure 25. (A) Live necropsy tumors. (B) H&E staining of tumor necropsy specimens. (C) Paraffin-embedded tumor sections (5 µm) on glass slides were processed for immunohistochemistry using primary anti-E-cadherin, N-cadherin, and VE-cadherin antibodies followed with polyclonal goat anti-rabbit Alexa Fluor® 488 (Life Technologies Inc, Burlington, ON, Canada) secondary antibody and Permount mounting media. Background control sections were prepared without the primary antibodies. Tissue sections were visualized and photographed using a Zeiss Imager M2 fluorescence microscope (Carl Zeiss AG, Oberkochen, Germany) at 400× magnification. Images are representative of at least five fields of view from two tumor sections. H&E staining of necropsy (**D**) liver and (**E**) lung for metastasis. (Abbreviation: H&E = hematoxylin and eosin staining; Bkg = background; cad = cadherin; mets = metastasis; Gem = gemcitabine.)

Besides a mesenchymal phenotype predicting resistance to the checkpoint inhibitors, resistance to PD-1 and PD-L1 blockers has also been found to be associated with upregulation of angiogenic pathways. Increased VEGF secretion reduces the function of effector T-cells and their migration to tumors (Ohm et al., 2003). Both Oseltamivir phosphate (O'Shea et al., 2014) and Cox 2 inhibitors (Sawaoka et al., 1999) have been shown to be highly effective therapeutic agents disrupting angiogenesis.

Oseltamivir Phosphate by itself was able to show a significant downregulation of the angiogenic marker AE-cadherin in human PANC1 cancer cell lines, including drug resistant cancer cell lines, with the exception of the cancer cell line double resistant to gemcitabine and cisplatin (see Figure 25). Furthermore, a similar result was obtained in tumor tissue obtained from mouse xenografts heterotopically implanted with human PANC-1 cancer cells. Specifically, tumor tissue obtained from mice treated with a combination of Oseltamivir Phosphate with the chemotherapy drug Gemcitabine showed a significant downregulation of the angiogenic maker AE-cadherin relative to treatment with chemotherapy alone (see Figure 26).

One important mechanism of resistance to immunotherapy is through the activation/upregulation of specific oncogenic signaling pathways. The upregulation of these pathways may be an important mechanism responsible for the distinct pattern of expression of PD-L1 and the corresponding responsiveness to PD-1/PD-L1 blockade therapy. In a retrospective analysis of 58 patients with non-small cell lung cancer (NSCLC) treated with PD-1/PD-L1 inhibitors, objective responses were observed in only 1 of 28 patients who harbored mutations in EGFR or ALK, while patients with wild type EGFR had a response rate to the checkpoint inhibitors of approximately 25%. Mutated EGFR signaling appears highly predictive of lack of response to immunotherapy with the checkpoint inhibitors. This is one important factor in extend immunotherapy treatments to other malignancies, as mutated EGFR has been found in many different types of malignancies besides NSCLC, including breast, head and neck, ovarian, and pancreatic cancers (Mendelsohn et al., 2003).

Another central oncogenic signaling pathway that appears to play a very important role in resistance to PD-1/PD-L1 blockade is the phosphatidylinositol 3-kinase (PI3K) pathway (Peng et al., 2016). Signaling through this pathway (PI3K/AKT/mTOR) has pleiotropic effects on cellular physiology including proliferation, apoptosis, and motility. A common way to upregulate this pathway is through loss of expression of the tumor suppressor gene PTEN (Peng et al., 2016). Metastatic melanoma patients who received anti-PD-1 antibodies with functional PTEN expression had a significant shrinkage in the size of their tumors relative to those patients with abnormal PTEN expression. Pathological examination of resected melanomas showed that PTEN loss was associated with a significantly lower cytotoxic T cell infiltration into the tumors relative to the melanomas with functional expression of PTEN. Loss of PTEN was also associated with an increase in the expression of angiogenic cytokines VEGF and CCL2, with a reduction in cytotoxic T cell infiltration into the tumors.

Because of the association between the upregulation and or mutation of a variety of signaling pathways including (PI3K/AKT/mTOR), mutant PTEN, mutated EGFR, and resistance to treatment with the checkpoint inhibitors, other therapeutic modalities may also downregulate these signaling pathways to render resistant tumors sensitive to immunotherapy treatments.

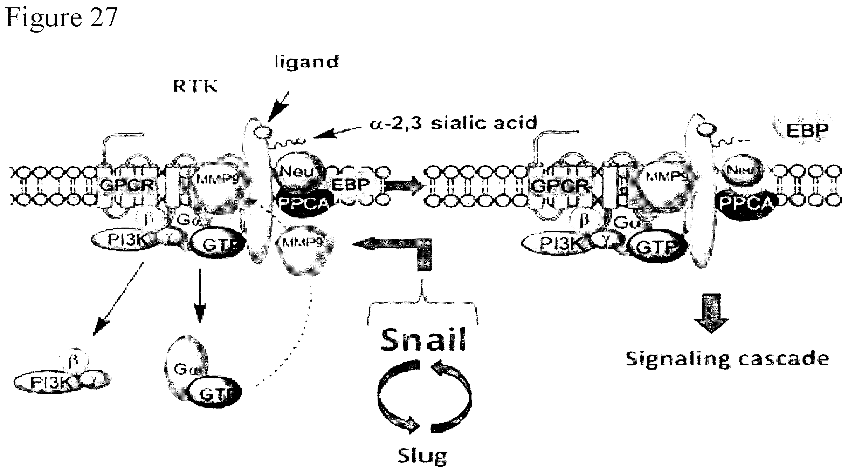

Firstly, the ability of inhibitors of the neuraminidase 1 enzyme to interfere with a number of these signaling pathways simultaneously were investigated. The EGFR receptor is closely associated with a G protein-coupled receptor (GPCR)-signaling platform essential for the activation of EGFR in pancreatic cancer (Uddin et al., 2010). Neuramindase 1 and MMP9 form a complex tethered at the ectodomain of EGFRs on the cell surface (see Figure 27).

Referring to Figure 27 (PRIOR ART), Snail and MMP9 expressions are closely connected in invasive tumor processes. Snail induces MMP9 secretion via multiple signaling pathways, but particularly in cooperation with oncogenic H-Ras (RasV12), Snail upregulates the transcription of MMP9. This Snail-MMP9 signaling axis is the connecting link to promote RTK glycosylation modification involving this novel receptor-signaling platform. Activated MMP9 is proposed to remove the elastin-binding protein (EBP) as part of the molecular multi-enzymatic complex that contains β-galactosidase/Neu1 and protective protein cathepsin A (PPCA) to induce Neu1. Activated Neu1 hydrolyzes α-2,3-sialic acid residues of the glycosylated receptors at the ectodomain to remove steric hindrance and to facilitate receptor association and activation. This process sets the stage for multistages of tumorigenesis. (Abbreviations: Neu1, neuraminidase-1; MMP, matrix metalloproteinase; PI3K, phosphatidylinositol 3-kinase; GTP, guanine triphosphate; GPCR, G protein-coupled receptor; EBP, elastin binding protein; PPCA, protective protein cathepsin A.)

EGF binding to its receptor causes a conformational change of EGFR, which results in the activation of neuromedin B GPCR (NMBR) also tethered to the receptor. Activated NMBR initiates Gαi-protein signaling which triggers the activation of MMP9 to subsequently induce Neu1 which hydrolyzes the α-2,3-sialyl residues linked to β-galactosides of EGFR. This process by Neu1 is predicted to remove steric hindrance of EGFR to facilitate receptor association/dimerization and downstream signaling. Disrupting this process by targeting Neuraminidase 1 using drugs such as Oseltamivir phosphate is predicted to prevent activation of downstream signaling pathways such as PI3K/AKT/mTOR activated by EFGR and other tyrosine kinase receptors with a similar signaling platform.

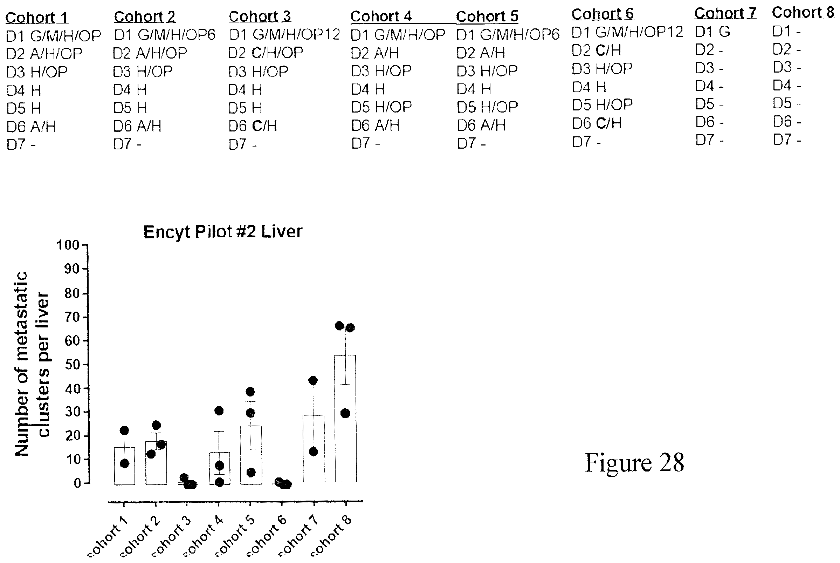

The PI3K/AKT/mTOR pathway and its association with resistance to checkpoint inhibitors, provides a mechanistic basis for other complementary treatments that would be effective at blocking this signaling platform. Cox inhibitors, particularly COX-2 inhibitors, were found to be very effective at diminishing the kinase activity of this pathway in patients with ovarian cancer (Uddin et al., 2010). A variety of different COX inhibitors in combination with Oseltamivir phosphate were investigated in mouse xenografts heterotopically implanted with human PANC-1 cancer cells. Surprisingly, treatment with a selective COX-2 inhibitor, Celecoxib, in combination with standard chemotherapy and Oseltamivir phosphate given at a dose of 50 mg per kilogram 3x weekly, was completely able to prevent the development of metastatic disease in the mouse cohorts given this combination (see Figure 28 Cohorts 3 and 6 treated with combination of OP with celecoxib). In particular, this is an highly metastatic cell line that is often resistant to therapy, including highly metastatic human PANC1 cell line in xenografts.

In one embodiment, a combination treatment with a neuraminidase 1 inhibitor such as Oseltamivir Phosphate (Tamiflu) and a cyclooxygenase inhibitor such as Celecoxib.

In one embodiment, the COX-2 inhibitor is nimesulide, celecoxib, meloxicam, etodolac, or rofecoxib. In preferred embodiments, COX-2 inhibitor is celecoxib.

In some embodiments, Oseltamivir Phosphate is administered at a dose of between about 1 mg/kg and 500 mg/kg, e.g. 1 mg/kg, 5 mg/kg, 10 mg/kg, 50 mg/kg, 100mg/kg, or 500 mg/kg; preferably, 50 mg/kg.

In some embodiments, celecoxib is administered at 50 mg daily, 100 mg daily, 200 mg daily, or 400 mg daily; or preferably 200 mg daily.

In one embodiment, the neuraminidase 1 inhibitor and the cyclooxygenase (COX) inhibitor is administered prior to the immunotherapy. In some embodiments they are administered for 24 hours to 10 days prior to chemotherapy, including immunotherapy. In some embodiments, they are administered for 48 hours to 7 days prior to chemotherapy, including immunotherapy. In preferred embodiments they are administered for 7 days prior to chemotherapy, including immunotherapy.

The results shown in Figure 28, provides strong support for the combination treatment with an inhibitor of neuraminidase 1 coupled with a cyclooxygenase 2 inhibitor such as celecoxib to prevent an induction of a partial EMT in vivo, given the known association between metastatic competency and reversion towards a mesenchymal phenotype (Mani et al., 2008). Furthermore, given the ability of OP to reverse an EMT phenotype in vitro (see Figure 25), treatment with a combination neuraminidase 1 inhibitor such as Oseltamivir phosphate plus a cyclooxygenase inhibitor such as celecoxib represents a novel treatment strategy that can be used to sensitize primary resistant tumors to immunotherapy with checkpoint inhibitors. Furthermore, similar therapeutic strategy can be used to reverse or prevent acquired resistance to checkpoint inhibitors given the ability to reverse the mesenchymal phenotype using a neuraminidase inhibitor such as oseltamivir phosphate (Figure 25).

**References:**

1. Sharma, P and Allison J.P. The future of immune checkpoint therapy, Science 348 (6230) (2015) 56-61.

2. Hugo, W et al. Genomic and transcriptomic features of response to anti-PD-1 therapy in metastatic melanoma, Cell 165 (1) (2016) 35-44.

3. O'Shea, Leah K et al. "Therapeutic Targeting of Neu1 Sialidase with Oseltamivir Phosphate (Tamiflu®) Disables Cancer Cell Survival in Human Pancreatic Cancer with Acquired Chemoresistance." OncoTargets and therapy 7 (2014): 117-134.

4. Ohm, J.E et al..VEGF inhibits T-cell development and may contribute to tumor-induced immune suppression, Blood 101 (12) (2003) 4878-4886.

5. Sawaoka, H et al. Cyclooxygenase inhibitors suppress angiogenesis and reduce tumor growth in vivo. Lab Investigation 79 (12) (1999):1469-77.

6. Mendelsohn J and Baselga J. Status of epidermal growth factor receptor antagonists in the biology and treatment of cancer. J Clin Oncol. 2003; 21(14):2787-2799.

7. Peng, W. et al. Loss of PTEN promotes resistance to T cell-mediated immunotherapy, Cancer Discov. 6 (2) (2016) 202-216.

8. Uddin, S. et al. Cyclooxygenase-2 inhibition inhibits PI3K/AKT kinase activity in epithelial ovarian cancer. International Journal of Cancer 126, (2010) 382-394.

9. Mani, S. A.. et al. "The Epithelial-Mesenchymal Transition Generates Cells with Properties of Stem Cells." Cell 133.4 (2008): 704-715.

10. Zhang, Hui-Hui and Guo, Xiu-Li, "Combinational strategies of metformin and chemotherapy in cancers" Cancer Chemother. Pharmacol. 2016; 78:13-26

**Claims**

1. An anti-cancer cytotoxic therapeutic agent, metformin, a neu-1 sialidase inhibitor and aspirin for use in an anti cancer treatment regimen in a patient with cancer, said treatment regimen comprising a treatment cycle comprising the steps of:

   administering said anti-cancer cytotoxic therapeutic agent to the patient with cancer on day 1 of the treatment cycle;
   administering the metformin to the patient on days 2, 3, 4, and/or 5 of the treatment cycle;
   administering the neu-1 sialidase inhibitor to the patient on days 2, 3, 4, and/or 5 of the treatment cycle;
   administering the aspirin to the patient on days 2, 3, 4, and/or 5 of the treatment cycle; and
   wherein the anti-cancer cytotoxic therapeutic agent is selected from alkylators, cytotoxic antibiotics, topoisomerase inhibitors 1 and/or 2, taxanes, vinca alkaloids and anti metabolites; and
   wherein the neu-1 sialidase inhibitor is oseltamivir phosphate.

2. The anti-cancer cytotoxic therapeutic agent, metformin and the neu-1 sialidase inhibitor and aspirin for use according to claim 1, wherein the anti-cancer cytotoxic therapeutic agent administered on day 1 comprises an antimetabolite.

3. The anti-cancer cytotoxic therapeutic agent, metformin and the neu-1 sialidase inhibitor and aspirin for use according to claim 2, wherein the antimetabolite is gemcitabine.

4. The anti-cancer cytotoxic therapeutic agent, metformin and the neu-1 sialidase inhibitor and aspirin for use according to any of claims 1 to 3, wherein the anti-cancer cytotoxic therapeutic agent is selected from

   - busulphan, carmustine, carboplatin, chlorambucil, cyclophosphamide, cisplatin, dacarbazine, estramustine, lomustine, melphalan, thiotepa and treosulphan;
   - anthracyclines, in particular selected from the group consisting of doxorubicin, idarubicin, epirubicin, aclarubicin and mitozantrone;
   - doxorubicin, irinotecan, etoposide and topotecan;
   - docetaxel, paclitaxel, and abraxane;
   - vincristine, vinblastine, vindesine and vinorelbine; and
   - 5-FU, gemcitabine, cladribine, cytarabine, fludarabine, mercaptopurine, methotexate, pemetrexed, pentostatin and tioguanine.

5. The anti-cancer cytotoxic therapeutic agent, metformin and the neu-1 sialidase inhibitor and aspirin for use according to any one of claims 1 to 4, wherein the patient has pancreatic cancer.

**Patentansprüche**

1. Ein zytotoxisches Antikrebsmittel, Metformin, ein Neu-1-Sialidase-Inhibitor und Aspirin zur Verwendung in einem Behandlungsschema gegen Krebs bei einem Patienten mit Krebs, wobei das Behandlungsschema einen Behandlungszyklus umfasst, der die folgenden Schritte umfasst:

   Verabreichung des zytotoxischen Therapeutikums gegen Krebs an den Patienten mit Krebs am Tag 1 des Behandlungszyklus;
   Verabreichung des Metformins an den Patienten an den Tagen 2, 3, 4 und/oder 5 des Behandlungszyklus;
   Verabreichung des Neu-1-Sialidase-Inhibitors an den Patienten an den Tagen 2, 3, 4 und/oder 5 des Behandlungszyklus;
Verabreichung des Aspirins an den Patienten an den Tagen 2, 3, 4 und/oder 5 des Behandlungszyklus; und
wobei das zytotoxische Antikrebsmittel aus Alkylatoren, zytotoxischen Antibiotika, Topoisomerase-Inhibitoren 1
und/oder 2, Taxanen, Vinca-Alkaloiden und Antimetaboliten ausgewählt ist; und
wobei der Neu-1-Sialidase-Inhibitor Oseltamivirphosphat ist.

2. Das zytotoxische Antikrebsmittel, Metformin und der Neu-1-Sialidase-Inhibitor und Aspirin zur Verwendung gemäß Anspruch 1, wobei das am Tag 1 verabreichte zytotoxische Antikrebsmittel einen Antimetaboliten umfasst.

3. Das zytotoxische Antikrebsmittel, Metformin und der Neu-1-Sialidase-Inhibitor und Aspirin zur Verwendung gemäß Anspruch 2, wobei der Antimetabolit Gemcitabin ist.

4. Das Antikrebsmittel, Metformin und der Neu-1-Sialidase-Inhibitor und Aspirin zur Verwendung gemäß einem der Ansprüche 1 bis 3, wobei das zytotoxische Antikrebsmittel ausgewählt ist aus

- Busulfan, Carmustin, Carboplatin, Chlorambucil, Cyclophosphamid, Cisplatin, Dacarbazin, Estramustin, Lomustin, Melphalan, Thiotepa und Treosulfan;
- Anthrazykline, insbesondere ausgewählt aus der Gruppe bestehend aus Doxorubicin, Idarubicin, Epirubicin, Aclarubicin und Mitozantron;
- Doxorubicin, Irinotecan, Etoposid und Topotecan;
- Docetaxel, Paclitaxel und Abraxane;
- Vincristin, Vinblastin, Vindesin und Vinorelbin; und
- 5-FU, Gemcitabin, Cladribin, Cytarabin, Fludarabin, Mercaptopurin, Methotrexat, Pemetrexed, Pentostatin und Tioguanin.

5. Das zytotoxische Antikrebsmittel, Metformin und der Neu-1-Sialidase-Inhibitor und Aspirin zur Verwendung gemäß einem der Ansprüche 1 bis 4, wobei der Patient an Bauchspeicheldrüsenkrebs leidet.


**Revendications**

1. Agent thérapeutique cytotoxique anticancéreux, metformine, inhibiteur de neu-1 sialidase et aspirine pour utilistaion dans un régime de traitement anticancéreux chez un patient atteint de cancer, ledit régime de traitement comprenant un cycle de traitement comprenant les étapes suivantes:

administrer ledit agent thérapeutique cytotoxique anticancéreux au patient atteint de cancer le jour 1 du cycle de traitement;
administrer la metformine au patient les jours 2, 3, 4 et/ou 5 du cycle de traitement;
administrer l'inhibiteur de neu-1 sialidase au patient les jours 2, 3, 4 et/ou 5 du cycle de traitement ;
administrer l'aspirine au patient les jours 2, 3, 4 et/ou 5 du cycle de traitement; et
dans lequels l'agent thérapeutique cytotoxique anticancéreux est choisi parmi les alkylants, les antibiotiques cytotoxiques, les inhibiteurs de topoisomérase 1 et/ou 2, les taxanes, les alcaloïdes de la pervenche et les antimétabolites; et
dans lequels l'inhibiteur de neu-1 sialidase est le phosphate d'oseltamivir.

2. Agent thérapeutique cytotoxique anticancéreux, metformine et inhibiteur de neu-1 sialidase et aspirine pour utilisation selon la revendication 1, dans lesquels l'agent thérapeutique cytotoxique anticancéreux administré le jour 1 comprend un antimétabolite.

3. Agent thérapeutique cytotoxique anticancéreux, metformine et inhibiteur de neu-1 sialidase et aspirine pour utilisation selon la revendication 2, dans lequels l'antimétabolite est la gemcitabine.

4. Agent thérapeutique cytotoxique anticancéreux, metformine et inhibiteur de neu-1 sialidase et aspirine pour utilisation selon l'une quelconque des revendications 1 à 3, dans lequels l'agent thérapeutique cytotoxique anticancéreux est choisi parmi

- le busulfan, la carmustine, le carboplatine, le chlorambucil, le cyclophosphamide, le cisplatine, la dacarbazine, l'estramustine, la lomustine, le melphalan, le thiotépa et le tréosulfan ;

- les anthracyclines choisies, en particulier, dans le groupe constitué par la doxorubicine, l'idarubicine, l'épirubicine, l'aclarubicine et le mitozantrone ;
- la doxorubicine, l'irinotécan, l'étoposide et le topotécan ;
- le docétaxel, le paclitaxel et l'abraxane ;
- la vincristine, la vinblastine, la vindésine et la vinorelbine ; et
- le 5-FU, la gemcitabine, la cladribine, la cytarabine, la fludarabine, la mercaptopurine, le méthotrexate, le pemetrexed, la pentostatine et la tioguanine.

5. Agent thérapeutique cytotoxique anticancéreux, metformine et inhibiteur de neu-1 sialidase et aspirine pour utilisation selon selon l'une quelconque des revendications 1 à 4, dans lesquels le patient est atteint d'un cancer du pancréas.

# FIG. 1

# FIG. 2a

## No Cytokines

R4

EpCAM-A488, CD44-PE

| Population | Count | %Total | %Gated | %Plotted |
|---|---|---|---|---|
| R4 & R3 & R2 & R1 | 182 | 0.26 | 100 | 100 |
| R5 & R4 & R3 & R2 & R1 | 130 | 0.18 | 71.4 | 100 |
| R6 & R4 & R3 & R2 & R1 | 4 | 0.01 | 2.2 | 100 |
| R7 & R4 & R3 & R2 & R1 | 8 | 0.01 | 4.4 | 100 |
| R8 & R4 & R3 & R2 & R1 | 40 | 0.06 | 22 | 100 |

# FIG. 2b

## Full Cocktail – IL-6 + HGF + PGE-2 + PGF + MMP-9

EpCAM-A488, CD44-PE

| Population | Count | %Total | %Gated | %Plotted |
|---|---|---|---|---|
| R4 & R3 & R2 & R1 | 762 | 0.95 | 100 | 100 |
| R5 & R4 & R3 & R2 & R1 | 481 | 0.6 | 63.1 | 100 |
| R6 & R4 & R3 & R2 & R1 | 73 | 0.09 | 9.58 | 100 |
| R7 & R4 & R3 & R2 & R1 | 14 | 0.02 | 1.84 | 100 |
| R8 & R4 & R3 & R2 & R1 | 181 | 0.23 | 23.8 | 100 |

# FIG. 2c

IL-6

EpCAM-A488, CD44-PE

| Population | Count | %Total | %Gated | %Plotted |
|---|---|---|---|---|
| R4 & R3 & R2 & R1 | 635 | 1.17 | 100 | 100 |
| R5 & R4 & R3 & R2 & R1 | 349 | 0.64 | 55 | 100 |
| R6 & R4 & R3 & R2 & R1 | 39 | 0.07 | 6.14 | 100 |
| R7 & R4 & R3 & R2 & R1 | 17 | 0.03 | 2.68 | 100 |
| R8 & R4 & R3 & R2 & R1 | 226 | 0.42 | 35.6 | 100 |

# FIG. 2d

## PGE-2

### EpCAM-A488, CD44-PE

| Population | Count | %Total | %Gated | %Plotted |
|---|---|---|---|---|
| R4 & R3 & R2 & R1 | 685 | 0.69 | 100 | 100 |
| R5 & R4 & R3 & R2 & R1 | 449 | 0.45 | 65.5 | 100 |
| R6 & R4 & R3 & R2 & R1 | 29 | 0.03 | 4.23 | 100 |
| R7 & R4 & R3 & R2 & R1 | 12 | 0.01 | 1.75 | 100 |
| R8 & R4 & R3 & R2 & R1 | 186 | 0.19 | 27.2 | 100 |

# FIG. 2e

HGF

EpCAM-A488, CD44-PE

| Population | Count | %Total | %Gated | %Plotted |
|---|---|---|---|---|
| R4 & R3 & R2 & R1 | 613 | 1.09 | 100 | 100 |
| R5 & R4 & R3 & R2 & R1 | 459 | 0.81 | 74.9 | 100 |
| R6 & R4 & R3 & R2 & R1 | 13 | 0.02 | 2.12 | 100 |
| R7 & R4 & R3 & R2 & R1 | 30 | 0.05 | 4.89 | 100 |
| R8 & R4 & R3 & R2 & R1 | 103 | 0.18 | 16.8 | 100 |

# FIG. 2f

## TGF-Beta

EpCAM-A488, CD44-PE

| Population | Count | %Total | %Gated | %Plotted |
|---|---|---|---|---|
| R4 & R3 & R2 & R1 | 546 | 1.04 | 100 | 100 |
| R5 & R4 & R3 & R2 & R1 | 393 | 0.75 | 72 | 100 |
| R6 & R4 & R3 & R2 & R1 | 17 | 0.03 | 3.11 | 100 |
| R7 & R4 & R3 & R2 & R1 | 27 | 0.05 | 4.95 | 100 |
| R8 & R4 & R3 & R2 & R1 | 108 | 0.21 | 19.8 | 100 |

# FIG. 2g

## IL-6 + PGE-2 + TGF-Beta

EpCAM-A488, CD44-PE

| Population | Count | %Total | %Gated | %Plotted |
|---|---|---|---|---|
| R4 & R3 & R2 & R1 | 895 | 0.92 | 100 | 100 |
| R5 & R4 & R3 & R2 & R1 | 699 | 0.72 | 78.1 | 100 |
| R6 & R4 & R3 & R2 & R1 | 25 | 0.03 | 2.79 | 100 |
| R7 & R4 & R3 & R2 & R1 | 18 | 0.02 | 2.01 | 100 |
| R8 & R4 & R3 & R2 & R1 | 146 | 0.15 | 16.3 | 100 |

# FIG. 2h

## HGF + PGE-2 + TGF-Beta

R4

EpCAM-A488, CD44-PE

| Population | Count | %Total | %Gated | %Plotted |
|---|---|---|---|---|
| R4 & R3 & R2 & R1 | 628 | 0.96 | 100 | 100 |
| R5 & R4 & R3 & R2 & R1 | 484 | 0.74 | 77.1 | 100 |
| R6 & R4 & R3 & R2 & R1 | 11 | 0.02 | 1.75 | 100 |
| R7 & R4 & R3 & R2 & R1 | 14 | 0.02 | 2.23 | 100 |
| R8 & R4 & R3 & R2 & R1 | 113 | 0.17 | 18 | 100 |

# FIG. 2i

## IL-6 + HGF + TGF-Beta

EpCAM-A488, CD44-PE

| Population | Count | %Total | %Gated | %Plotted |
|---|---|---|---|---|
| R4 & R3 & R2 & R1 | 577 | 1.21 | 100 | 100 |
| R5 & R4 & R3 & R2 & R1 | 392 | 0.82 | 67.9 | 100 |
| R6 & R4 & R3 & R2 & R1 | 16 | 0.03 | 2.77 | 100 |
| R7 & R4 & R3 & R2 & R1 | 13 | 0.03 | 2.25 | 100 |
| R8 & R4 & R3 & R2 & R1 | 150 | 0.31 | 26 | 100 |

# FIG. 2j

## IL6 + HGF + PGE-2

EpCAM-A488, CD44-PE

| Population | Count | %Total | %Gated | %Plotted |
|---|---|---|---|---|
| R4 & R3 & R2 & R1 | 745 | 0.74 | 100 | 100 |
| R5 & R4 & R3 & R2 & R1 | 487 | 0.49 | 65.4 | 100 |
| R6 & R4 & R3 & R2 & R1 | 40 | 0.04 | 5.37 | 100 |
| R7 & R4 & R3 & R2 & R1 | 17 | 0.02 | 2.28 | 100 |
| R8 & R4 & R3 & R2 & R1 | 190 | 0.19 | 25.5 | 100 |

# FIG. 3a

## No Cytokines

EpCAM-A488, CD133-PE

| Population | Count | %Total | %Gated | %Plotted |
|---|---|---|---|---|
| R4 & R3 & R2 & R1 | 465 | 0.57 | 100 | 100 |
| R5 & R4 & R3 & R2 & R1 | 4 | 0 | 0.86 | 100 |
| R6 & R4 & R3 & R2 & R1 | 314 | 0.38 | 67.5 | 100 |
| R7 & R4 & R3 & R2 & R1 | 2 | 0 | 0.43 | 100 |
| R8 & R4 & R3 & R2 & R1 | 145 | 0.18 | 31.2 | 100 |

# FIG. 3b

## Full Cocktail - IL-6 + HGF + PGE-2 + PGF + MMP-9

EpCAM-A488, CD133-PE

| Population | Count | %Total | %Gated | %Plotted |
|---|---|---|---|---|
| R4 & R3 & R2 & R1 | 643 | 0.64 | 100 | 100 |
| R5 & R4 & R3 & R2 & R1 | 14 | 0.01 | 2.18 | 100 |
| R6 & R4 & R3 & R2 & R1 | 484 | 0.48 | 75.3 | 100 |
| R7 & R4 & R3 & R2 & R1 | 3 | 0 | 0.47 | 100 |
| R8 & R4 & R3 & R2 & R1 | 139 | 0.14 | 21.6 | 100 |

# FIG. 3c

## IL-6

EpCAM-A488, CD133-PE

| Population | Count | %Total | %Gated | %Plotted |
|---|---|---|---|---|
| R4 & R3 & R2 & R1 | 549 | 0.58 | 100 | 100 |
| R5 & R4 & R3 & R2 & R1 | 8 | 0.01 | 1.46 | 100 |
| R6 & R4 & R3 & R2 & R1 | 410 | 0.44 | 74.7 | 100 |
| R7 & R4 & R3 & R2 & R1 | 0 | 0 | 0 | 0 |
| R8 & R4 & R3 & R2 & R1 | 129 | 0.14 | 23.5 | 100 |

# FIG. 3d

HGF

EpCAM-A488, CD133-PE

| Population | Count | %Total | %Gated | %Plotted |
|---|---|---|---|---|
| R4 & R3 & R2 & R1 | 505 | 0.66 | 100 | 100 |
| R5 & R4 & R3 & R2 & R1 | 8 | 0.01 | 1.58 | 100 |
| R6 & R4 & R3 & R2 & R1 | 408 | 0.53 | 80.8 | 100 |
| R7 & R4 & R3 & R2 & R1 | 1 | 0 | 0.2 | 100 |
| R8 & R4 & R3 & R2 & R1 | 87 | 0.11 | 17.2 | 100 |

# FIG. 3e

PGE-2

R4

EpCAM-A488, CD133-PE

| Population | Count | %Total | %Gated | %Plotted |
|---|---|---|---|---|
| R4 & R3 & R2 & R1 | 345 | 0.38 | 100 | 100 |
| R5 & R4 & R3 & R2 & R1 | 9 | 0.01 | 2.61 | 100 |
| R6 & R4 & R3 & R2 & R1 | 299 | 0.33 | 86.7 | 100 |
| R7 & R4 & R3 & R2 & R1 | 0 | 0 | 0 | 0 |
| R8 & R4 & R3 & R2 & R1 | 36 | 0.04 | 10.4 | 100 |

# FIG. 3f

## TGF-Beta

EpCAM-A488, CD133-PE

| Population | Count | %Total | %Gated | %Plotted |
|---|---|---|---|---|
| R4 & R3 & R2 & R1 | 374 | 0.5 | 100 | 100 |
| R5 & R4 & R3 & R2 & R1 | 10 | 0.01 | 2.67 | 100 |
| R6 & R4 & R3 & R2 & R1 | 325 | 0.44 | 86.9 | 100 |
| R7 & R4 & R3 & R2 & R1 | 0 | 0 | 0 | 0 |
| R8 & R4 & R3 & R2 & R1 | 38 | 0.05 | 10.2 | 100 |

# FIG. 3g

## HGF + PGE-2 + TGF-Beta

EpCAM-A488, CD133-PE

| Population | Count | %Total | %Gated | %Plotted |
|---|---|---|---|---|
| R4 & R3 & R2 & R1 | 255 | 0.28 | 100 | 100 |
| R5 & R4 & R3 & R2 & R1 | 15 | 0.02 | 5.88 | 100 |
| R6 & R4 & R3 & R2 & R1 | 213 | 0.23 | 83.5 | 100 |
| R7 & R4 & R3 & R2 & R1 | 0 | 0 | 0 | 0 |
| R8 & R4 & R3 & R2 & R1 | 27 | 0.03 | 10.6 | 100 |

# FIG. 3h

## IL-6 + HGF + TGF-Beta

EpCAM-A488, CD133-PE

| Population | Count | %Total | %Gated | %Plotted |
|---|---|---|---|---|
| R4 & R3 & R2 & R1 | 473 | 0.57 | 100 | 100 |
| R5 & R4 & R3 & R2 & R1 | 7 | 0.01 | 1.48 | 100 |
| R6 & R4 & R3 & R2 & R1 | 391 | 0.47 | 82.7 | 100 |
| R7 & R4 & R3 & R2 & R1 | 1 | 0 | 0.21 | 100 |
| R8 & R4 & R3 & R2 & R1 | 74 | 0.09 | 15.6 | 100 |

# FIG. 3i

## IL-6 + PGE-2 + TGF-Beta

EpCAM-A488, CD133-PE

| Population | Count | %Total | %Gated | %Plotted |
|---|---|---|---|---|
| R4 & R3 & R2 & R1 | 513 | 0.74 | 100 | 100 |
| R5 & R4 & R3 & R2 & R1 | 4 | 0.01 | 0.78 | 100 |
| R6 & R4 & R3 & R2 & R1 | 412 | 0.6 | 80.3 | 100 |
| R7 & R4 & R3 & R2 & R1 | 1 | 0 | 0.19 | 100 |
| R8 & R4 & R3 & R2 & R1 | 95 | 0.14 | 18.5 | 100 |

# FIG. 3j

## IL-6 + HGF + PGE-2

EpCAM-A488, CD133-PE

| Population | Count | %Total | %Gated | %Plotted |
|---|---|---|---|---|
| R4 & R3 & R2 & R1 | 458 | 0.54 | 100 | 100 |
| R5 & R4 & R3 & R2 & R1 | 6 | 0.01 | 1.31 | 100 |
| R6 & R4 & R3 & R2 & R1 | 408 | 0.49 | 89.1 | 100 |
| R7 & R4 & R3 & R2 & R1 | 0 | 0 | 0 | 0 |
| R8 & R4 & R3 & R2 & R1 | 43 | 0.05 | 9.39 | 100 |

# FIG. 4

Cell proliferation in HCT-15 Cell line

# FIG. 5

**CELL PROLIFERATION IN SW 620 CELL LINE**

■ CD133-CD44+   ▨ CD133+CD44-   ▨ CD133+CD44+

# FIG. 6

## Cell Proliferation in Cultured CTCs
## (EpCAM, CD44 Abs)

■ EpCAM-CD44+    ⊠ EpCAM+CD44-    ⊠ EpCAM+CD44+

# FIG. 7

Cell Proliferation in Cultured CTCs
(EpCAM, CD133 Abs)

# FIG. 8

Evidence of EMT in Patient Blood Sample

# FIG. 9a

R3 - Before Culture

EpCAM A488, CD133 PE

| Population | Count | % Total | % Gated | % Plotted |
|---|---|---|---|---|
| R3 & R2 & R1 | 29332 | 55.3 | 100 | 100 |
| R4 & R3 & R2 & R1 | 29298 | 55.3 | 99.9 | 100 |
| R5 & R3 & R2 & R1 | 4 | 0.01 | 0.01 | 100 |
| R6 & R3 & R2 & R1 | 1 | 0 | 0 | 100 |
| R7 & R3 & R2 & R1 | 14 | 0.03 | 0.05 | 100 |

# FIG. 9b

R3 - Before Culture

## EpCAM A488, Lgr5 PE-Vio770

| Population | Count | %Total | %Gated | %Plotted |
|---|---|---|---|---|
| R3 & R2 & R1 | 29332 | 55.3 | 100 | 100 |
| R8 & R3 & R2 & R1 | 29181 | 55 | 99.5 | 100 |
| R9 & R3 & R2 & R1 | 125 | 0.24 | 0.43 | 100 |
| R10 & R3 & R2 & R1 | 1 | 0 | 0 | 100 |
| R11 & R3 & R2 & R1 | 0 | 0 | 0 | 0 |

# FIG. 9c

R3 - Control

EpCAM A488, CD133 PE

| Population | Count | %Total | %Gated | %Plotted |
|---|---|---|---|---|
| R3 & R2 & R1 | 9868 | 22.2 | 100 | 100 |
| R4 & R3 & R2 & R1 | 9126 | 20.5 | 92.5 | 100 |
| R5 & R3 & R2 & R1 | 16 | 0.04 | 0.16 | 100 |
| R6 & R3 & R2 & R1 | 21 | 0.05 | 0.21 | 100 |
| R7 & R3 & R2 & R1 | 197 | 0.44 | 2 | 100 |

# FIG. 9d

R3 - IL6

EpCAM A488, CD133 PE

| Population | Count | %Total | %Gated | %Plotted |
|---|---|---|---|---|
| R3 & R2 & R1 | 15580 | 24.3 | 100 | 100 |
| R4 & R3 & R2 & R1 | 14094 | 22 | 90.5 | 100 |
| R5 & R3 & R2 & R1 | 18 | 0.03 | 0.12 | 100 |
| R6 & R3 & R2 & R1 | 16 | 0.02 | 0.1 | 100 |
| R7 & R3 & R2 & R1 | 465 | 0.73 | 2.98 | 100 |

# FIG. 9e

R3 - IL8

**EpCAM A488, CD133 PE**

| Population | Count | %Total | %Gated | %Plotted |
|---|---|---|---|---|
| R3 & R2 & R1 | 13442 | 20.6 | 100 | 100 |
| R4 & R3 & R2 & R1 | 12203 | 18.7 | 90.8 | 100 |
| R5 & R3 & R2 & R1 | 12 | 0.02 | 0.09 | 100 |
| R6 & R3 & R2 & R1 | 18 | 0.03 | 0.13 | 100 |
| R7 & R3 & R2 & R1 | 337 | 0.52 | 2.51 | 100 |

# FIG. 9f

### R3 - PDGF BB

EpCAM A488

### EpCAM A488, CD133 PE

| Population | Count | %Total | %Gated | %Plotted |
|---|---|---|---|---|
| R3 & R2 & R1 | 14262 | 20.6 | 100 | 100 |
| R4 & R3 & R2 & R1 | 12908 | 18.6 | 90.5 | 100 |
| R5 & R3 & R2 & R1 | 14 | 0.02 | 0.1 | 100 |
| R6 & R3 & R2 & R1 | 25 | 0.04 | 0.18 | 100 |
| R7 & R3 & R2 & R1 | 327 | 0.47 | 2.29 | 100 |

# FIG. 9g

## R3 - Control

EpCAM A488 (x-axis), Lgr5 PE-Vio770 (y-axis)

## EpCAM A488, Lgr5 PE-Vio770

| Population | Count | %Total | %Gated | %Plotted |
|---|---|---|---|---|
| R3 & R2 & R1 | 9868 | 22.2 | 100 | 100 |
| R8 & R3 & R2 & R1 | 9473 | 21.3 | 96 | 100 |
| R9 & R3 & R2 & R1 | 327 | 0.74 | 3.31 | 100 |
| R10 & R3 & R2 & R1 | 4 | 0.01 | 0.04 | 100 |
| R11 & R3 & R2 & R1 | 31 | 0.07 | 0.31 | 100 |

# FIG. 9h

R3  - IL6

EpCAM A488, Lgr5 PE-Vio770

| Population | Count | %Total | %Gated | %Plotted |
|---|---|---|---|---|
| R3 & R2 & R1 | 15580 | 24.3 | 100 | 100 |
| R8 & R3 & R2 & R1 | 14560 | 22.7 | 93.5 | 100 |
| R9 & R3 & R2 & R1 | 887 | 1.38 | 5.69 | 100 |
| R10 & R3 & R2 & R1 | 6 | 0.01 | 0.04 | 100 |
| R11 & R3 & R2 & R1 | 44 | 0.07 | 0.28 | 100 |

# FIG. 9i

EpCAM A488, Lgr5 PE-Vio770

| Population | Count | %Total | %Gated | %Plotted |
|---|---|---|---|---|
| R3 & R2 & R1 | 13442 | 20.6 | 100 | 100 |
| R8 & R3 & R2 & R1 | 12652 | 19.4 | 94.1 | 100 |
| R9 & R3 & R2 & R1 | 620 | 0.95 | 4.61 | 100 |
| R10 & R3 & R2 & R1 | 16 | 0.02 | 0.12 | 100 |
| R11 & R3 & R2 & R1 | 72 | 0.11 | 0.54 | 100 |

# FIG. 9j

R3 - PDGF BB

EpCAM A488

## EpCAM A488, Lgr5 PE-Vio770

| Population | Count | %Total | %Gated | %Plotted |
|---|---|---|---|---|
| R3 & R2 & R1 | 14262 | 20.6 | 100 | 100 |
| R8 & R3 & R2 & R1 | 13512 | 19.5 | 94.7 | 100 |
| R9 & R3 & R2 & R1 | 586 | 0.85 | 4.11 | 100 |
| R10 & R3 & R2 & R1 | 21 | 0.03 | 0.15 | 100 |
| R11 & R3 & R2 & R1 | 75 | 0.11 | 0.53 | 100 |

# FIG. 10

**% CD44+CD133-**

# FIG. 11

% CD44+CD1433-

# FIG. 12

**% CD44+CD133-**

# FIG. 13

**% Cellular Apoptosis (efluor 506)**

# FIG. 14

**% Cellular Apoptosis (efluor 506)**

# FIG. 15

## % Cellular Apoptosis (efluor 506)

# FIG. 16

**% CD44-CD133+**

# FIG. 17

## % CD44-CD133+

# FIG. 18

**% CD44-CD133+**

# FIG. 19

**% CD44+CD133+**

# FIG. 20

% CD44+CD133+

# FIG. 21

**% CD44+CD133+**

# FIG. 22

**Cage 4 TV**

| | D1 | D2 | D3 | D4 | D5 | D6 |
|---|---|---|---|---|---|---|
| Cohort4 (4) | G/H | A/M/H/O | M/H/O | M/H/O | M/H/O | A/H |

**Cohort 4: Gem + Aspirin + Heparin + Metformin+ Oselt**

Treatment protocol dosages (in 0.2mL PBS I.P.):
Gem: (30 mg/kg) - 0.78mg (D1 1x weekly)
A: (100 μg/g) - 2.6mg
H: (6 mg/kg) - 0.156mg
M: (15mg/kg) - 0.39mg
O: (20 mg/kg) - 0.52mg

# FIG. 23

% TV reduce D3 post Gem

% Tumor volume redcution D3 post Gem

42.0%
29.2%
33.3%

Cohort 1: untreated
Cohort 2: G-A-M-H
Cohort 3: Gem
Cohort 4: G-A-M-H-O

% TV reduce D5 post Gem

% Tumor volume redcution D5 post Gem

48.5%
5.6%
55.4%

Cohort 1: untreated
Cohort 2: G-A-M-H
Cohort 3: Gem
Cohort 4: G-A-M-H-O

## Figure 24

**A**

**B**

| Cohort | Treatment |
|---|---|
| 1 | ASA and OP on Day 1 |
| 7 | Gem on Day 1 |
| 8 | Untreated CTL |

Figure 25

PANC1
CisR 80μM

Phase · E-cad · Phase · N-cad · Phase · VE-cad

Untreated

Tamiflu

No 1⁰ Ab

E-cadherin

N-cadherin

VE-cadherin

$P=0.0635$

$P=0.0000$

Figure 26

Figure 27

| Cohort 1 | Cohort 2 | Cohort 3 | Cohort 4 | Cohort 5 | Cohort 6 | Cohort 7 | Cohort 8 |
|---|---|---|---|---|---|---|---|
| D1 G/M/H/OP | D1 G/M/H/OP6 | D1 G/M/H/OP12 | D1 G/M/H/OP | D1 G/M/H/OP6 | D1 G/M/H/OP12 | D1 G | D1 - |
| D2 A/H/OP | D2 A/H/OP | D2 C/H/OP | D2 A/H | D2 A/H | D2 C/H | D2 - | D2 - |
| D3 H/OP | D3 H/OP | D3 H/OP | D3 H/OP | D3 H/OP | D3 H/OP | D3 - | D3 - |
| D4 H | D4 H | D4 H | D4 H | D4 H | D4 H | D4 - | D4 - |
| D5 H | D5 H | D5 H | D5 H/OP | D5 H/OP | D5 H/OP | D5 - | D5 - |
| D6 A/H | D6 A/H | D6 C/H | D6 A/H | D6 A/H | D6 C/H | D6 - | D6 - |
| D7 - | D7 - | D7 - | D7 - | D7 - | D7 - | D7 - | D7 - |

Figure 28

## REFERENCES CITED IN THE DESCRIPTION

### Patent documents cited in the description

- US 5670373 A
- US 5866689 A
- CA 2700498
- CA 2763039
- CA 2632628
- US 623528 A
- US 5959085 A
- US 7482436 B
- US 7718174 B
- CA 2472383
- CA 2524329
- US 609984 A
- CA 2812756
- CA 2664763
- US 8624002 B
- CA 2607471
- US 7482004 B
- CA 2601267
- CA 2609349
- US 7342106 B
- EP 1888114 A
- US 7687606 B
- US 8013125 B
- US 9120863 B
- US 8999332 B
- US 2379373 A
- US 8008445 B

### Non-patent literature cited in the description

- **SHARMA, P** ; **ALLISON J.P**. The future of immune checkpoint therapy. *Science*, 2015, vol. 348 (6230), 56-61
- **HUGO, W et al.** Genomic and transcriptomic features of response to anti-PD-1 therapy in metastatic melanoma. *Cell*, 2016, vol. 165 (1), 35-44
- **O'SHEA** ; **LEAH K et al.** Therapeutic Targeting of Neu1 Sialidase with Oseltamivir Phosphate (Tamiflu®) Disables Cancer Cell Survival in Human Pancreatic Cancer with Acquired Chemoresistance.. *OncoTargets and therapy*, 2014, vol. 7, 117-134
- **OHM, J.E et al.** VEGF inhibits T-cell development and may contribute to tumor-induced immune suppression. *Blood*, 2003, vol. 101 (12), 4878-4886
- **SAWAOKA, H et al.** Cyclooxygenase inhibitors suppress angiogenesis and reduce tumor growth in vivo.. *Lab Investigation*, 1999, vol. 79 (12), 1469-77
- **MENDELSOHN J** ; **BASELGA J**. Status of epidermal growth factor receptor antagonists in the biology and treatment of cancer.. *J Clin Oncol.*, 2003, vol. 21 (14), 2787-2799
- **PENG, W. et al.** Loss of PTEN promotes resistance to T cell-mediated immunotherapy. *Cancer Discov.*, 2016, vol. 6 (2), 202-216
- **UDDIN, S. et al.** Cyclooxygenase-2 inhibition inhibits PI3K/AKT kinase activity in epithelial ovarian cancer.. *International Journal of Cancer*, 2010, vol. 126, 382-394
- **MANI, S. A.. et al.** The Epithelial-Mesenchymal Transition Generates Cells with Properties of Stem Cells.. *Cell*, 2008, vol. 133 (4), 704-715
- **ZHANG, HUI-HUI** ; **GUO, XIU-LI**. Combinational strategies of metformin and chemotherapy in cancers. *Cancer Chemother. Pharmacol.*, 2016, vol. 78, 13-26